# EUROPEAN PATENT APPLICATION

(11) **EP 4 722 361 A1**
(43) Date of publication of application: **08.04.2026**
(21) Application number: 24810495.2
(22) Date of filing: 24.05.2024
(51) Int. Cl.: C12N 15/11, C12N 15/85, A61K 31/7088

(54) **VECTOR USED FOR PREPARING CIRCULAR RNA AND METHOD**

(30) Priority: 24.05.2023 CN 202310597592; 11.08.2023 CN 202311018953
(71) Applicant: Nanjing GenScript Biotech Co., Ltd., Nanjing, Jiangsu 211100 (CN)
(72) Inventor: ZHAO, Youshang, Nanjing, Jiangsu 211100 (CN); SUN, Zhen, Nanjing, Jiangsu 211100 (CN); YUAN, Xiaohui, Nanjing, Jiangsu 211100 (CN); HU, Ting, Nanjing, Jiangsu 211100 (CN); CHEN, Kangming, Nanjing, Jiangsu 211100 (CN)
(74) Representative: Ström & Gulliksson AB
(86) International application number: PCT/CN2024/095316
(87) International publication number: WO 2024/240260

(57) **Abstract**

The present invention relates to a vector used for preparing a circular RNA, said vector comprising: a) an optional 5' homology arm, b) a 3' self-splicing intron fragment containing a 3' splice site, c) an optional 5' spacer sequence, d) an optional internal ribosome entry site (IRES) encoding sequence, e) a target gene, f) an optional 3' spacer sequence, g) a 5' self-splicing intron fragment containing a 5' splice site, and h) an optional 3' homology arm, said elements being operatively connected in sequence. The 3' self-splicing intron fragment containing the 3' splice site and the 5' self-splicing intron fragment containing the 5' splice site are derived from introns of the recA gene of *Bacillus anthracis,* the 23S ribosomal RNA gene of *Coxiella burnetii,* the tRNA-fMet gene of *Scytonema hofmanni,* the tRNA-fMet gene of *Synechocystis sp.,* the 23S rRNA gene of *Thermotoga naphthophila,* the ndhL and trnL-UAA genes of *Synechococcus elongatus,* or the 23S rRNA gene of *Thermotoga subterranea.*

## Description

### Cross-Reference to Related Applications

The present invention claims the priority of Chinese patent applications with the Application Nos. 2023105975929 filed on May 24, 2023 and 2023110189536 filed on August 11, 2023, the entire contents of which are incorporated herein by reference.

### Field of the Invention

The present application relates to the field of molecular biology and bioengineering technology, and in particular to a DNA vector for preparing a circular RNA, a circularizable RNA molecule, and a circular RNA molecule.

### Background Art

With technological breakthroughs, ribonucleic acid (RNA) therapy, including the use of messenger RNA (mRNA), small interfering RNA (siRNA) and microRNA (miRNA), has developed into a new field with great potential in the biopharmaceutical industry. For example, severe acute respiratory syndrome coronavirus 2 (SARS-CoV-2) ^{[1, 2]}, which was widely prevalent around the world not long ago, has attracted people's attention to mRNA vaccines as they bring new hope. The overall process of mRNA design, production and testing is so fast that it undoubtedly represents a novel and potential medical approach for dealing with rapidly mutating virus types in emergencies. With the success of mRNA drugs, methods for converting mRNA into circular RNA (circRNA) to prolong protein translation duration have aroused great concern. Previously, methods for improving mRNA stability mainly involved mimicking natural mRNA structures, including the use of untranslated regions, methylguanosine cap analogs, nucleoside modifications, and codon optimisation, which have improved mRNA stability to some extent ^{[3, 4-7]}. Because circRNA has natural structural advantages, it is theoretically desirable to use it as an alternative method for improving RNA stability.

circRNA is a type of closed, circular, single-stranded RNA molecule whose two ends are covalently linked ^{[8]}. It has been recently found that circRNA mainly exists widely in eukaryotes in the form of non-coding RNA ^{[9, 10, 11]}, and most of the discovered circRNAs are produced by backsplicing ^{[12-14]}. With the discovery of more and more circRNAs, researchers have proposed that certain specific circRNA molecules also have protein-coding functions ^{[15, 16]}. In addition, researchers have already proven that circRNA, after the addition of an internal ribosome entry site (IRES), can undergo translation both *in vivo* and *in vitro* ^{[8, 10, 14]}. Compared with mRNA, circRNA has connected ends, thereby lacking 5' and 3' ends. Therefore, circRNA exhibits stronger resistance to exonuclease degradation and consequently possesses a longer half-life ^{[12, 17]}. Some scholars have attempted to circularize synthesised long linear transcripts into circRNAs ^{[10, 14]} and have made considerable progress.

Most introns require the interaction of multiple proteins for splicing and removal, but some special introns are removed by self-splicing. Such introns with self-splicing function can be divided into two groups, group I and group II, based on their structure and splicing mechanism. The processing of group I introns is initiated by the transesterification reaction mediated by exogenous guanosine cofactors (GTP) ^{[18, 19]}. The splicing of group II introns is similar to that of eukaryotic precursor mRNA, wherein the 5' splice site is attacked by the -OH group of a nucleotide known as the branchpoint-bulge, initially generating an intron lariat structure. Subsequently, the -OH released at the 5' exon attacks the 3' splice site, ultimately splicing the exons at both ends together ^{[19, 20]}. Researchers have achieved the circularization of a target sequence by utilising the self-splicing function of group I and group II introns through sequence design. This involves splitting the self-splicing intron into two parts and loading them onto the two ends of the target RNA sequence to be circularized, followed by *in vitro* catalysis ^{[21-24]}. Circular RNA is covalently closed at both ends and has no exposed 5' end. Therefore, RNA translation cannot be initiated due to the lack of a cap structure. To solve the problem of circular RNA translation, researchers screened and used internal ribosome entry site (IRES) sequences, and successfully synthesised circular RNAs with stable translation function *in vitro.* Daniel G. Andersonet al. ^{[21-24]} achieved *in vitro* RNA circularization by using the self-splicing function of group I introns, such as the intron of the pre-tRNA-Leu gene of *Anabaena* or the Td gene of the T4 phage *(T4td),* wherein the intron of the former has a superior circularization capability.

In the production process of circular RNA, the circularization efficiency has a crucial impact on the yield, purity, and cost of circRNAs. High circularization efficiency can reduce the amount of enzymes, thereby lowering production costs and process residues. However, the current circularization efficiency still has room for improvement.

Citation of any document in the present application does not constitute an admission that such document is prior art to the present application.

### Summary of the Invention

The inventors of the present application, starting from multiple aspects including screening novel introns with an *in vitro* self-splicing capability, optimising homology arms of vectors for preparing a circular RNA, and optimising production processes, have conducted research and improvements on circular RNA preparation technology. This not only enhances the RNA circularization efficiency but also reduces preparation steps, which will positively impact multiple aspects, including increasing circRNA yield, controlling costs, and reducing process residues.

First, the inventors of the present application screen introns derived from various organisms, and find that the introns of *Bacillus anthracis, Coxiella burnetii (Rickettsia burneti), Scytonema hofmanii, Synechocystis sp., Thermotoga naphthophila., Synechococcus elongatus* or *Thermotoga subterranea* (specifically the introns from the recA gene of *Bacillus anthracis,* the 23S ribosomal RNA gene of *Coxiella burnetii,* the tRNA-fMet gene of *Scytonema hofmanii,* the tRNA-fMet gene of *Synechocystis sp.,* the 23S rRNA gene of *Thermotoga naphthophila,* the ndhL and trnL-UAA genes of *Synechococcus elongatus,* or the 23S rRNA gene of *Thermotoga subterranea,* or the trnL gene of *Phormidium sp.)* have great self-splicing capability. Further, the self-splicing introns screened are truncated at different sites to prepare 5' intron fragments and 3' intron fragments. It is found that intron fragments prepared by truncating the introns at given sites can achieve higher RNA circularization efficiency than the introns in the prior art, such as the *Anabaena* introns.

Moreover, the inventors find that when the intron fragments prepared by truncating introns from *Bacillus anthracis* at given sites are used, precursor RNA undergoes circularization simultaneously with *in vitro* transcription. Therefore, there is no need for a circularization step and a corresponding post-circularization purification step after the *in vitro* transcription step. This greatly streamlines the process flow, reduces process residues and, to some extent, decreases the risk of yield reduction caused by multiple purification steps.

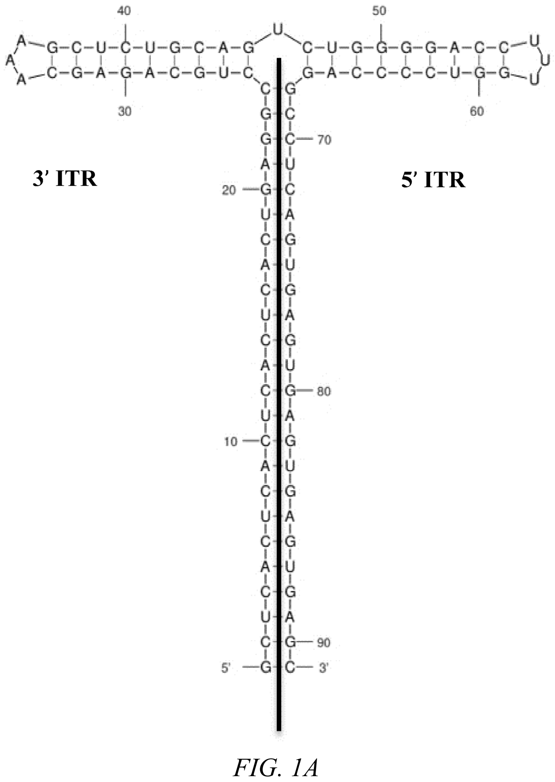

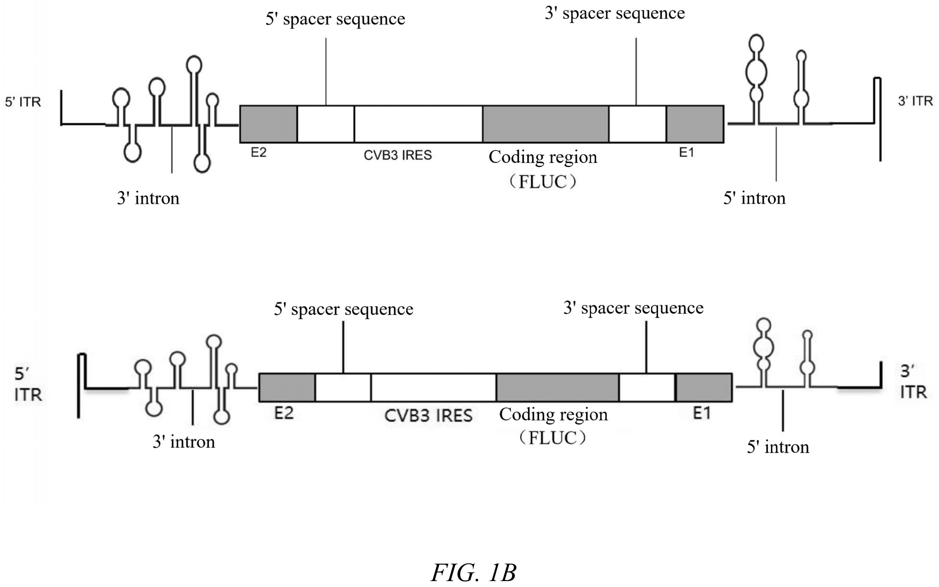

Finally, the inventors optimise the homology arms in the vector for preparing a circular RNA based on prior art, such as the method disclosed by Daniel G. Anderson et al., such that the 5' homology arm and the 3' homology arm are capable of complementarily binding when brought into proximity and forming a stem-loop structure, an inverted terminal repeat (ITR) structure, or a similar structure, and the 5' homology arm and the 3' homology arm exhibit an asymmetric state in their lengths. Accordingly, the intron fragments at both ends of the precursor RNA transcribed from the vector may be more tightly and stably bound together, thereby enhancing the RNA circularization efficiency. In particular, in an exemplary embodiment, the inverted terminal repeat (ITR) contained in the adeno-associated virus (AAV) vector or a similar sequence is asymmetrically split into two parts that serve as the 5' homology arm and 3' homology arm, respectively. As shown in FIGs. 1A and 1B, when the 5' homology arm and the 3' homology arm in the transcript are brought into proximity, two palindromic arms and a long palindromic stem are formed. The presence of palindromic arms, and/or the closed conformation of the terminus opposite the palindromic stem (i.e., the junction of the two palindromic arms) may enable the precursor RNA, particularly the intron ribozyme therein, to have a more stable structure, thereby enhancing RNA circularization efficiency and circular RNA yield.

Therefore, in a first aspect, the present application provides the use of an intron derived from the recA gene of *Bacillus anthracis,* the 23S ribosomal RNA gene of *Coxiella burnetii,* the tRNA-fMet gene of *Synechocystis sp.,* the 23S rRNA gene of *Thermotoga naphthophila,* the tRNA-fMet gene of *Scytonema hofmanni,* the ndhL and trnL-UAA genes of *Synechococcus elongatus,* or the 23S rRNA gene of *Thermotoga subterranea* in the preparation of a vector for the preparation of a circular RNA.

The introns derived from the recA gene of *Bacillus anthracis,* the 23S ribosomal RNA gene of *Coxiella burnetii,* the tRNA-fMet gene of *Synechocystis sp.,* the 23S rRNA gene of *Thermotoga naphthophila,* the tRNA-fMet gene of *Scytonema hofmanni,* the ndhL and trnL-UAA genes of *Synechococcus elongatus,* and the 23S rRNA gene of *Thermotoga subterranea* are all group I self-splicing introns, wherein the intron of the recA gene of *Bacillus anthracis* can comprise a nucleotide sequence that has at least 75%, 80%, 85%, 90%, 91%, 92%, 93%, 94%, 95%, 96%, 97%, 98%, 99% or 100% sequence identity to SEQ ID NO: 18; the intron of the 23S ribosomal RNA gene of *Coxiella burnetii* can comprise a nucleotide sequence that has at least 75%, 80%, 85%, 90%, 91%, 92%, 93%, 94%, 95%, 96%, 97%, 98%, 99% or 100% sequence identity to SEQ ID NO: 19 or 59; the intron of the tRNA-fMet gene of *Synechocystis sp.* can comprise a nucleotide sequence that has at least 75%, 80%, 85%, 90%, 91%, 92%, 93%, 94%, 95%, 96%, 97%, 98%, 99% or 100% sequence identity to SEQ ID NO: 20; the intron of the 23S rRNA gene of *Thermotoga naphthophila* can comprise a nucleotide sequence that has at least 75%, 80%, 85%, 90%, 91%, 92%, 93%, 94%, 95%, 96%, 97%, 98%, 99% or 100% sequence identity to SEQ ID NO: 21; the intron of the tRNA-fMet gene of *Scytonema hofmanni* can comprise a nucleotide sequence that has at least 75%, 80%, 85%, 90%, 91%, 92%, 93%, 94%, 95%, 96%, 97%, 98%, 99% or 100% sequence identity to SEQ ID NO: 60; the introns of the ndhL and trnL-UAA genes of *Synechococcus elongatus* can comprise a nucleotide sequence that has at least 75%, 80%, 85%, 90%, 91%, 92%, 93%, 94%, 95%, 96%, 97%, 98%, 99% or 100% sequence identity to SEQ ID NO: 61; or the intron of the 23S rRNA gene of *Thermotoga subterranea* can comprise a nucleotide sequence that has at least 75%, 80%, 85%, 90%, 91%, 92%, 93%, 94%, 95%, 96%, 97%, 98%, 99% or 100% sequence identity to SEQ ID NO: 62.

In particular, the present application provides a single-stranded DNA molecule, sequentially comprising from the 5' end to the 3' end: a) a 3' self-splicing intron fragment containing a 3' splice site, and b) a 5' self-splicing intron fragment containing a 5' splice site, wherein the elements are operatively connected, wherein the 3' self-splicing intron fragment containing the 3' splice site and the 5' self-splicing intron fragment containing the 5' splice site are derived from introns of the recA gene of *Bacillus anthracis,* the 23S ribosomal RNA gene of *Coxiella burnetii,* the tRNA-fMet gene of *Scytonema hofmanni,* the tRNA-fMet gene of *Synechocystis sp.,* the 23S rRNA gene of *Thermotoga naphthophila,* the ndhL and trnL-UAA genes of *Synechococcus elongatus,* or the 23S rRNA gene of *Thermotoga subterranea.* The introns derived from the recA gene of *Bacillus anthracis,* the 23S ribosomal RNA gene of *Coxiella burnetii,* the tRNA-fMet gene of *Scytonema hofmanni,* the tRNA-fMet gene of *Synechocystis sp.,* the 23S rRNA gene of *Thermotoga naphthophila,* the ndhL and trnL-UAA genes of *Synechococcus elongatus,* and the 23S rRNA gene of *Thermotoga subterranea are* all group I self-splicing introns, wherein the intron of the recA gene of *Bacillus anthracis* can comprise a nucleotide sequence that has at least 75%, 80%, 85%, 90%, 91%, 92%, 93%, 94%, 95%, 96%, 97%, 98%, 99% or 100% sequence identity to SEQ ID NO: 18; the intron of the 23S ribosomal RNA gene of *Coxiella burnetii* can comprise a nucleotide sequence that has at least 75%, 80%, 85%, 90%, 91%, 92%, 93%, 94%, 95%, 96%, 97%, 98%, 99% or 100% sequence identity to SEQ ID NO: 19 or 59; the intron of the tRNA-fMet gene of *Synechocystis sp.* can comprise a nucleotide sequence that has at least 75%, 80%, 85%, 90%, 91%, 92%, 93%, 94%, 95%, 96%, 97%, 98%, 99% or 100% sequence identity to SEQ ID NO: 20; the intron of the 23S rRNA gene of *Thermotoga naphthophila* can comprise a nucleotide sequence that has at least 75%, 80%, 85%, 90%, 91%, 92%, 93%, 94%, 95%, 96%, 97%, 98%, 99% or 100% sequence identity to SEQ ID NO: 21; the intron of the tRNA-fMet gene of *Scytonema hofmanni* can comprise a nucleotide sequence that has at least 75%, 80%, 85%, 90%, 91%, 92%, 93%, 94%, 95%, 96%, 97%, 98%, 99% or 100% sequence identity to SEQ ID NO: 60; the introns of the ndhL and trnL-UAA genes of *Synechococcus elongatus* can comprise a nucleotide sequence that has at least 75%, 80%, 85%, 90%, 91%, 92%, 93%, 94%, 95%, 96%, 97%, 98%, 99% or 100% sequence identity to SEQ ID NO: 61; or the intron of the 23S rRNA gene of *Thermotoga subterranea* can comprise a nucleotide sequence that has at least 75%, 80%, 85%, 90%, 91%, 92%, 93%, 94%, 95%, 96%, 97%, 98%, 99% or 100% sequence identity to SEQ ID NO: 62.

In some embodiments, the intron of the recA gene of *Bacillus anthracis* can comprise the nucleotide sequence as shown in SEQ ID NO: 18; the intron of the 23S ribosomal RNA gene of *Coxiella burnetii* can comprise the nucleotide sequence as shown in SEQ ID NO: 19 or 59; the intron of the tRNA-fMet gene of *Scytonema hofmanni* can comprise the nucleotide sequence as shown in SEQ ID NO: 60; the intron of the tRNA-fMet gene of *Synechocystis sp.* can comprise the nucleotide sequence as shown in SEQ ID NO: 20; the intron of the 23S rRNA of *Thermotoga naphthophila* can comprise the nucleotide sequence as shown in SEQ ID NO: 21; the introns of the ndhL and trnL-UAA genes of *Synechococcus elongatus* can comprise the nucleotide sequence as shown in SEQ ID NO: 61; or the intron of the 23S rRNA gene of *Thermotoga subterranea* can comprise the nucleotide sequence as shown in SEQ ID NO: 62. In other embodiments, the intron of the recA gene of *Bacillus anthracis* can be as shown in SEQ ID NO: 18; the intron of the 23S ribosomal RNA gene of *Coxiella burnetii* can be as shown in SEQ ID NO: 19 or 59; the intron of the tRNA-fMet gene of *Scytonema hofmanni* can be as shown in SEQ ID NO: 60; the intron of the tRNA-fMet gene of *Synechocystis sp.* can be as shown in SEQ ID NO: 20; the intron of the 23S rRNA gene of *Thermotoga naphthophila* can be as shown in SEQ ID NO: 21; the introns of the ndhL and trnL-UAA genes of *Synechococcus elongatus* can be as shown in SEQ ID NO: 61; or the intron of the 23S rRNA gene of *Thermotoga subterranea* can be as shown in SEQ ID NO: 62.

The 3' self-splicing intron fragment can comprise a natural adjacent exon or a sequence corresponding to the natural adjacent exon at the 3' end. In some embodiments, the 3' self-splicing intron fragment can comprise a natural adjacent exon at the 3' end. The 5' self-splicing intron fragment can comprise a natural adjacent exon or a sequence corresponding to the natural adjacent exon at the 5' end. In some embodiments, the 5' self-splicing intron fragment can comprise a natural adjacent exon at the 5' end. The exon can be about 3-200 nucleotides in length.

The 3' self-splicing intron fragment and the 5' self-splicing intron fragment can be from the same intron. The 3' self-splicing intron fragment and the 5' self-splicing intron fragment can be obtained by truncating SEQ ID NO: 18 after the nucleotide at position 133, 195 or 294 of SEQ ID NO: 18, truncating SEQ ID NO: 19 after the nucleotide at position 100, 157, 206 or 245 of SEQ ID NO: 19, truncating SEQ ID NO: 20 after the nucleotide at position 112, 246, 298, 393 or 500 of SEQ ID NO: 20, truncating SEQ ID NO: 21 after the nucleotide at position 130, 314, 369 or 408 of SEQ ID NO: 21, truncating SEQ ID NO: 59 after the nucleotide at position 313 or 453 of SEQ ID NO: 59, truncating SEQ ID NO: 60 after the nucleotide at position 71, 140, 184 or 216 of SEQ ID NO: 60, truncating SEQ ID NO: 61 after the nucleotide at position 135 or 186 of SEQ ID NO: 61, or truncating SEQ ID NO: 62 after the nucleotide at position 94, 336, 492 or 577 of SEQ ID NO: 62.

In some embodiments, the 3' self-splicing intron fragment and the 5' self-splicing intron fragment can comprise nucleotide sequences having at least 85%, 90%, 91%, 92%, 93%, 94%, 95%, 96%, 97%, 98%, 99% or 100% sequence identity to i) SEQ ID NOs: 41 and 42, ii) SEQ ID NOs: 43 and 44, iii) SEQ ID NOs: 47 and 48, iv) SEQ ID NOs: 75 and 76, v) SEQ ID NOs: 39 and 40, vi) SEQ ID NOs: 49 and 50, vii) SEQ ID NOs: 51 and 52, viii) SEQ ID NOs: 53 and 54, ix) SEQ ID NOs: 55 and 56, x) SEQ ID NOs: 57 and 58, xi) SEQ ID NOs: 67 and 68, xii) SEQ ID NOs: 69 and 70, xiii) SEQ ID NOs: 71 and 72, xiv) SEQ ID NOs: 73 and 74, xv) SEQ ID NOs: 45 and 46, xvi) SEQ ID NOs: 77 and 78, xvii) SEQ ID NOs: 79 and 80, xviii) SEQ ID NOs: 81 and 82, xix) SEQ ID NOs: 83 and 84, xx) SEQ ID NOs: 85 and 86, xxi) SEQ ID NOs: 87 and 88, or xxii) SEQ ID NOs: 89 and 90, respectively. In other embodiments, the 3' self-splicing intron fragment and the 5' self-splicing intron fragment can comprise the nucleotide sequences as shown in i) SEQ ID NOs: 41 and 42, ii) SEQ ID NOs: 43 and 44, iii) SEQ ID NOs: 47 and 48, iv) SEQ ID NOs: 75 and 76, v) SEQ ID NOs: 39 and 40, vi) SEQ ID NOs: 49 and 50, vii) SEQ ID NOs: 51 and 52, viii) SEQ ID NOs: 53 and 54, ix) SEQ ID NOs: 55 and 56, x) SEQ ID NOs: 57 and 58, xi) SEQ ID NOs: 67 and 68, xii) SEQ ID NOs: 69 and 70, xiii) SEQ ID NOs: 71 and 72, xiv) SEQ ID NOs: 73 and 74, xv) SEQ ID NOs: 45 and 46, xvi) SEQ ID NOs: 77 and 78, xvii) SEQ ID NOs: 79 and 80, xviii) SEQ ID NOs: 81 and 82, xix) SEQ ID NOs: 83 and 84, xx) SEQ ID NOs: 85 and 86, xxi) SEQ ID NOs: 87 and 88, or xxii) SEQ ID NOs: 89 and 90, respectively.

The 3' splice site and the 5' splice site can be dinucleotides, such as AG and GU, or AC and AU, respectively.

The single-stranded DNA molecule of the present application can further comprise a target gene between the 3' self-splicing intron fragment containing the 3' splice site and the 5' self-splicing intron fragment containing the 5' splice site. In some embodiments, the target gene is a protein coding sequence or a non-coding sequence.

The single-stranded DNA molecule of the present application further comprises a 5' homology arm at the 5' end of the 3' self-splicing intron fragment containing the 3' splice site and a 3' homology arm at the 3' end of the 5' self-splicing intron fragment containing the 5' splice site. The sequences of the 5' homology arm and the 3' homology arm are complementary.

In some embodiments, the sequences of the 5' homology arm and the 3' homology arm are capable of complementarily forming a stem-loop structure, wherein the 5' end of the 5' homology arm and the 3' end of the 3' homology arm are adjacent in the stem; the sequences of the 5' homology arm and the 3' homology arm are capable of complementarily forming a structure containing a palindromic stem and a stem-loop, wherein the palindromic stem is connected to the stem of the stem-loop, wherein the 5' end of the 5' homology arm and the 3' end of the 3' homology arm are adjacent in the palindromic stem; or the sequences of the 5' homology arm and the 3' homology arm are capable of complementarily forming a structure containing a palindromic stem and two stem-loops, wherein the palindromic stem is connected to the stems of the two stem-loops, wherein the 5' end of the 5' homology arm and the 3' end of the 3' homology arm are adjacent in the palindromic stem or in the loop of one of the stem-loops. The lengths of the 5' homology arm and the 3' homology arm are different and may be in the range of 10-120 nucleotides.

In some embodiments, the 5' homology arm and/or the 3' homology arm can comprise a CpG-containing sequence, or a CpG-free sequence. In some embodiments, the 5' homology arm and/or the 3' homology arm can comprise a CpG-free sequence.

The single-stranded DNA molecule of the present application can further comprise an internal ribosome entry site (IRES) encoding sequence between the 3' self-splicing intron fragment containing the 3' splice site and the target gene.

In some embodiments, the IRES can be selected from the IRESs of Coxsackie virus B3 (CVB3), Coxsackie virus A (CVB1/2), Taura syndrome virus, Triatoma virus, Theiler's encephalomyelitis virus, Simian virus 40, Solenopsis invicta virus 1, Rhopalosiphum padi virus, Reticuloendotheliosis virus, Human poliovirus 1, Soybean looper virus, Kashmir bee virus, Human rhinovirus 2, Homalodisca coagulata virus-1, Human immunodeficiency virus type 1, Louse P virus, Hepatitis C virus, Hepatitis A virus, Hepatitis GB virus, Foot and mouth disease virus, Human enterovirus 71, Equine rhinitis virus, Ectropis obliqua picorna-like virus, Encephalomyocarditis virus (EMCV), Drosophila C virus, Crucifer tobamovirus, Cricket paralysis virus, Bovine viral diarrhea virus 1, Black queen cell virus, Aphid lethal paralysis virus, Avian encephalomyelitis virus, Acute bee paralysis virus, Hibiscus chlorotic ringspot virus, Classical swine fever virus, Human FGF2, Human SFTPA1, Human AML1/RUNX1, *Drosophila* antennapedia, Human AQP4, Human AT1R, Human BAG-1, Human BCL2, Human BiP, Human c-IAPl, Human c-myc, Human eIF4G, Mouse NDST4L, Human LEF1, Mouse HIF1α, Human n.myc, Mouse Gtx, Human p27kipl, Human PDGF2/c-sis, Human p53, Human Pim-1, Mouse Rbm3, *Drosophila* reaper, Canine Scamper, *Drosophila* Ubx, Salivirus, Cosavirus, Parechovirus, Human UNR, Mouse UtrA, Human VEGF-A, Human XIAP, *Drosophila* hairless, *Saccharomyces cerevisiae* TFIID, *Saccharomyces cerevisiae* YAP1, Human c-src, Human FGF-1, Simian picornavirus, Turnip crinkle virus or an eIF4G aptamer.

The single-stranded DNA molecule of the present application can further comprise a 3' spacer sequence between the target gene and the 5' self-splicing intron fragment containing the 5' splice site. In some embodiments, the 5' spacer sequence and/or 3' spacer sequence can be at least 10 nucleotides in length.

The single-stranded DNA molecule of the present application can further comprise an RNA polymerase promoter upstream of the 5' homology arm. In some embodiments, the RNA polymerase promoter is an RNA polymerase promoter derived from T7 virus, T6 virus, SP6 virus, T3 virus or T4 virus.

In a second aspect, the present application provides a DNA molecule and vector for preparing a circular RNA.

In particular, the present application provides a single-stranded DNA molecule for preparing a circular RNA, which single-stranded DNA molecule can sequentially comprise from the 5' end to the 3' end: a) a 3' self-splicing intron fragment containing a 3' splice site, b) a target gene, and c) a 5' self-splicing intron fragment containing a 5' splice site, wherein the elements are operatively connected, wherein the 3' self-splicing intron fragment containing the 3' splice site and the 5' self-splicing intron fragment containing the 5' splice site are derived from introns of the recA gene of *Bacillus anthracis,* the 23S ribosomal RNA gene of *Coxiella burnetii,* the tRNA-fMet gene of *Synechocystis sp.,* the 23S rRNA gene of *Thermotoga naphthophila,* the tRNA-fMet gene of *Scytonema hofmanni,* the ndhL and trnL-UAA genes of *Synechococcus elongatus,* or the 23S rRNA gene of *Thermotoga subterranea.*

The introns derived from the recA gene of *Bacillus anthracis,* the 23S ribosomal RNA gene of *Coxiella burnetii,* the tRNA-fMet gene of *Scytonema hofmanni,* the tRNA-fMet gene of *Synechocystis sp.,* the 23S rRNA gene of *Thermotoga naphthophila,* the ndhL and trnL-UAA genes of *Synechococcus elongatus,* and the 23S rRNA gene of *Thermotoga subterranea* are all group I self-splicing introns, wherein the intron of the recA gene of *Bacillus anthracis can* comprise a nucleotide sequence that has at least 75%, 80%, 85%, 90%, 91%, 92%, 93%, 94%, 95%, 96%, 97%, 98%, 99% or 100% sequence identity to SEQ ID NO: 18; the intron of the 23S ribosomal RNA gene of *Coxiella burnetii* can comprise a nucleotide sequence that has at least 75%, 80%, 85%, 90%, 91%, 92%, 93%, 94%, 95%, 96%, 97%, 98%, 99% or 100% sequence identity to SEQ ID NO: 19 or 59; the intron of the tRNA-fMet gene of *Synechocystis sp.* can comprise a nucleotide sequence that has at least 75%, 80%, 85%, 90%, 91%, 92%, 93%, 94%, 95%, 96%, 97%, 98%, 99% or 100% sequence identity to SEQ ID NO: 20; the intron of the 23S rRNA gene of *Thermotoga naphthophila* can comprise a nucleotide sequence that has at least 75%, 80%, 85%, 90%, 91%, 92%, 93%, 94%, 95%, 96%, 97%, 98%, 99% or 100% sequence identity to SEQ ID NO: 21; the intron of the tRNA-fMet gene of *Scytonema hofmanni* can comprise a nucleotide sequence that has at least 75%, 80%, 85%, 90%, 91%, 92%, 93%, 94%, 95%, 96%, 97%, 98%, 99% or 100% sequence identity to SEQ ID NO: 60; the introns of the ndhL and trnL-UAA genes of *Synechococcus elongatus* can comprise a nucleotide sequence that has at least 75%, 80%, 85%, 90%, 91%, 92%, 93%, 94%, 95%, 96%, 97%, 98%, 99% or 100% sequence identity to SEQ ID NO: 61; or the intron of the 23S rRNA gene of *Thermotoga subterranea* can comprise a nucleotide sequence that has at least 75%, 80%, 85%, 90%, 91%, 92%, 93%, 94%, 95%, 96%, 97%, 98%, 99% or 100% sequence identity to SEQ ID NO: 62.

In some embodiments, the intron of the recA gene of *Bacillus anthracis* can comprise the nucleotide sequence as shown in SEQ ID NO: 18; the intron of the 23S ribosomal RNA gene of *Coxiella burnetii* can comprise the nucleotide sequence as shown in SEQ ID NO: 19 or 59; the intron of the tRNA-fMet gene of *Scytonema hofmanni* can comprise the nucleotide sequence as shown in SEQ ID NO: 60; the intron of the tRNA-fMet gene of *Synechocystis sp.* can comprise the nucleotide sequence as shown in SEQ ID NO: 20; the intron of the 23S rRNA of *Thermotoga naphthophila* can comprise the nucleotide sequence as shown in SEQ ID NO: 21; the introns of the ndhL and trnL-UAA genes of *Synechococcus elongatus* can comprise the nucleotide sequence as shown in SEQ ID NO: 61; or the intron of the 23S rRNA gene of *Thermotoga subterranea can* comprise the nucleotide sequence as shown in SEQ ID NO: 62. In other embodiments, the intron of the recA gene of *Bacillus anthracis* can be as shown in SEQ ID NO: 18; the intron of the 23S ribosomal RNA gene of *Coxiella burnetii* can be as shown in SEQ ID NO: 19 or 59; the intron of the tRNA-fMet gene of *Scytonema hofmanni* can be as shown in SEQ ID NO: 60; the intron of the tRNA-fMet gene of *Synechocystis sp.* can be as shown in SEQ ID NO: 20; the intron of the 23S rRNA gene of *Thermotoga naphthophila* can be as shown in SEQ ID NO: 21; the introns of the ndhL and trnL-UAA genes of *Synechococcus elongatus* can be as shown in SEQ ID NO: 61; or the intron of the 23S rRNA gene of *Thermotoga subterranea* can be as shown in SEQ ID NO: 62.

The 3' self-splicing intron fragment can comprise a natural adjacent exon or a sequence corresponding to the natural adjacent exon at the 3' end. In some embodiments, the 3' self-splicing intron fragment can comprise a natural adjacent exon at the 3' end. The 5' self-splicing intron fragment can comprise a natural adjacent exon or a sequence corresponding to the natural adjacent exon at the 5' end. In some embodiments, the 5' self-splicing intron fragment can comprise a natural adjacent exon at the 5' end. The exon can be about 3-200 nucleotides in length.

The 3' self-splicing intron fragment and the 5' self-splicing intron fragment can be from the same intron. The 3' self-splicing intron fragment and the 5' self-splicing intron fragment can be obtained by truncating SEQ ID NO: 18 after the nucleotide at position 133, 195 or 294 of SEQ ID NO: 18, truncating SEQ ID NO: 19 after the nucleotide at position 100, 157, 206 or 245 of SEQ ID NO: 19, truncating SEQ ID NO: 20 after the nucleotide at position 112, 246, 298, 393 or 500 of SEQ ID NO: 20, truncating SEQ ID NO: 21 after the nucleotide at position 130, 314, 369 or 408 of SEQ ID NO: 21, truncating SEQ ID NO: 59 after the nucleotide at position 313 or 453 of SEQ ID NO: 59, truncating SEQ ID NO: 60 after the nucleotide at position 71, 140, 184 or 216 of SEQ ID NO: 60, truncating SEQ ID NO: 61 after the nucleotide at position 135 or 186 of SEQ ID NO: 61, or truncating SEQ ID NO: 62 after the nucleotide at position 94, 336, 492 or 577 of SEQ ID NO: 62.

In some embodiments, the 3' self-splicing intron fragment and the 5' self-splicing intron fragment can comprise nucleotide sequences having at least 85%, 90%, 91%, 92%, 93%, 94%, 95%, 96%, 97%, 98%, 99% or 100% sequence identity to i) SEQ ID NOs: 41 and 42, ii) SEQ ID NOs: 43 and 44, iii) SEQ ID NOs: 47 and 48, iv) SEQ ID NOs: 75 and 76, v) SEQ ID NOs: 39 and 40, vi) SEQ ID NOs: 49 and 50, vii) SEQ ID NOs: 51 and 52, viii) SEQ ID NOs: 53 and 54, ix) SEQ ID NOs: 55 and 56, x) SEQ ID NOs: 57 and 58, xi) SEQ ID NOs: 67 and 68, xii) SEQ ID NOs: 69 and 70, xiii) SEQ ID NOs: 71 and 72, xiv) SEQ ID NOs: 73 and 74, xv) SEQ ID NOs: 45 and 46, xvi) SEQ ID NOs: 77 and 78, xvii) SEQ ID NOs: 79 and 80, xviii) SEQ ID NOs: 81 and 82, xix) SEQ ID NOs: 83 and 84, xx) SEQ ID NOs: 85 and 86, xxi) SEQ ID NOs: 87 and 88, or xxii) SEQ ID NOs: 89 and 90, respectively. In other embodiments, the 3' self-splicing intron fragment and the 5' self-splicing intron fragment can comprise the nucleotide sequences as shown in i) SEQ ID NOs: 41 and 42, ii) SEQ ID NOs: 43 and 44, iii) SEQ ID NOs: 47 and 48, iv) SEQ ID NOs: 75 and 76, v) SEQ ID NOs: 39 and 40, vi) SEQ ID NOs: 49 and 50, vii) SEQ ID NOs: 51 and 52, viii) SEQ ID NOs: 53 and 54, ix) SEQ ID NOs: 55 and 56, x) SEQ ID NOs: 57 and 58, xi) SEQ ID NOs: 67 and 68, xii) SEQ ID NOs: 69 and 70, xiii) SEQ ID NOs: 71 and 72, xiv) SEQ ID NOs: 73 and 74, xv) SEQ ID NOs: 45 and 46, xvi) SEQ ID NOs: 77 and 78, xvii) SEQ ID NOs: 79 and 80, xviii) SEQ ID NOs: 81 and 82, xix) SEQ ID NOs: 83 and 84, xx) SEQ ID NOs: 85 and 86, xxi) SEQ ID NOs: 87 and 88, or xxii) SEQ ID NOs: 89 and 90, respectively.

The 3' splice site and the 5' splice site can be dinucleotides, such as AG and GU, or AC and AU, respectively.

The term "target gene" mentioned in the context of the single-stranded DNA molecule of the present application refers to a DNA sequence that can be transcribed into a coding RNA or a non-coding RNA. The target gene can be a protein coding sequence or a non-coding sequence. The target gene can be 50-8000 nucleotides in length. The protein coding sequence can encode a protein of eukaryotic or prokaryotic origin. The protein coding sequence can encode a protein of human origin or a protein of non-human origin. In some embodiments, the protein coding sequence can encode an antigenic protein, an antibody, or a Cas9 endonuclease. In some embodiments, the protein coding sequence can encode firefly luciferase or *Gaussia* luciferase.

The single-stranded DNA molecule for preparing a circular RNA of the present application can comprise a 5' homology arm at the 5' end of the 3' self-splicing intron fragment and a 3' homology arm at the 3' end of the 5' self-splicing intron fragment. The sequences of the 5' homology arm and the 3' homology arm are complementary.

In some embodiments, the sequences of the 5' homology arm and the 3' homology arm are capable of complementarily forming a stem-loop structure, wherein the 5' end of the 5' homology arm and the 3' end of the 3' homology arm are adjacent in the stem; the sequences of the 5' homology arm and the 3' homology arm are capable of complementarily forming a structure containing a palindromic stem and a stem-loop, wherein the palindromic stem is connected to the stem of the stem-loop, wherein the 5' end of the 5' homology arm and the 3' end of the 3' homology arm are adjacent in the palindromic stem; or the sequences of the 5' homology arm and the 3' homology arm are capable of complementarily forming a structure containing a palindromic stem and two stem-loops, wherein the palindromic stem is connected to the stems of the two stem-loops, wherein the 5' end of the 5' homology arm and the 3' end of the 3' homology arm are adjacent in the palindromic stem or in the loop of one of the stem-loops. The lengths of the 5' homology arm and the 3' homology arm are different and may be in the range of 10-120 nucleotides.

In one embodiment, the 5' homology arm can sequentially comprise, from the 5' end to the 3' end, a first sequence and a second sequence, and the 3' homology arm can sequentially comprise, from the 5' end to the 3' end, a third sequence, a fourth sequence, a fifth sequence and a sixth sequence, wherein the first sequence is the reverse complement of the sixth sequence, the second sequence is the reverse complement of the third sequence, and the fourth sequence is the reverse complement of the fifth sequence. The first sequence, the fourth sequence, the fifth sequence and the sixth sequence can be 3-20 nucleotides in length. The second sequence and the third sequence can be 20-50 nucleotides in length. In some embodiments, the first sequence, the fourth sequence, the fifth sequence and the sixth sequence can be 3 nucleotides or more, 5 nucleotides or more, 10 nucleotides or more, or 15 nucleotides or more, and 20 nucleotides or less, 15 nucleotides or less, or 10 nucleotides or less in length. In some embodiments, the second sequence and the third sequence can be 20 nucleotides or more, 25 nucleotides or more, 30 nucleotides or more, 35 nucleotides or more, 40 nucleotides or more, or 45 nucleotides or more in length. A seventh sequence can be further comprised between the fourth sequence and the fifth sequence, wherein the seventh sequence can be 1-15 nucleotides in length, and the internal sequence thereof does not undergo complementary pairing. The RNA sequence transcribed from the seventh sequence forms the loop of a hairpin (stem-loop) structure, or the turn of a palindromic arm. In some embodiments, the seventh sequence can be about 3 nucleotides in length, comprising, for example, 3 adenylic acids (A) or thymidylic acids (T). A linker sequence of 1-3 nucleotides in length can be further comprised between the fifth sequence and the sixth sequence. In some embodiments, the linker sequence can be one nucleotide in length.

In one embodiment, the 5' homology arm sequentially comprises, from the 5' end to the 3' end, a first sequence, a second sequence, a third sequence and a fourth sequence, and the 3' homology arm sequentially comprises, from the 5' end to the 3' end, a fifth sequence and a sixth sequence, wherein the first sequence is the reverse complement of the sixth sequence, the fourth sequence is the reverse complement of the fifth sequence, and the second sequence is the reverse complement of the third sequence. The first sequence, the second sequence, the third sequence and the sixth sequence can be 3-20 nucleotides in length. The fourth sequence and the fifth sequence can be 20-50 nucleotides in length. In some embodiments, the first sequence, the second sequence, the third sequence and the sixth sequence can be 3 nucleotides or more, 5 nucleotides or more, 10 nucleotides or more, or 15 nucleotides or more, and 20 nucleotides or less, 15 nucleotides or less, or 10 nucleotides or less in length. In some embodiments, the fourth sequence and the fifth sequence can be 20 nucleotides or more, 25 nucleotides or more, 30 nucleotides or more, 35 nucleotides or more, 40 nucleotides or more, or 45 nucleotides or more in length. A seventh sequence can be further comprised between the second sequence and the third sequence, wherein the seventh sequence can be 1-15 nucleotides in length, and the internal sequence thereof does not undergo complementary pairing. The RNA transcribed from the seventh sequence forms the loop of a hairpin (stem-loop) structure, or the turn of a palindromic arm. In some embodiments, the seventh sequence can be about 3 nucleotides in length, comprising, for example, 3 adenylic acids (A) or thymidylic acids (T). A linker sequence of 1-3 nucleotides in length can be further comprised between the first sequence and the second sequence. In some embodiments, the linker sequence can be one nucleotide in length.

The 5' homology arm and/or the 3' homology arm can comprise a CpG-containing sequence, or a CpG-free sequence. In some embodiments, the 5' homology arm and/or the 3' homology arm can comprise a CpG-free sequence.

In some embodiments, the 5' homology arm and the 3' homology arm can comprise nucleotide sequences having at least 85%, 90%, 91%, 92%, 93%, 94%, 95%, 96%, 97%, 98%, 99% or 100% sequence identity to i) SEQ ID NOs: 13 and 14; or ii) SEQ ID NOs: 15 and 16, respectively.

The single-stranded DNA molecule for preparing a circular RNA of the present application can further comprise an IRES encoding sequence between the 3' self-splicing intron fragment and the target gene. The IRES can be selected from the IRESs of Coxsackie virus B3 (CVB3), Coxsackie virus A (CVB1/2), Taura syndrome virus, Triatoma virus, Theiler's encephalomyelitis virus, Simian virus 40, Solenopsis invicta virus 1, Rhopalosiphum padi virus, Reticuloendotheliosis virus, Human poliovirus 1, Soybean looper virus, Kashmir bee virus, Human rhinovirus 2, Homalodisca coagulata virus-1, Human immunodeficiency virus type 1, Louse P virus, Hepatitis C virus, Hepatitis A virus, Hepatitis GB virus, Foot and mouth disease virus, Human enterovirus 71, Equine rhinitis virus, Ectropis obliqua picorna-like virus, Encephalomyocarditis virus (EMCV), Drosophila C virus, Crucifer tobamovirus, Cricket paralysis virus, Bovine viral diarrhea virus 1, Black queen cell virus, Aphid lethal paralysis virus, Avian encephalomyelitis virus, Acute bee paralysis virus, Hibiscus chlorotic ringspot virus, Classical swine fever virus, Human FGF2, Human SFTPA1, Human AML1/RUNX1, *Drosophila* antennapedia, Human AQP4, Human AT1R, Human BAG-1, Human BCL2, Human BiP, Human c-IAPl, Human c-myc, Human eIF4G, Mouse NDST4L, Human LEF1, Mouse HIF1α, Human n.myc, Mouse Gtx, Human p27kipl, Human PDGF2/c-sis, Human p53, Human Pim-1, Mouse Rbm3, *Drosophila* reaper, Canine Scamper, *Drosophila* Ubx, Salivirus, Cosavirus, Parechovirus, Human UNR, Mouse UtrA, Human VEGF-A, Human XIAP, *Drosophila* hairless, *Saccharomyces cerevisiae* TFIID, *Saccharomyces cerevisiae* YAP1, Human c-src, Human FGF-1, Simian picornavirus, Turnip crinkle virus or an eIF4G aptamer. In some embodiments, the IRES can be the IRES of Coxsackie virus B3 (CVB3), wherein the encoding sequence thereof can comprise a nucleotide sequence that has at least 85%, 90%, 91%, 92%, 93%, 94%, 95%, 96%, 97%, 98%, 99% or 100% sequence identity to SEQ ID NO: 5. In some embodiments, the IRES encoding sequence can be as shown in SEQ ID NO: 5.

The single-stranded DNA molecule for preparing a circular RNA of the present application can further comprise a 5' spacer sequence between the 3' self-splicing intron fragment and the IRES encoding sequence and/or comprise a 3' spacer sequence between the target gene and the 5' self-splicing intron fragment. The 5' spacer sequence and/or the 3' spacer sequence can be 5-70 nucleotides in length, for example, at least 10 nucleotides, at least 20 nucleotides, at least 30 nucleotides, at least 40 nucleotides, at least 50 nucleotides, or at least 60 nucleotides. In some embodiments, the 5' spacer sequence and/or the 3' spacer sequence is polyA, polyC or polyA-C. In some embodiments, the 5' spacer sequence and the 3' spacer sequence comprise the nucleotide sequences as shown in SEQ ID NOs: 4 and 9, respectively.

The single-stranded DNA molecule for preparing a circular RNA of the present application can further comprise an RNA polymerase promoter at the 5' end, such as upstream of the 3' self-splicing intron fragment or the 5' homology arm. The RNA polymerase promoter can be an RNA polymerase promoter derived from T7 virus, T6 virus, SP6 virus, T3 virus or T4 virus. In some embodiments, the RNA polymerase promoter can be a T7 virus promoter, which can comprise a nucleotide sequence that has at least 85%, 90%, 91%, 92%, 93%, 94%, 95%, 96%, 97%, 98%, 99% or 100% sequence identity to SEQ ID NO: 1. In some embodiments, the RNA polymerase promoter can be as shown in SEQ ID NO: 1.

The single-stranded DNA molecule for preparing a circular RNA of the present application can further comprise a restriction endonuclease site at the 3' end, such as downstream of the 5' self-splicing intron fragment or the 3' homology arm. The restriction endonuclease site can be, for example, EcoRI or EcoRV.

In some embodiments, the single-stranded DNA molecule for preparing a circular RNA of the present application can comprise: a) a 5' homology arm, b) a 3' self-splicing intron fragment containing a 3' splice site, c) a 5' spacer sequence, d) an internal ribosome entry site (IRES) encoding sequence, e) a protein coding sequence, f) a 3' spacer sequence, g) a 5' self-splicing intron fragment containing a 5' splice site, and h) a 3' homology arm; or a) a 5' homology arm, b) a 3' self-splicing intron fragment containing a 3' splice site, c) a 5' spacer sequence, d) an internal ribosome entry site (IRES), e) a protein coding sequence, f) a 5' self-splicing intron fragment containing a 5' splice site, and g) a 3' homology arm.

In some embodiments, the single-stranded DNA molecule for preparing a circular RNA of the present application can comprise: a) a 5' homology arm, b) a 3' self-splicing intron fragment containing a 3' splice site, c) a non-coding sequence, d) a 5' self-splicing intron fragment containing a 5' splice site, and e) a 3' homology arm.

The present application further provides a single-stranded DNA molecule for preparing a circular RNA, which single-stranded DNA molecule can sequentially comprise, from the 5' end to the 3' end: a) a 5' homology arm, b) a 3' self-splicing intron fragment containing a 3' splice site, c) a target gene, d) a 5' self-splicing intron fragment containing a 5' splice site, and e) a 3' homology arm, wherein the elements are operatively connected, wherein the sequences of the 5' homology arm and the 3' homology arm are capable of i) complementarily forming a stem-loop structure, wherein the 5' end of the 5' homology arm and the 3' end of the 3' homology arm are adjacent in the stem; ii) complementarily forming a structure containing a palindromic stem and a stem-loop, wherein the palindromic stem is connected to the stem of the stem-loop, wherein the 5' end of the 5' homology arm and the 3' end of the 3' homology arm are adjacent in the palindromic stem; or iii) complementarily forming a structure containing a palindromic stem and two stem-loops, wherein the palindromic stem is connected to the stem of the two stem-loops, wherein the 5' end of the 5' homology arm and the 3' end of the 3' homology arm are adjacent in the palindromic stem or in the loop of one of the stem-loops.

The 3' self-splicing intron fragment containing the 3' splice site and the 5' self-splicing intron fragment containing the 5' splice site can be derived from any self-splicing intron, including groups I and II introns, particularly group I self-splicing intron. In some embodiments, the 3' self-splicing intron fragment containing the 3' splice site and the 5' self-splicing intron fragment containing the 5' splice site can be derived from the recA gene of *Bacillus anthracis,* the 23S ribosomal RNA gene of *Coxiella burnetii,* the tRNA-fMet gene of *Synechocystis sp.,* the 23S rRNA gene of *Thermotoga naphthophila,* the tRNA-fMet gene of *Scytonema hofmanni,* the ndhL and trnL-UAA genes of *Synechococcus elongatus,* or the 23S rRNA gene of *Thermotoga subterranea.*

Optionally, the single-stranded DNA molecule can further comprise a 5' spacer sequence, an IRES encoding sequence and a 3' spacer sequence.

With regard to other aspects of the definitions of the target gene, the 5' spacer sequence, the IRES encoding sequence, the 3' spacer sequence, the 5' homology arm and the 3' homology arm, as well as the 3' self-splicing intron fragment and 5' self-splicing intron fragment derived from the recA gene of *Bacillus anthracis,* the 23S ribosomal RNA gene of *Coxiella burnetii,* the tRNA-fMet gene of *Synechocystis sp.,* the 23S rRNA gene of *Thermotoga naphthophila,* the tRNA-fMet gene of *Scytonema hofmanni,* the ndhL and trnL-UAA genes of *Synechococcus elongatus,* or the 23S rRNA gene of *Thermotoga subterranea* in the single-stranded DNA molecule, they are the same as the above definitions of the elements in the single-stranded DNA molecule.

The present application further provides a double-stranded DNA molecule, which can comprise i) the single-stranded DNA molecule of the present application, and ii) a second strand substantially complementary to the single-stranded DNA molecule. In some embodiments, the double-stranded DNA molecule of the present application can comprise i) the single-stranded DNA molecule of the present application, and ii) a second strand fully complementary to the single-stranded DNA molecule. In some embodiments, the double-stranded DNA molecule is a double-stranded DNA molecule for preparing a circular RNA.

The present application further provides a vector comprising the single-stranded DNA molecule or the double-stranded DNA molecule of the present application. In some embodiments, the vector can be a vector for preparing a circular RNA, and comprises the single-stranded DNA molecule for preparing a circular RNA or the double-stranded DNA molecule for preparing a circular RNA disclosed in the present application.

The vector can be circular or linear. In some embodiments, the vector can be linear. In some embodiment, the vector can be circular and treated to be linear.

A circular RNA of about 500 to about 10000 nucleotides can be transcribed from the vector of the present application. In some embodiments, a circular RNA of at least 500, 550, 600, 650, 700, 750, 800, 850, 900, 950, 1000, 1100, 1200, 1300, 1400, 1500, 1600, 1700, 1800, 1900, 2000, 2500, 3000, 3500, 4000, 4500 or 5000 nucleotides can be transcribed from the vector of the present application.

In a third aspect, the present application provides a method for preparing a circular RNA molecule, comprising: i) *in vitro* transcribing a precursor RNA from the vector for preparing a circular RNA of the present application under suitable conditions, and ii) incubating the precursor RNA under suitable conditions, wherein the suitable conditions in step ii) comprise the presence of magnesium ions and guanosine triphosphate (GTP). The concentration of GTP may be 2 mM; and the concentration of magnesium ions may be 10 mM-20 mM.

In some embodiments, the suitable conditions in step i) comprise: incubation at about 37°C for about 2 hours in the presence of a nucleoside triphosphate. In some embodiments, the suitable conditions in step i) comprise: incubation at about 37°C for about 2 hours in the presence of a nucleoside triphosphate, and addition of DNase I followed by incubation at about 37°C for about 30 minutes. In some embodiments, the suitable conditions in step ii) comprise: incubation at about 70°C for 5 minutes, placement on ice for 3 minutes, addition of GTP and Mg²⁺, and incubation at about 55°C for 8 minutes.

The present application further provides a method for preparing a circular RNA molecule, comprising subjecting the vector for preparing a circular RNA of the present application to *in vitro* transcription under suitable conditions, wherein the 3' self-splicing intron fragment and the 5' self-splicing intron fragment in the vector comprise nucleotide sequences having at least 85%, 90%, 91%, 92%, 93%, 94%, 95%, 96%, 97%, 98%, 99% or 100% sequence identity to SEQ ID NOs: 41 and 42 or SEQ ID NOs: 43 and 44, respectively, wherein the suitable conditions comprise the presence of magnesium ions and a nucleoside triphosphate. In some embodiments, the suitable conditions comprise: incubation at about 37°C in the presence of a nucleoside triphosphate and Mg²⁺ for about 2 hours. In some embodiments, the suitable conditions comprise: incubation at about 37°C in the presence of a nucleoside triphosphate and Mg²⁺ for about 2 hours, and addition of DNase I followed by incubation at about 37°C for about 30 minutes. In some embodiments, the nucleoside triphosphate is guanosine triphosphate.

In a fourth aspect, the present application provides a circularizable RNA molecule, sequentially comprising from the 5' end to the 3' end: a) an optional 5' homology arm, b) a 3' self-splicing intron fragment containing a 3' splice site, c) a 5' spacer sequence, d) an IRES, e) a target gene, f) a 3' spacer sequence, g) a 5' self-splicing intron fragment containing a 5' splice site, and h) an optional 3' homology arm; or a) an optional 5' homology arm, b) a 3' self-splicing intron fragment containing a 3' splice site, c) a 5' spacer sequence, d) an IRES, e) a target gene, f) a 5' self-splicing intron fragment containing a 5' splice site, and g) an optional 3' homology arm, wherein the target gene is a protein coding sequence, and the elements are operatively connected, wherein the 3' self-splicing intron fragment containing the 3' splice site and the 5' self-splicing intron fragment containing the 5' splice site are derived from the recA gene of *Bacillus anthracis,* the 23S ribosomal gene of *Coxiella burnetii,* the tRNA-fMet gene of *Synechocystis sp.,* the 23S rRNA gene of *Thermotoga naphthophila,* the tRNA-fMet gene of *Scytonema hofmanni,* the ndhL and trnL-UAA genes of *Synechococcus elongatus,* or the 23S rRNA gene of *Thermotoga subterranea.*

The present application further provides a circularizable RNA molecule, sequentially comprising from the 5' end to the 3' end: a) an optional 5' homology arm, b) a 3' self-splicing intron fragment containing a 3' splice site, c) a target gene, d) a 5' self-splicing intron fragment containing a 5' splice site, and e) an optional 3' homology arm, wherein the target gene is a non-coding sequence, and the elements are operatively connected, wherein the 3' self-splicing intron fragment containing the 3' splice site and the 5' self-splicing intron fragment containing the 5' splice site are derived from the recA gene of *Bacillus anthracis,* the 23S ribosomal RNA gene of *Coxiella burnetii,* the tRNA-fMet gene of *Synechocystis sp.,* the 23S rRNA gene of *Thermotoga naphthophila,* the tRNA-fMet gene of *Scytonema hofmanni,* the ndhL and trnL-UAA genes of *Synechococcus elongatus,* or the 23S rRNA gene of *Thermotoga subterranea.*

The introns of the recA gene of *Bacillus anthracis,* the 23S ribosomal RNA gene of *Coxiella burnetii,* the tRNA-fMet gene of *Synechocystis sp.,* the 23S rRNA gene of *Thermotoga naphthophila,* the 23S ribosomal RNA gene-2 of *Coxiella burnetii,* the tRNA-fMet gene of *Scytonema hofmanni,* the ndhL and trnL-UAA genes of *Synechococcus elongatus,* or the 23S rRNA gene of *Thermotoga subterranea* can comprise nucleotide sequences having at least 75%, 80%, 85%, 90%, 91%, 92%, 93%, 94%, 95%, 96%, 97%, 98%, 99% or 100% sequence identity to SEQ ID NOs: 18-21 and SEQ ID NOs: 59-62, respectively. In some embodiments, the introns of the recA gene of *Bacillus anthracis,* the tRNA-fMet gene of *Scytonema hofmanni,* the tRNA-fMet gene of *Synechocystis sp.,* the 23S rRNA gene of *Thermotoga naphthophila,* the ndhL and trnL-UAA genes of *Synechococcus elongatus,* or the 23S rRNA gene of *Thermotoga subterranea* can comprise the nucleotide sequences as shown in SEQ ID NO: 18, 60, 20, 21, 61 or 62, respectively; the intron of the 23S ribosomal RNA gene of *Coxiella burnetii* can comprise the nucleotide sequence as shown in SEQ ID NO: 19 or 59.

The 3' self-splicing intron fragment can comprise an RNA sequence corresponding to the natural adjacent exon at the 3' end. The 5' self-splicing intron fragment can comprise an RNA sequence corresponding to the natural adjacent exon at the 5' end. The exon can be about 3-200 nucleotides in length.

The 3' self-splicing intron fragment and the 5' self-splicing intron fragment can be derived from the same intron. The 3' self-splicing intron fragment and the 5' self-splicing intron fragment can be transcribed from nucleotide sequences having at least 75%, 80%, 85%, 90%, 91%, 92%, 93%, 94%, 95%, 96%, 97%, 98%, 99% or 100% sequence identity to i) SEQ ID NOs: 41 and 42, ii) SEQ ID NOs: 43 and 44, iii) SEQ ID NOs: 47 and 48, iv) SEQ ID NOs: 75 and 76, v) SEQ ID NOs: 39 and 40, vi) SEQ ID NOs: 49 and 50, vii) SEQ ID NOs: 51 and 52, viii) SEQ ID NOs: 53 and 54, ix) SEQ ID NOs: 55 and 56, x) SEQ ID NOs: 57 and 58, xi) SEQ ID NOs: 67 and 68, xii) SEQ ID NOs: 69 and 70, xiii) SEQ ID NOs: 71 and 72, xiv) SEQ ID NOs: 73 and 74, xv) SEQ ID NOs: 45 and 46, xvi) SEQ ID NOs: 77 and 78, xvii) SEQ ID NOs: 79 and 80, xviii) SEQ ID NOs: 81 and 82, xix) SEQ ID NOs: 83 and 84, xx) SEQ ID NOs: 85 and 86, xxi) SEQ ID NOs: 87 and 88, or xxii) SEQ ID NOs: 89 and 90, respectively, that is, the intron fragments comprise the corresponding RNA sequences of nucleotide sequences having at least 75%, 80%, 85%, 90%, 91%, 92%, 93%, 94%, 95%, 96%, 97%, 98%, 99% or 100% sequence identity to i) SEQ ID NOs: 41 and 42, ii) SEQ ID NOs:43 and 44, iii) SEQ ID NOs:47 and 48, iv) SEQ ID NOs: 75 and 76, v) SEQ ID NOs:39 and 40, vi) SEQ ID NOs: 49 and 50, vii) SEQ ID NOs: 51 and 52, viii) SEQ ID NOs: 53 and 54, ix) SEQ ID NOs: 55 and 56, x) SEQ ID NOs: 57 and 58, xi) SEQ ID NOs: 67 and 68, xii) SEQ ID NOs: 69 and 70, xiii) SEQ ID NOs: 71 and 72, xiv) SEQ ID NOs: 73 and 74, xv) SEQ ID NOs: 45 and 46, xvi) SEQ ID NOs: 77 and 78, xvii) SEQ ID NOs: 79 and 80, xviii) SEQ ID NOs: 81 and 82, xix) SEQ ID NOs: 83 and 84, xx) SEQ ID NOs: 85 and 86, xxi) SEQ ID NOs: 87 and 88, or xxii) SEQ ID NOs: 89 and 90.

The 3' splice site and the 5' splice site can be dinucleotides, such as AG and GU, or AC and AU, respectively.

The sequences of the 5' homology arm and the 3' homology arm can be substantially complementary or fully complementary.

In some embodiments, the sequences of the 5' homology arm and the 3' homology arm are capable of complementarily forming a stem-loop structure, wherein the 5' end of the 5' homology arm and the 3' end of the 3' homology arm are adjacent in the stem; the sequences of the 5' homology arm and the 3' homology arm are capable of complementarily forming a structure containing a palindromic stem and a stem-loop, wherein the palindromic stem is connected to the stem of the stem-loop, wherein the 5' end of the 5' homology arm and the 3' end of the 3' homology arm are adjacent in the palindromic stem; or the sequences of the 5' homology arm and the 3' homology arm are capable of complementarily forming a structure containing a palindromic stem and two stem-loops, wherein the palindromic stem is connected to the stems of the two stem-loops, wherein the 5' end of the 5' homology arm and the 3' end of the 3' homology arm are adjacent in the palindromic stem or in the loop of one of the stem-loops. The lengths of the 5' homology arm and the 3' homology arm are different and may be in the range of 10-120 nucleotides.

In one embodiment, the 5' homology arm can sequentially comprise, from the 5' end to the 3' end, a first sequence and a second sequence, and the 3' homology arm can sequentially comprise, from the 5' end to the 3' end, a third sequence, a fourth sequence, a fifth sequence and a sixth sequence, wherein the first sequence is the reverse complement of the sixth sequence, the second sequence is the reverse complement of the third sequence, and the fourth sequence is the reverse complement of the fifth sequence. The first sequence, the fourth sequence, the fifth sequence and the sixth sequence can be 3-20 nucleotides in length. The second sequence and the third sequence can be 20-50 nucleotides in length. In some embodiments, the first sequence, the fourth sequence, the fifth sequence and the sixth sequence can be 3 nucleotides or more, 5 nucleotides or more, 10 nucleotides or more, or 15 nucleotides or more, and 20 nucleotides or less, 15 nucleotides or less, or 10 nucleotides or less in length. In some embodiments, the second sequence and the third sequence can be 20 nucleotides or more, 25 nucleotides or more, 30 nucleotides or more, 35 nucleotides or more, 40 nucleotides or more, or 45 nucleotides or more in length. A seventh sequence can be further comprised between the fourth sequence and the fifth sequence, wherein the seventh sequence can be 1-15 nucleotides in length, and the internal sequence thereof does not undergo complementary pairing. The RNA sequence transcribed from the seventh sequence forms the loop of a hairpin (stem-loop) structure, or the turn of a palindromic arm. In some embodiments, the seventh sequence can be about 3 nucleotides in length, comprising, for example, 3 adenylic acids (A) or thymidylic acids (T). A linker sequence of 1-3 nucleotides in length can be further comprised between the fifth sequence and the sixth sequence. In some embodiments, the linker sequence can be one nucleotide in length.

In one embodiment, the 5' homology arm sequentially comprises, from the 5' end to the 3' end, a first sequence, a second sequence, a third sequence and a fourth sequence, and the 3' homology arm sequentially comprises, from the 5' end to the 3' end, a fifth sequence and a sixth sequence, wherein the first sequence is the reverse complement of the sixth sequence, the fourth sequence is the reverse complement of the fifth sequence, and the second sequence is the reverse complement of the third sequence. The first sequence, the second sequence, the third sequence and the sixth sequence can be 3-20 nucleotides in length. The fourth sequence and the fifth sequence can be 20-50 nucleotides in length. In some embodiments, the first sequence, the second sequence, the third sequence and the sixth sequence can be 3 nucleotides or more, 5 nucleotides or more, 10 nucleotides or more, or 15 nucleotides or more, and 20 nucleotides or less, 15 nucleotides or less, or 10 nucleotides or less in length. In some embodiments, the fourth sequence and the fifth sequence can be 20 nucleotides or more, 25 nucleotides or more, 30 nucleotides or more, 35 nucleotides or more, 40 nucleotides or more, or 45 nucleotides or more in length. A seventh sequence can be further comprised between the second sequence and the third sequence, wherein the seventh sequence can be 1-15 nucleotides in length, and the internal sequence thereof does not undergo complementary pairing. The RNA transcribed from the seventh sequence forms the loop of a hairpin (stem-loop) structure, or the turn of a palindromic arm. In some embodiments, the seventh sequence can be about 3 nucleotides in length, comprising, for example, 3 adenylic acids (A) or thymidylic acids (T). A linker sequence of 1-3 nucleotides in length can be further comprised between the first sequence and the second sequence. In some embodiments, the linker sequence can be one nucleotide in length.

The 5' homology arm and/or the 3' homology arm can comprise a CpG-containing sequence, or a CpG-free sequence. In some embodiments, the 5' homology arm and/or the 3' homology arm can comprise a CpG-free sequence.

The 5' spacer sequence and/or the 3' spacer sequence can be 5-70 nucleotides in length, for example, at least 10 nucleotides, at least 20 nucleotides, at least 30 nucleotides, at least 40 nucleotides, at least 50 nucleotides, or at least 60 nucleotides. In some embodiments, the 5' spacer sequence and/or the 3' spacer sequence is polyA, polyC or polyA-C. In some embodiments, the 5' spacer sequence and the 3' spacer sequence can be transcribed from SEQ ID NOs: 4 and 9, respectively, that is, the sequences comprise the corresponding RNA sequences of SEQ ID NOs: 4 and 9.

The internal ribosome entry site (IRES) can be the IRES of Coxsackie virus B3 (CVB3), Coxsackie virus A (CVB1/2), Taura syndrome virus, Triatoma virus, Theiler's encephalomyelitis virus, Simian virus 40, Solenopsis invicta virus 1, Rhopalosiphum padi virus, Reticuloendotheliosis virus, Human poliovirus 1, Soybean looper virus, Kashmir bee virus, Human rhinovirus 2, Homalodisca coagulata virus-1, Human immunodeficiency virus type 1, Louse P virus, Hepatitis C virus, Hepatitis A virus, Hepatitis GB virus, Foot and mouth disease virus, Human enterovirus 71, Equine rhinitis virus, Ectropis obliqua picorna-like virus, Encephalomyocarditis virus (EMCV), Drosophila C virus, Crucifer tobamovirus, Cricket paralysis virus, Bovine viral diarrhea virus 1, Black queen cell virus, Aphid lethal paralysis virus, Avian encephalomyelitis virus, Acute bee paralysis virus, Hibiscus chlorotic ringspot virus, Classical swine fever virus, Human FGF2, Human SFTPA1, Human AML1/RUNX1, *Drosophila* antennapedia, Human AQP4, Human AT1R, Human BAG-1, Human BCL2, Human BiP, Human c-IAPl, Human c-myc, Human eIF4G, Mouse NDST4L, Human LEF1, Mouse HIF1α, Human n.myc, Mouse Gtx, Human p27kipl, Human PDGF2/c-sis, Human p53, Human Pim-1, Mouse Rbm3, *Drosophila* reaper, Canine Scamper, *Drosophila* Ubx, Salivirus, Cosavirus, Parechovirus, Human UNR, Mouse UtrA, Human VEGF-A, Human XIAP, *Drosophila* hairless, *Saccharomyces cerevisiae* TFIID, *Saccharomyces cerevisiae* YAP1, Human c-src, Human FGF-1, Simian picornavirus, Turnip crinkle virus or an eIF4G aptamer. In some embodiments, the IRES can be the IRES of Coxsackie virus B3 (CVB3) and is transcribed from the nucleotide sequence as shown in SEQ ID NO: 5, that is, the IRES comprises the corresponding RNA sequence of SEQ ID NO: 5.

The term "target gene" mentioned in the context of the circularizable RNA molecule of the present application refers to a coding RNA or a non-coding RNA, wherein the coding RNA can be further translated into a protein. The target gene can be a protein coding sequence or a non-coding sequence. The target gene can be 50-8000 nucleotides in length. The protein coding sequence can encode a protein of eukaryotic or prokaryotic origin. The protein coding sequence can encode a protein of human origin or a protein of non-human origin. In some embodiments, the protein coding sequence can encode an antigenic protein, an antibody, or a Cas9 endonuclease. In some embodiments, the protein coding sequence can encode firefly luciferase or *Gaussia* luciferase.

In some embodiments, the circularizable RNA molecule of the present application can be obtained by transcription *(in vitro* transcription or intracellular transcription) using the vector of the present application.

The present application further provides a circularizable RNA molecule, sequentially comprising from the 5' end to the 3' end: a) a 5' homology arm, b) a 3' self-splicing intron fragment containing a 3' splice site, c) a 5' spacer sequence, d) an IRES, e) a target gene, f) a 3' spacer sequence, g) a 5' self-splicing intron fragment containing a 5' splice site, and h) a 3' homology arm, wherein the target gene is a protein coding sequence; or a) a 5' homology arm, b) a 3' self-splicing intron fragment containing a 3' splice site, c) a 5' spacer sequence, d) an IRES, e) a target gene, f) a 5' self-splicing intron fragment containing a 5' splice site, and g) a 3' homology arm, wherein the target gene is a protein coding sequence; or a) a 5' homology arm, b) a 3' self-splicing intron fragment containing a 3' splice site, c) a target gene, d) a 5' self-splicing intron fragment containing a 5' splice site, and e) a 3' homology arm, wherein the target gene is a non-coding sequence, wherein the elements are operatively connected, wherein the sequences of the 5' homology arm and the 3' homology arm are capable of complementarily forming a stem-loop structure, wherein the 5' end of the 5' homology arm and the 3' end of the 3' homology arm are adjacent in the stem; the sequences of the 5' homology arm and the 3' homology arm are capable of complementarily forming a structure containing a palindromic stem and a stem-loop, wherein the palindromic stem is connected to the stem of the stem-loop, wherein the 5' end of the 5' homology arm and the 3' end of the 3' homology arm are adjacent in the palindromic stem; or the sequences of the 5' homology arm and the 3' homology arm are capable of complementarily forming a structure containing a palindromic stem and two stem-loops, wherein the palindromic stem is connected to the stems of the two stem-loops, wherein the 5' end of the 5' homology arm and the 3' end of the 3' homology arm are adjacent in the palindromic stem or in the loop of one of the stem-loops.

The 3' self-splicing intron fragment containing the 3' splice site and the 5' self-splicing intron fragment containing the 5' splice site can be derived from any self-splicing intron, including groups I and II introns, particularly group I self-splicing intron. In some embodiments, the 3' self-splicing intron fragment containing the 3' splice site and the 5' self-splicing intron fragment containing the 5' splice site can be derived from the recA gene of *Bacillus anthracis,* the 23S ribosomal RNA gene of *Coxiella burnetii,* the tRNA-fMet gene of *Synechocystis sp.,* the 23S rRNA gene of *Thermotoga naphthophila,* the tRNA-fMet gene of *Scytonema hofmanni,* the ndhL and trnL-UAA genes of *Synechococcus elongatus,* or the 23S rRNA gene of *Thermotoga subterranea.*

With regard to other aspects of the definitions of the 5' homology arm, the 5' spacer sequence, the internal ribosome entry site (IRES), the target gene, the 3' spacer sequence, the 3' homology arm, as well as the 3' self-splicing intron fragment and 5' self-splicing intron fragment derived from the recA gene of *Bacillus anthracis,* the 23S ribosomal RNA gene of *Coxiella burnetii,* the tRNA-fMet gene of *Synechocystis sp.,* the 23S rRNA gene of *Thermotoga naphthophila,* the tRNA-fMet gene of *Scytonema hofmanni,* the ndhL and trnL-UAA genes of *Synechococcus elongatus,* or the 23S rRNA gene of *Thermotoga subterranea* in the circularizable RNA molecule, they are the same as the above definitions of the elements in the circularizable RNA molecule.

In a fifth aspect, the present application provides a circular RNA molecule, which can be obtained by the method for preparing a circular RNA of the present application using the vector for preparing a circular RNA of the present application.

The present application further provides a circular RNA, which can be obtained by treating the circularizable RNA molecule of the present application under suitable conditions. The suitable conditions refer to the presence of magnesium ions and guanosine triphosphate (GTP). In some embodiments, the suitable conditions comprise: incubation at about 37°C in the presence of guanosine triphosphate and Mg²⁺ for about 2 hours. In some embodiments, the suitable conditions comprise: incubation at about 37°C in the presence of guanosine triphosphate and Mg²⁺ for about 2 hours, and addition of DNase I followed by incubation at about 37°C for about 30 minutes. The concentration of GTP may be 2 mM; and the concentration of magnesium ions may be 10 mM-20 mM.

The circular RNA molecule can be translated into a protein in a cell, or exist as a biologically active non-coding RNA. The circular RNA can be at least 500, 550, 600, 650, 700, 750, 800, 850, 900, 950, 1000, 1100, 1200, 1300, 1400, 1500, 1600, 1700, 1800, 1900, 2000, 2500, 3000, 3500, 4000, 4500 or 5000 nucleotides in length.

In a sixth aspect, the present application provides a host cell, which can comprise the single-stranded DNA molecule, the double-stranded DNA molecule, the vector, the circularizable RNA molecule or the circular RNA molecule of the present application.

The present application further provides a composition, which can comprise the single-stranded DNA molecule, the double-stranded DNA molecule, the vector, the circularizable RNA molecule, or the host cell of the present application. The composition can be used to prepare a circularizable RNA and/or a circular RNA, particularly for producing a circular RNA that can be translated into a protein or a biologically active circular RNA *in vitro* or in a cell. The biologically active circular RNA can be, for example, a miRNA sponge or a non-coding RNA. The composition (comprising a single-stranded DNA molecule, a double-stranded DNA molecule) of the present application can be used to prepare a vector for preparing a circular RNA.

The present application further provides a composition, which can comprise the circular RNA molecule of the present application. The composition can be a pharmaceutical composition, and can further comprise a pharmaceutically acceptable carrier. The composition can be transfected into cells via, for example, lipofection, electroporation, or nanocarrier encapsulation.

The present application also provides the use of the composition for the preparation of a circular RNA, or for *in vivo* therapy, etc. For example, in one embodiment, the present application provides a method for expressing a vaccine, a therapeutic protein, or other types of proteins in a subject in need thereof, comprising administering to the subject a composition comprising a circular RNA of the present application. The therapeutic protein can be, for example, an antibody or a fusion protein.

In a seventh aspect, the present application provides a method for screening an intron having self-splicing capability, comprising: i) providing a double-stranded DNA molecule comprising, from the 5' end to the 3' end, a promoter, a first sequence, a 5' natural adjacent exon of an intron to be screened or a sequence corresponding to the natural adjacent exon, the intron to be screened, a 3' natural adjacent exon of the intron to be screened or a sequence corresponding to the natural adjacent exon, and a second sequence; ii) subjecting the double-stranded DNA molecule in step i) to transcription to obtain an RNA molecule; iii) treating the RNA molecule of step ii) under suitable conditions, wherein the suitable conditions comprise the presence of magnesium ion and guanosine triphosphate (GTP); and iv) analysing whether the RNA molecule treated in step iii) comprises the transcript sequence of the intron to be screened, wherein the absence of the transcript sequence of the intron to be screened in the RNA molecule treated in step iii) indicates that the intron to be screened is an intron having self-splicing capability.

The first sequence and the second sequence can be any sequence.

The promoter can be a T7 promoter.

The transcription in step ii) can be performed by *in vitro* transcription.

The analysing in step iv) can comprise: reverse transcribing the RNA molecule treated in step iii) into a single-stranded DNA molecule and sequencing the single-stranded DNA molecule, or sequencing the RNA molecule treated in step iii), wherein the absence of the sequence of the intron to be screened indicates that the intron to be screened is an intron having self-splicing capability.

If the structure of an intron is determined, but it is uncertain whether the intron has self-splicing capability, whether the sequence of the intron to be screened is absent can be analysed according to step iv).

The analysing in step iv) can comprise: reverse transcribing the RNA molecule treated in step iii) into a single-stranded DNA molecule, converting the single-stranded DNA molecule into a double-stranded DNA molecule, and comparing the length of the double-stranded DNA molecule with that of the double-stranded DNA molecule in step i), wherein the length of the double-stranded DNA molecule being shorter than that of the double-stranded DNA molecule of step i) indicates that the intron to be screened is an intron having self-splicing capability.

The analysing in step iv) can comprise: comparing the length of the RNA molecule of step ii) with that of the RNA molecule treated in step iii). Comparing the length of the RNA molecule of step ii) with that of the RNA molecule treated in step iii) can be performed by gel electrophoresis. In particular, the analysing in step iv) can comprise: comparing the migration rate of the RNA molecule of step ii) with that of the RNA molecule treated in step iii) in gel electrophoresis, wherein the migration rate of the RNA molecule treated in step iii) being greater than that of the RNA molecule of step ii) indicates that the intron to be screened is an intron having self-splicing capability. The gel in the gel electrophoresis may comprise 1% agarose, and the gel electrophoresis is performed for 20 minutes. The analysing in step iv) can further comprise, after the gel electrophoresis, recovering the RNA molecule treated in step iii) from the gel, reverse transcribing the RNA molecule into a single-stranded DNA molecule, converting the single-stranded DNA molecule into a double-stranded DNA molecule, and comparing the length of the double-stranded DNA molecule with that the double-stranded DNA molecule in step i), wherein the length of the double-stranded DNA molecule being shorter than that of the double-stranded DNA molecule of step i) indicates that the intron to be screened is an intron having self-splicing capability. Comparing the length of the double-stranded DNA molecule with that of the double-stranded DNA molecule in step i) can be performed by gel electrophoresis. Alternatively, the analysing in step iv) can further comprise, after the gel electrophoresis, recovering the RNA molecule treated in step iii) from the gel, reverse transcribing the RNA molecule into a single-stranded DNA molecule, and sequencing the single-stranded DNA molecule, wherein the absence of the sequence of the intron to be screened indicates that the intron to be screened is an intron having self-splicing capability.

Other features and advantages of the present disclosure will be apparent from the following detailed descriptions and examples, which should not be construed as limiting. All the documents, Genbank accession numbers, patents and published patent applications cited in the description are incorporated herein by reference.

### Brief Description of the Drawings

The following detailed description is given by way of example and is not intended to limit the present invention to the specific embodiments, and can be better understood in conjunction with the accompanying drawings.
FIGs. 1A-1D show the design rationale of the 5' homology arm and the 3' homology arm in the vector constructed in the present application (1A), a schematic diagram of the basic structure of the vector transcript (1B), a gel electrophoresis diagram (1C) and a capillary electrophoresis diagram (1D) of the *in vitro* transcription (IVT) transcript of the vector and the circular RNA circularized therefrom.

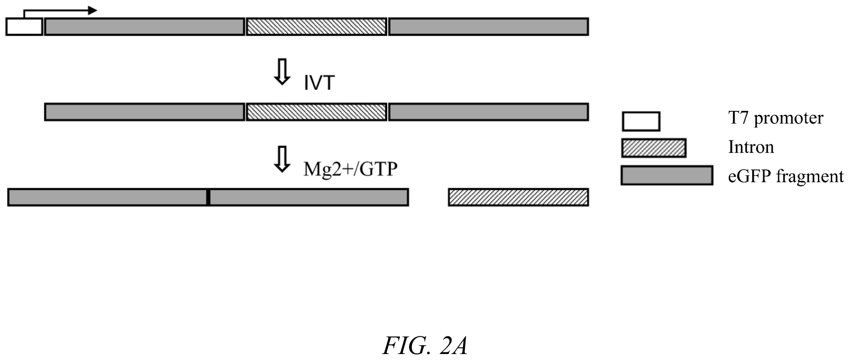

FIGs. 2A-2C show a schematic diagram of the construction of a vector for screening introns (2A), an E-Gel^{™} EX gel image of the vector transcript after intron splicing (2B), and an agarose gel electrophoresis diagram of the vector transcript after intron splicing (2C).

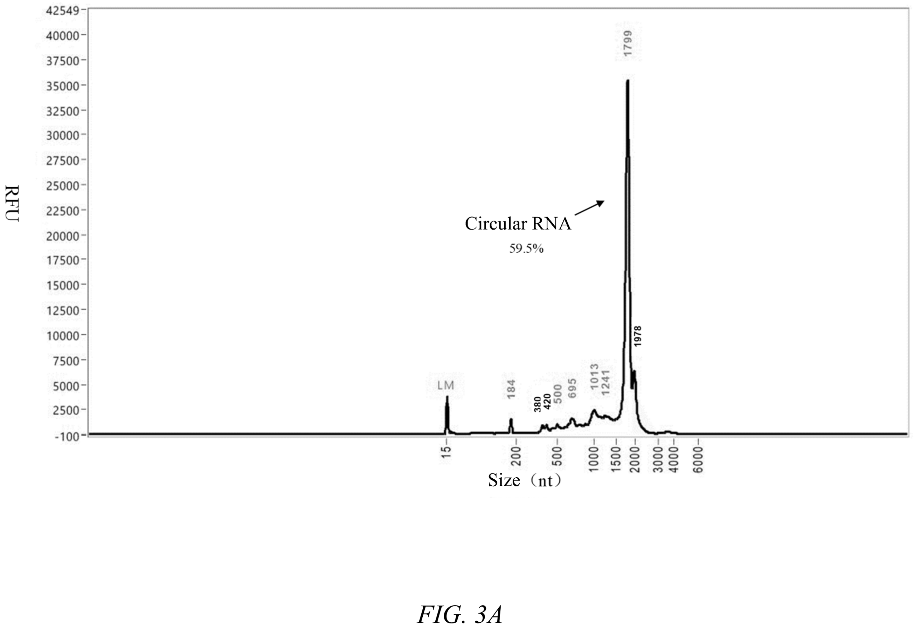

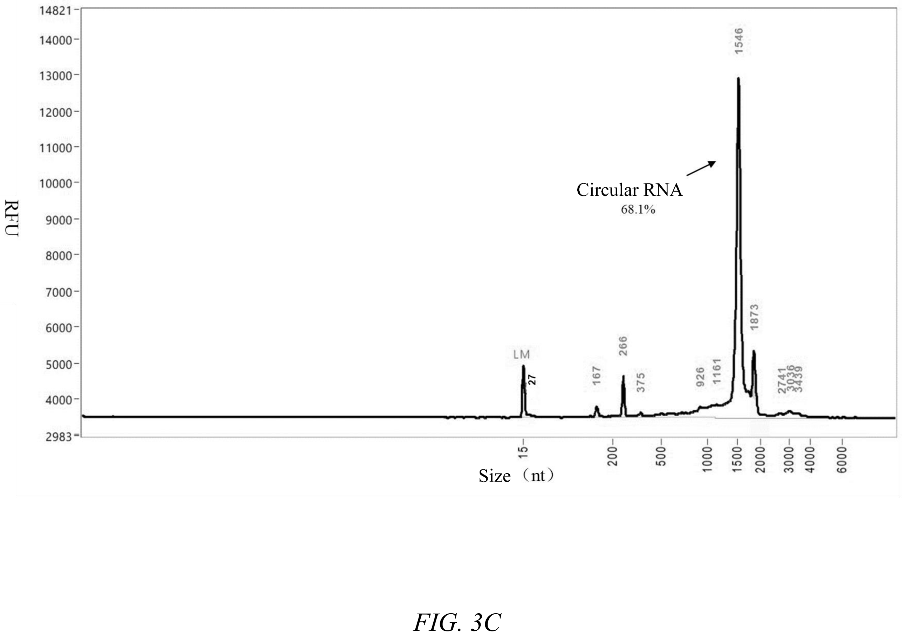

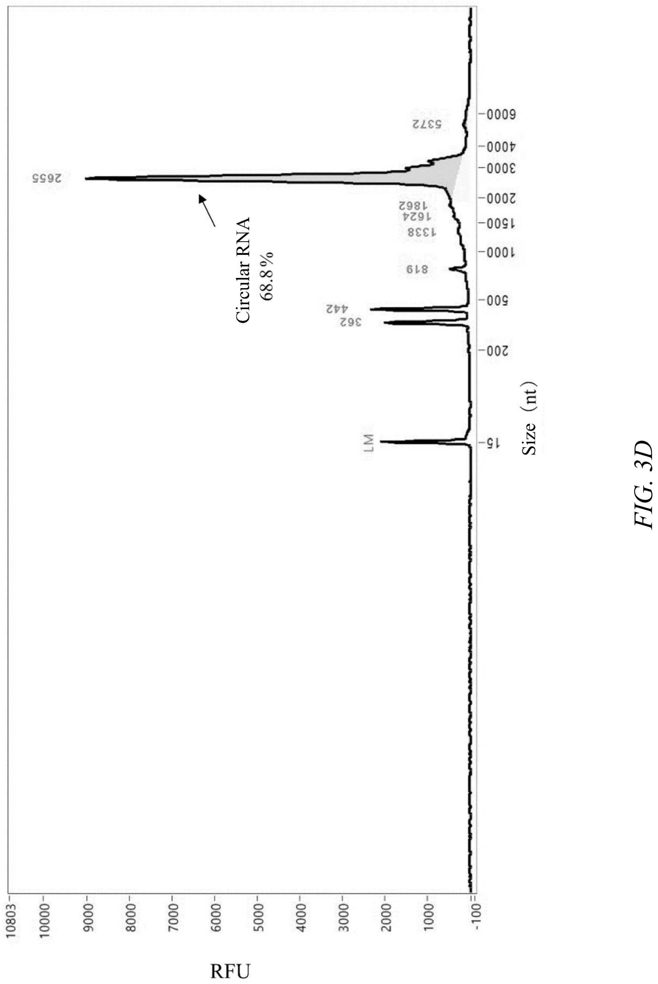

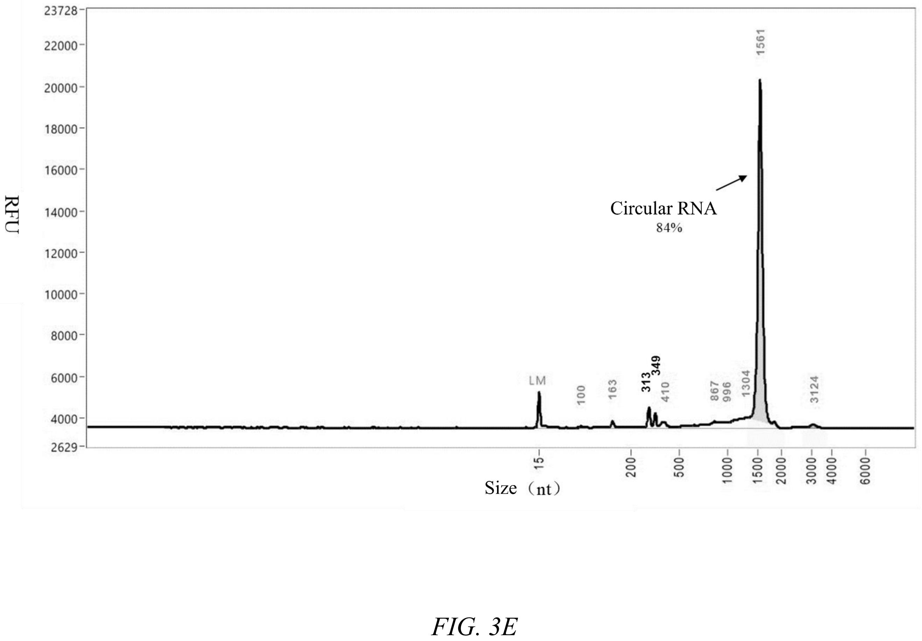

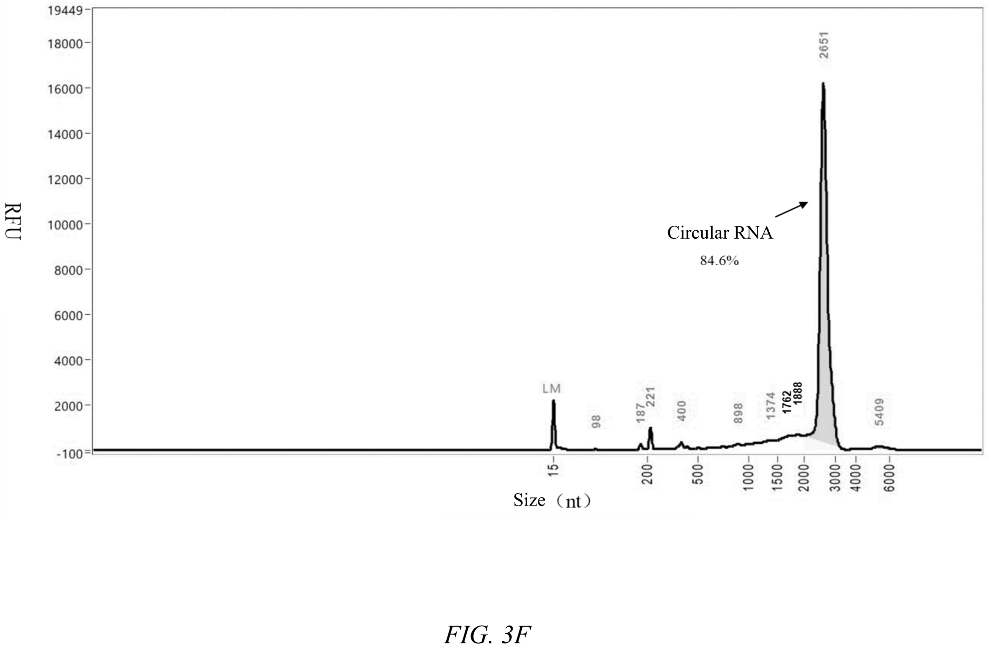

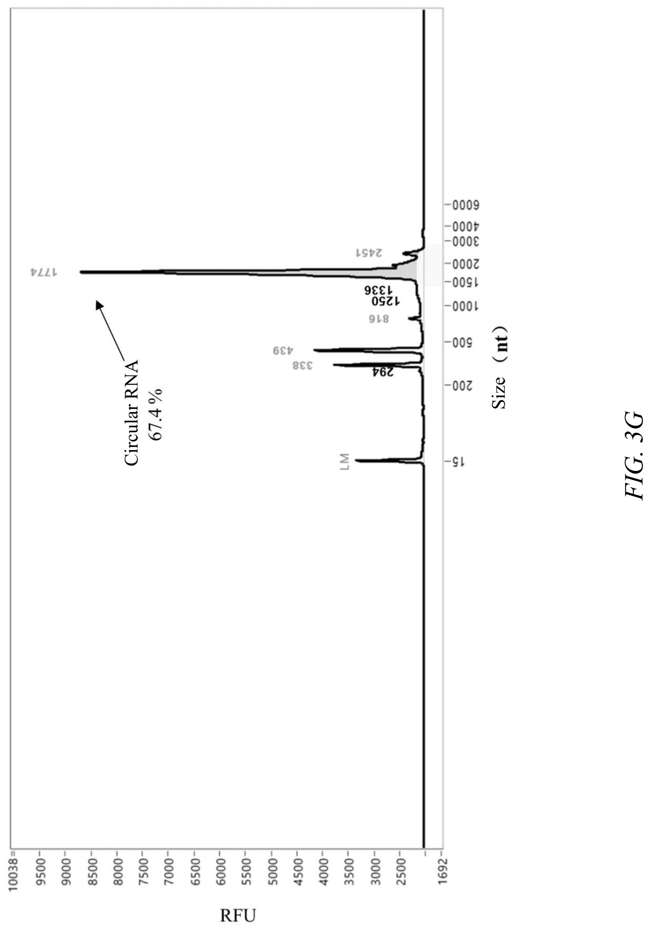

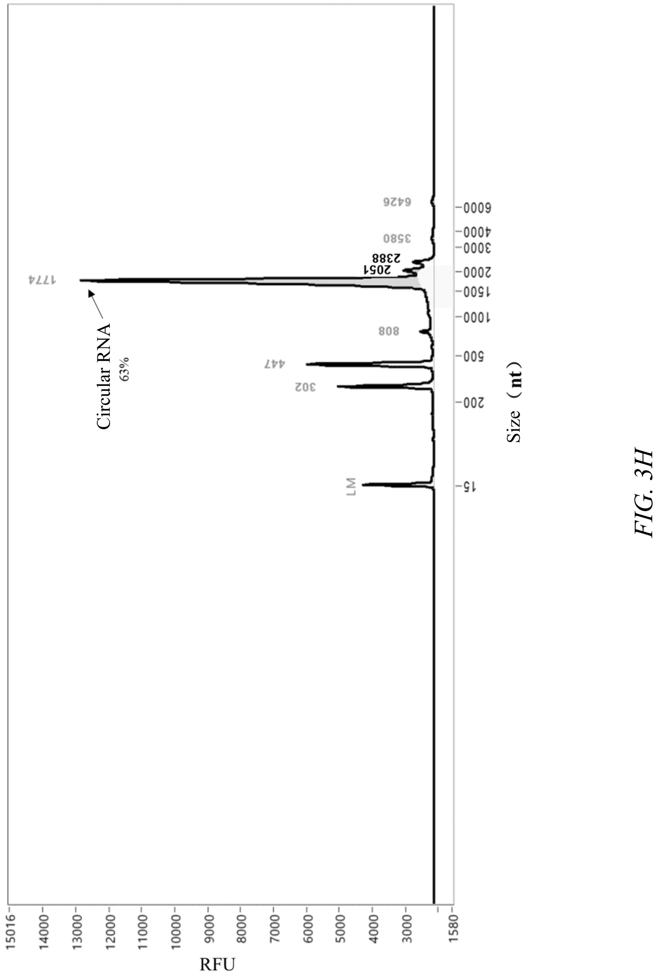

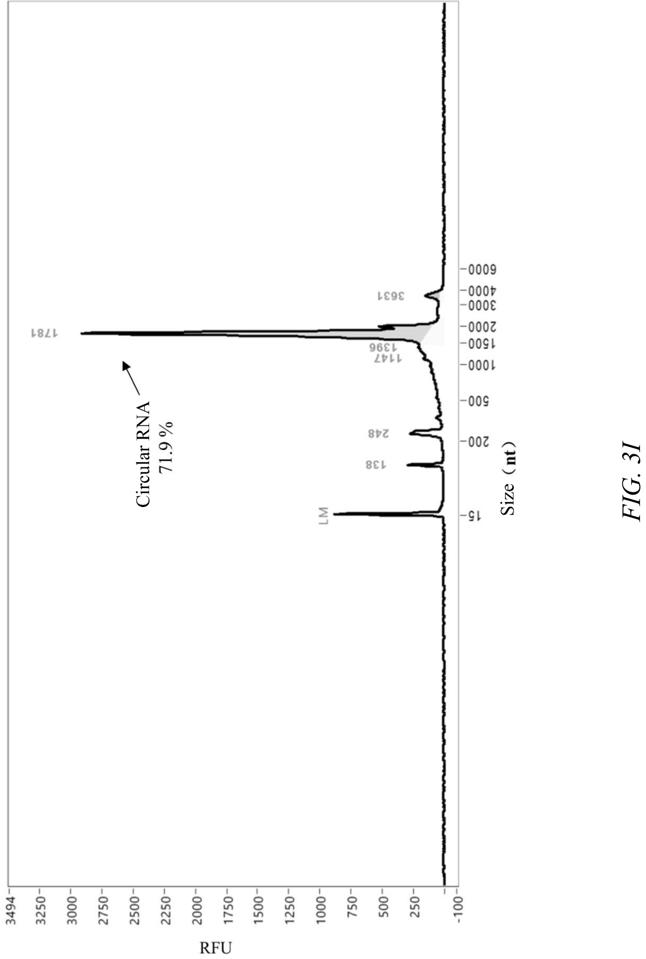

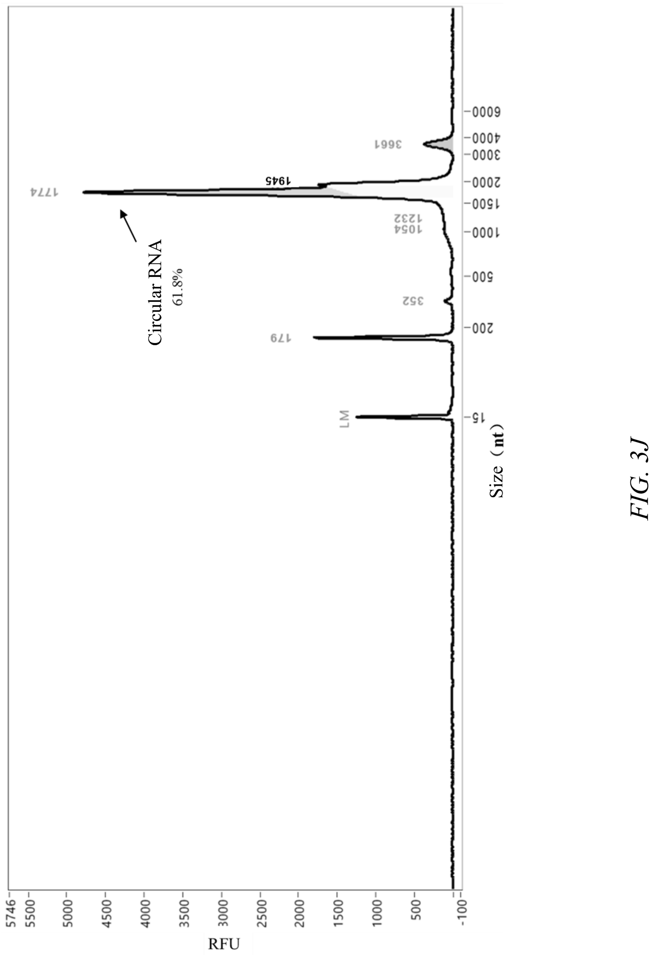

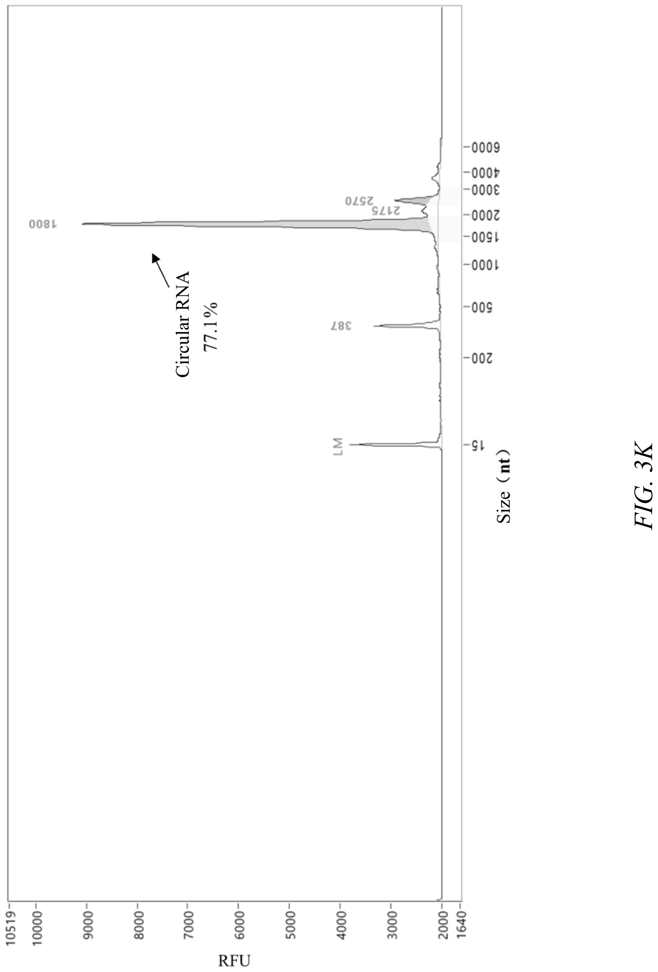

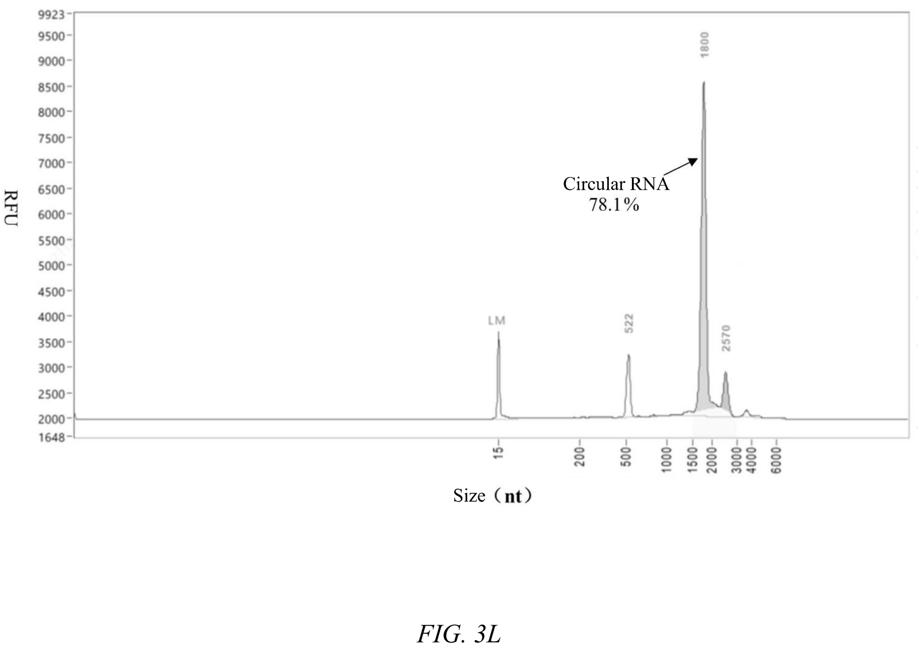

FIGs. 3A-3L show the purity of the circular RNAs obtained in the transcription and circularization step of the vectors comprising the *Anabaena* intron (3A), the *Synechocystis sp.* tRNA-fMet intron (truncated at P3, 3B), the *Coxiella burnetii* 23S ribosomal RNA intron (truncated at P4, 3C), the *Thermotoga naphthophila* 23S rRNA intron (truncated at P1, 3D), the *Bacillus anthracis* recA intron (truncated at P2, 3E), the *Bacillus anthracis* recA intron (truncated at P3, 3F), the *Coxiella burnetii* 23S ribosomal RNA gene intron (truncated at P1, 3G), the *Coxiella burnetii* 23S ribosomal RNA gene intron (truncated at P2, 3H), the *Scytonema hofmanni* tRNA-fMet gene intron (truncated at P3, 3I), the *Synechococcus elongatus* ndhL and trnL-UAA gene intron (truncated at P1, 3J), the *Thermotoga subterranea* 23S rRNA gene intron (truncated at P2, 3K) and the *Thermotoga subterranea* 23S rRNA gene intron (truncated at P3, 3L), as detected by capillary gel electrophoresis.

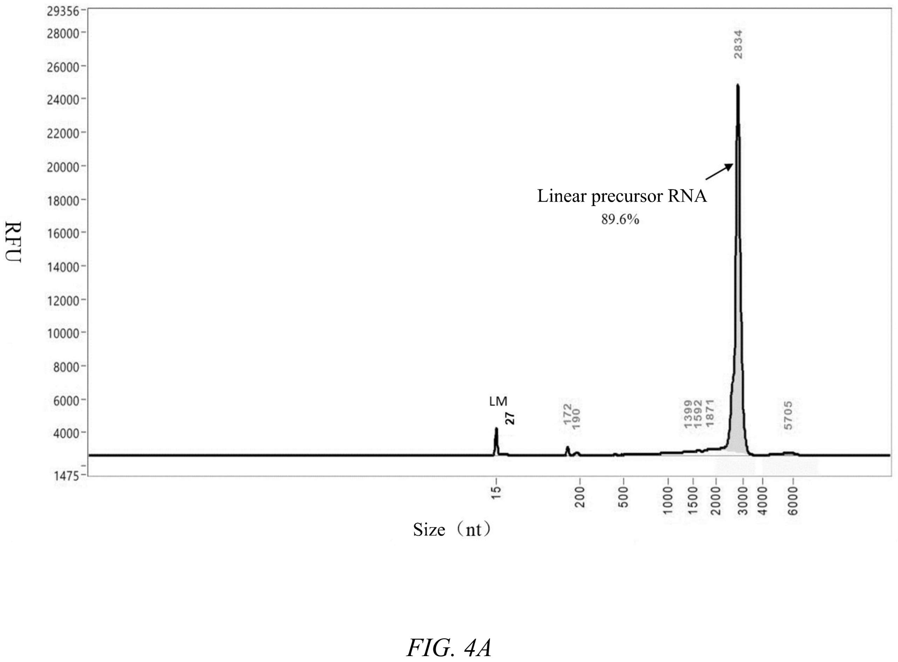

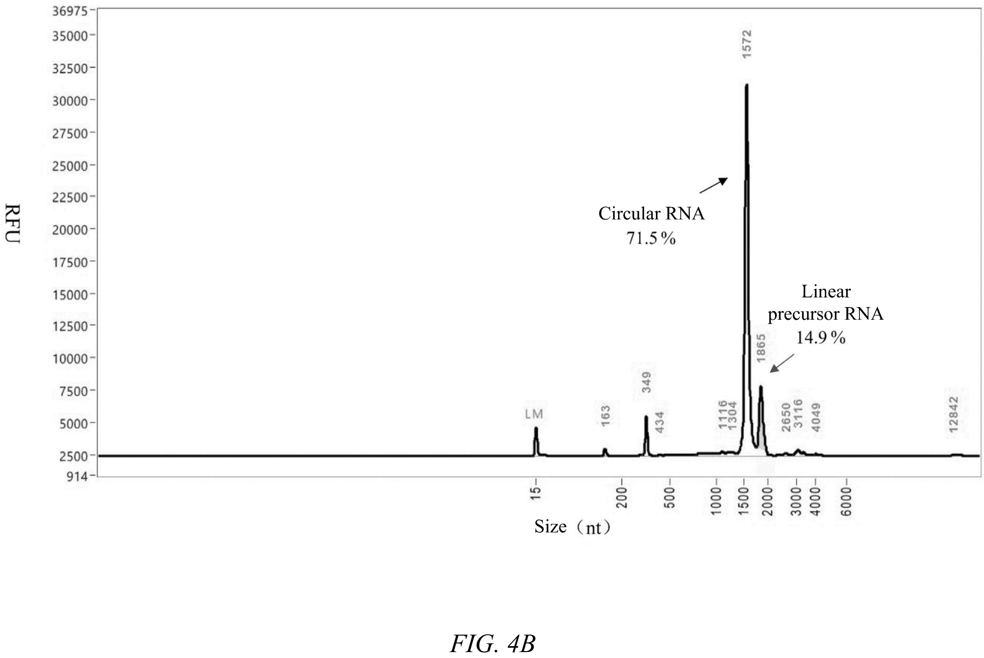

FIGs. 4A and 4B show the purity of the circular RNA obtained in the co-transcriptional circularization step of the vectors comprising the *Anabaena* intron (4A) and the *Bacillus anthracis* recA intron (truncated at P3, 4B), as detected by capillary gel electrophoresis.

### Detailed Description of Embodiments

The present application relates to a vector for preparing a circular RNA, comprising: a) an optional 5' homology arm, b) a 3' self-splicing intron fragment containing a 3' splice site, c) an optional 5' spacer sequence, d) an optional internal ribosome entry site (IRES) encoding sequence, e) a target gene, f) an optional 3' spacer sequence, g) a 5' self-splicing intron fragment containing a 5' splice site, and h) an optional 3' homology arm, wherein the elements are operatively connected in sequence.

The term "vector" refers to a naturally occurring or synthetic DNA fragment, including single-stranded and double-stranded DNA fragments, such as chemically synthesised DNA fragments, natural plasmids, or engineered viral genomes. A vector can have an exogenous DNA fragment inserted therein for cloning and/or expression of the exogenous DNA fragment. A vector may contain, for example, an origin of replication, a selectable marker or reporter gene, a multiple cloning site (MCS). The term includes linear DNA fragments (e.g., PCR products, linearized plasmid fragments), plasmid vectors, viral vectors, bacterial artificial chromosomes (BACs), yeast artificial chromosomes (YACs), etc. When the vector is double-stranded DNA, the description of the order of elements and the orientation of their sequences is directed to one of the DNA strands. In a double-stranded vector, the two DNA strands are substantially complementary or fully complementary, that is, at least about 75%, 80%, 85%, 90%, 95%, 96%, 97%, 98%, 99% or 100% base pairing is formed between the two DNA strands. The term "complementary" or "base pairing" refers to the connection of bases in the two DNA strands via hydrogen bonding and other interactions according to the A-T and C-G correspondences.

The elements within the vector are "operatively connected" in the sense that the elements can be transcribed *in vitro* or *in vivo* to form a precursor RNA product.

The term "precursor RNA", "IVT transcript", "messenger RNA", "mRNA" or "circularizable RNA" refers to an RNA product transcribed from the vector of the present application, which can be circularized under suitable conditions to form a circular RNA.

The term *"in vitro* transcription" or "IVT" refers to a process of simulating an *in vivo* transcription process with DNA as a template in an *in vitro* cell-free system under conditions containing an RNA transcriptase, NTP, etc. to form RNA. When a plasmid vector is used as a DNA template, the plasmid is subjected to enzyme cleavage at enzyme cleavage sites to linearize it before *in vitro* transcription.

Exons are sequences in DNA that are present in a mature RNA molecule. Exons are separated by introns and are connected together after the introns are removed by post-transcriptional processing. In contrast, introns are sequences that space between exons, are transcribed into precursor RNAs, are removed by splicing, and are ultimately not present in a mature RNA molecule. The removal of introns usually requires the participation of a spliceosome, which is a ribonucleoprotein complex dynamically composed of small nuclear RNAs (snRNAs, U1, U2, U4, U5, U6, etc.) and protein factors (about over 100 types), and which recognises the splice sites of precursor RNAs and catalyses the splicing reaction. However, a very small number of introns that form ribozymes undergo self-splicing, wherein the RNA alone performs the function of the splicing enzyme.

The term "self-splicing intron" in the present application refers to an intron that can undergo splicing independently of a spliceosome. The conditions for the splicing of self-splicing introns comprise the presence of GTP and magnesium ions. In some embodiments, the conditions for the splicing of self-splicing introns comprise: incubation at about 70°C for 5 minutes, placement on ice for 3 minutes, addition of GTP and Mg²⁺, and incubation at about 55°C for 8 minutes. Based on structure and splicing mechanism, self-splicing introns can be divided into two groups, group I and group II. The self-splicing intron in the present application refers primarily to group I introns.

The inventors screened introns derived from various organisms, and found that the introns from *Bacillus anthracis, Coxiella burnetii, Synechocystis sp., Thermotoga naphthophila., Scytonema hofmanii, Synechococcus elongatus* or *Thermotoga subterranea* (specifically the introns from the recA gene of *Bacillus anthracis,* the 23S ribosomal RNA gene of *Coxiella burnetii,* the tRNA-fMet gene of *Synechocystis sp.,* the 23S rRNA gene of *Thermotoga naphthophila,* the tRNA-fMet gene of *Scytonema hofmanii,* the ndhL and trnL-UAA genes of *Synechococcus elongatus,* or the 23S rRNA gene of *Thermotoga subterranea)* have great self-splicing capability. These introns are all group I self-splicing introns.

The self-splicing of an intron generally requires the assistance of exons at both ends. Therefore, in the preparation of the vector of the present application, partial exon sequences flanking the intron are also generally used. In particular, the above introns are truncated at different sites, the 3' end intron fragment and the exon fragment are placed on the 5' side of the vector (relative to one main DNA strand of the vector), and the 5' end exon fragment and the intron fragment are placed on the 3' side of the vector, with the target gene placed between them. The sequence of the exon is preferably one that naturally exists flanking the intron. The 3' intron fragment needs to contain a 3' splice site, and the 5' intron fragment needs to contain a 5' splice site. The 3' splice site and the 5' splice site can be dinucleotides, such as AG and GU, or AC and AU, respectively.

The 3' intron fragment can comprise a nucleotide sequence that has at least 75%, 80%, 85%, 90%, 91%, 92%, 93%, 94%, 95%, 96%, 97%, 98%, 99% or 100% sequence identity to the 3' fragment of a natural intron. In particular, the 3' intron fragment can comprise a nucleotide sequence that has at least 75%, 80%, 85%, 90%, 91%, 92%, 93%, 94%, 95%, 96%, 97%, 98%, 99% or 100% sequence identity to the 3' fragment of a natural intron and a 3' exon fragment. The 5' intron fragment can comprise a nucleotide sequence that has at least 75%, 80%, 85%, 90%, 91%, 92%, 93%, 94%, 95%, 96%, 97%, 98%, 99% or 100% sequence identity to the 5' fragment of a natural intron. In particular, the 5' intron fragment can comprise a nucleotide sequence that has at least 75%, 80%, 85%, 90%, 91%, 92%, 93%, 94%, 95%, 96%, 97%, 98%, 99% or 100% sequence identity to the 5' fragment of a natural intron and a 5' exon fragment.

The term "sequence identity" herein refers to the percentage of nucleotides/amino acids in a sequence that are identical to the nucleotides/amino acid residues in a reference sequence after a sequence alignment, and if desired, gaps are introduced in the sequence alignment to achieve the maximum percent sequence identity between the two sequences. A person skilled in the art can perform pairwise sequence alignment or multiple sequence alignment through various methods, such as computer software, to determine the percentage of sequence identity between two or more nucleic acid or amino acid sequences. Examples of such computer software include ClustalOmega, T-coffee, Kalign, MAFFT and the like.

The self-splicing introns screened are truncated at different sites to prepare 5' intron fragments and 3' intron fragments. It is found that intron fragments prepared by truncating the introns at given sites can achieve higher RNA circularization efficiency than the introns in the prior art, such as the *Anabaena* introns. Moreover, surprisingly, when the P3 truncation site of the *Bacillus anthracis* recA gene is selected, transcription and circularization can be completed simultaneously under *in vitro* transcription conditions alone. With regard to the truncation site of the intron, the inventors of the present application use RNA folding software, for example, RNAFold, to predict the folding configuration of the RNA transcript of the intron, select a suitable stem-loop (hairpin) structure from within the central region of the sequence, and perform truncation at the loop.

The term "homology arm" refers to i) an RNA sequence that is capable of forming at least about 25%, 50%, 75%, 80%, 85%, 90%, 95%, 96%, 97%, 98%, 99% or 100% base pairing with another RNA (such as another homology arm); ii) a DNA sequence encoding an RNA sequence that is capable of forming at least about 25%, 50%, 75%, 80%, 85%, 90%, 95%, 96%, 97%, 98%, 99% or 100% base pairing with an RNA sequence encoded by another DNA sequence (such as another homology arm). The "homology arm" in the circularizable RNA of the present application refers to i) described above, and the "homology arm" in the vector of the present application refers to ii) described above.

In certain embodiments, the 5' homology arm and the 3' homology arm in the vector of the present application can each be transcribed into a segment of RNA, and these two segments of RNA (corresponding to the 5' homology arm and the 3' homology arm in the circularizable RNA molecule), when brought into proximity with each other, are capable of: i) complementarily forming a stem-loop structure, wherein the 5' end of the transcript of the 5' homology arm and the 3' end of the transcript of the 3' homology arm are adjacent in the stem; ii) complementarily forming a structure containing a palindromic stem and a stem-loop, wherein the palindromic stem is connected to the stem of the stem-loop, wherein the 5' end of the transcript of the 5' homology arm and the 3' end of the transcript of the 3' homology arm are adjacent in the palindromic stem; or iii) complementarily forming a structure containing a palindromic stem and two stem-loops, wherein the palindromic stem is connected to the stems of the two stem-loops, wherein the 5' end of the transcript of the 5' homology arm and the 3' end of the transcript of the 3' homology arm are adjacent in the palindromic stem or in the loop of one of the stem-loops. Due to the presence of the palindromic structure and the selection of the junction position of the two arms, the structure of the intron ribozyme can be more stable, thereby improving the RNA circularization efficiency and the circular RNA yield. "Circular RNA" or "circRNA" is a type of RNA formed by covalent bonding (backsplicing) between the 5' end and the 3' end of a linear RNA. It can be naturally occurring or artificially prepared. Some circular RNAs can encode proteins, while others are non-coding RNAs. Circular RNAs, lacking a 5' end or a 3' end, are not cleaved by exonucleases and are thus more stable than linear RNAs. A broken form of a circular RNA molecule, resulting from hydrolysis or other factors, is referred to as "nicked RNA" herein. When the vector of the present application is used to prepare circular RNA, the proportion of nicked RNA is greatly reduced.

In one embodiment, the 5' homology arm can sequentially comprise, from the 5' end to the 3' end, a first sequence and a second sequence, and the 3' homology arm can sequentially comprise, from the 5' end to the 3' end, a third sequence, a fourth sequence, a fifth sequence and a sixth sequence, wherein the first sequence is the reverse complement of the sixth sequence, the second sequence is the reverse complement of the third sequence, and the fourth sequence is the reverse complement of the fifth sequence (FIG. 1B, upper panel); alternatively, the 5' homology arm sequentially comprises, from the 5' end to the 3' end, a first sequence, a second sequence, a third sequence and a fourth sequence, and the 3' homology arm sequentially comprises, from the 5' end to the 3' end, a fifth sequence and a sixth sequence, wherein the first sequence is the reverse complement of the sixth sequence, the fourth sequence is the reverse complement of the fifth sequence, and the second sequence is the reverse complement of the third sequence (FIG. 1B, lower panel). The term "reverse complement" refers to a sequence that, upon reversal of orientation, is capable of forming at least about 75%, 80%, 85%, 90%, 95%, 96%, 97%, 98%, 99%, or 100% base pairing with another sequence. For example, the first sequence, when read from the 3' end to the 5' end, can be complementarily paired with the sixth sequence read from the 5' end to the 3' end according to the rules of A-T and C-G. The 5' homology arm and the 3' homology arm can each be transcribed into a segment of RNA, and these two segments of RNA (corresponding to the 5' homology arm and the 3' homology arm in the circularizable RNA molecule), when brought into proximity with each other, are capable of: forming a palindromic stem of about 20-50 base pairs in length through complementary pairing between the portions corresponding to the second (or fifth) sequence and the third (or fourth) sequence, and forming a hairpin structure lacking a loop portion or a palindromic arm truncated at the turn through complementary pairing between the portions corresponding to the first (or sixth) sequence and the sixth (or first) sequence, wherein the complementary portion (the stem portion of the hairpin) is about 3-20 base pairs in length. In addition to the complementary pairing mentioned above, the portions corresponding to the fourth (or third) sequence and the fifth (or second) sequence in the RNAs transcribed from the 3' homology arm (FIG. 1B, upper panel) or the 5' homology arm (FIG. 1B, lower panel) also form a complete hairpin structure or palindromic arm, wherein the stem portion of the hairpin structure is about 3-20 base pairs in length, the loop portion is 1-15 nucleotides in length, and the sequence within the loop does not undergo complementary pairing. The term "complementary pairing" refers to complementary base pairing, i.e., the connection via hydrogen bonding or other interactions between bases of nucleotide residues in a nucleic acid molecule according to the A-T/A-U and C-G correspondence. In this way, in the RNA transcribed from the vector of the present application, the portions corresponding to the 5' homology arm and the 3' homology arm, in addition to forming, through base pairing with each other, a palindromic stem of about 20-50 base pairs in length and a hairpin structure lacking a loop portion or a palindromic arm having a complementary pairing region of about 3-20 base pairs in length, also form a complete hairpin structure or palindromic arm, the overall structure of which is close to an ITR, as shown in FIG. 1A. Although the hairpin structure or palindromic arm on the right side of FIG. 1A is incomplete, the presence of the palindromic arm on the left side and the closed conformation of the terminus opposite the palindromic stem (i.e., the junction of the two palindromic arms) may enable the precursor RNA, particularly the intron ribozyme therein, to have a more stable structure, thereby enhancing RNA circularization efficiency and circular RNA yield.

The 5' homology arm and the 3' homology arm can employ CpG-free sequences. CpG is an abbreviation for 5' cytosine (C)-phosphodiester bond (p)-guanine (G)-3'. DNA of bacteria and other organisms is rich in unmethylated CpG, whereas CpG in mammalian DNA is substantially methylated. Unmethylated CpG can activate the immune systems of vertebrates through TLR9, which recognises CpG-containing DNA, initiates signalling pathways, and induces the production of inflammatory cytokines such as IL-6 and IL-2. To reduce immune response, sequences that do not contain CpG or are engineered to remove CpG can be used.

The target gene in the vector of the present application can be a protein-coding gene or a non-coding gene. The target gene can be natural, or can be synthetic or engineered. The protein coding sequence can encode any protein. The protein coding sequence can encode a protein of eukaryotic or prokaryotic origin. The protein coding sequence can encode a protein of human origin or a protein of non-human origin. In some embodiments, the protein coding sequence can encode an antigenic protein, an antibody, or a Cas9 endonuclease. In some embodiments, the protein coding sequence can encode firefly luciferase or *Gaussia* luciferase. When the target gene is a non-coding gene, the circular RNA prepared from the vector can be, for example, a miRNA sponge or a non-coding RNA. The miRNA sponge can exist in the cytoplasm in the form of mRNA, and adsorb numerous miRNA molecules in the cytoplasm, exerting a miRNA-absorbing effect similar to that of a sponge. Non-coding RNAs refers to single-stranded RNAs that do not encode proteins and may play a regulatory role in processes such as DNA transcription and translation.

To facilitate the translation of protein coding sequences in a circular RNA, the vector of the present application can comprise an internal ribosome entry site (IRES) encoding sequence on one side, such as the 5' end, of the target gene. The term "internal ribosome entry site" or "IRES" refers to a nucleic acid sequence that enables the initiation of protein translation independent of the 5' cap structure, allowing translation to start from the middle of an mRNA. The IRES encoding sequence can comprise a nucleotide sequence that has at least 75%, 80%, 85%, 90%, 91%, 92%, 93%, 94%, 95%, 96%, 97%, 98%, 99% or 100% sequence identity to a natural IRES.

When the target gene is a protein coding sequence, the vector of the present application can comprise a spacer sequence between the 5' intron fragment and the IRES encoding sequence or the target gene. In particular, the vector of the present application can further comprise a spacer sequence between the target gene and the 3' intron fragment.

A spacer sequence refers to a sequence added to avoid mutual interference between adjacent or proximate, or even distant elements during processes such as transcription and RNA folding. Whether vector elements will interfere with each other during processes such as transcription and RNA folding can be predicted using computer software such as folding software (e.g., RNAFold). Spacer sequences can also be designed with the aid of computer software such as RNA folding software (e.g., RNAFold).

The vector of the present application can further comprise an RNA polymerase promoter upstream of the 5' homology arm for transcription of the vector. The RNA polymerase promoter can comprise a nucleotide sequence that has at least 75%, 80%, 85%, 90%, 91%, 92%, 93%, 94%, 95%, 96%, 97%, 98%, 99% or 100% sequence identity to a natural RNA polymerase promoter.

The vector of the present application can further comprise a restriction endonuclease site downstream of the 3' homology arm for linearization of the vector. In transcription of the vector, especially *in vitro* transcription, the vector needs to be linearized. The vector can be linearized by digesting at the restriction endonuclease site mentioned above. In some embodiments, the restriction endonuclease site in the vector is unique.

The circularizable RNA of the present application (including the precursor RNA prepared from the vector of the present application) can sequentially comprise from the 5' end to the 3' end: a) an optional 5' homology arm, b) a 3' self-splicing intron fragment containing a 3' splice site, c) a 5' spacer sequence, d) an internal ribosome entry site (IRES), e) a target gene, f) a 3' spacer sequence, g) a 5' self-splicing intron fragment containing a 5' splice site, and h) an optional 3' homology arm; or a) an optional 5' homology arm, b) a 3' self-splicing intron fragment containing a 3' splice site, c) a 5' spacer sequence, d) an internal ribosome entry site (IRES), e) a target gene, f) a 5' self-splicing intron fragment containing a 5' splice site, and g) an optional 3' homology arm, wherein the target gene is a protein coding sequence, and the elements are operatively connected.

The circularizable RNA of the present application (including the precursor RNA prepared from the vector of the present application) can sequentially comprise from the 5' end to the 3' end: a) an optional 5' homology arm, b) a 3' self-splicing intron fragment containing a 3' splice site, c) a target gene, d) a 5' self-splicing intron fragment containing a 5' splice site, and e) an optional 3' homology arm, wherein the target gene is a non-coding sequence, and the elements are operatively connected.

The 3' self-splicing intron fragment containing the 3' splice site and the 5' self-splicing intron fragment containing the 5' splice site in the circularized RNA can be derived from the recA gene of *Bacillus anthracis,* the 23S ribosomal RNA gene of *Coxiella burnetii,* the tRNA-fMet gene of *Synechocystis sp.,* the 23S rRNA gene of *Thermotoga naphthophila,* the tRNA-fMet gene of *Scytonema hofmanni,* the ndhL and trnL-UAA genes of *Synechococcus elongatus,* or the 23S rRNA gene of *Thermotoga subterranea,* and have great self-splicing capability. By truncating each intron at different sites, the RNA sequence corresponding to the 3' intron fragment and the exon is used as a 3' self-splicing intron fragment containing a 3' splicing site, and the RNA sequence corresponding to the 5' exon and the intron fragment is used as a 5' self-splicing intron fragment containing a 5' splice site. The 3' splice site and the 5' splice site can be dinucleotides, such as AG and GU, or AC and AU, respectively.

The 3' intron fragment can comprise a corresponding RNA sequence of a nucleotide sequence that has at least 75%, 80%, 85%, 90%, 91%, 92%, 93%, 94%, 95%, 96%, 97%, 98%, 99% or 100% sequence identity to a 3' fragment of a natural intron (and a 3' exon fragment). The 5' intron fragment can comprise a corresponding RNA sequence of a nucleotide sequence that has at least 75%, 80%, 85%, 90%, 91%, 92%, 93%, 94%, 95%, 96%, 97%, 98%, 99% or 100% sequence identity to a 5' fragment of a natural intron (and a 5' exon fragment). The term "corresponding RNA sequence" refers to an RNA sequence obtained by replacing T with U in a DNA sequence.

The term "homology arm" in the circularized RNA refers to an RNA sequence that is capable of forming at least about 25%, 50%, 75%, 80%, 85%, 90%, 95%, 96%, 97%, 98%, 99% or 100% base pairing with another RNA (such as another homology arm). The 5' homology arm and the 3' homology arm in the circularized RNA, when brought into proximity with each other, are capable of: i) complementarily forming a stem-loop structure, wherein the 5' end of the 5' homology arm and the 3' end of the 3' homology arm are adjacent in the stem; ii) complementarily forming a structure containing a palindromic stem and a stem-loop, wherein the palindromic stem is connected to the stems of the stem-loop, wherein the 5' end of the 5' homology arm and the 3' end of the 3' homology arm are adjacent in the palindromic stem; or iii) complementarily forming a structure containing a palindromic stem and two stem-loops, wherein the palindromic stem is connected to the stem of the two stem-loops, wherein the 5' end of the 5' homology arm and the 3' end of the 3' homology arm are adjacent in the palindromic stem or in the loop of one of the stem-loops.

In one embodiment, the 5' homology arm can sequentially comprise, from the 5' end to the 3' end, a first sequence and a second sequence, and the 3' homology arm can sequentially comprise, from the 5' end to the 3' end, a third sequence, a fourth sequence, a fifth sequence and a sixth sequence, wherein the first sequence is the reverse complement of the sixth sequence, the second sequence is the reverse complement of the third sequence, and the fourth sequence is the reverse complement of the fifth sequence (FIG. 1B, upper panel); alternatively, the 5' homology arm sequentially comprises, from the 5' end to the 3' end, a first sequence, a second sequence, a third sequence and a fourth sequence, and the 3' homology arm sequentially comprises, from the 5' end to the 3' end, a fifth sequence and a sixth sequence, wherein the first sequence is the reverse complement of the sixth sequence, the fourth sequence is the reverse complement of the fifth sequence, and the second sequence is the reverse complement of the third sequence (FIG. 1B, lower panel). The term "reverse complement" refers to a sequence that, upon reversal of orientation, is capable of forming at least about 25%, 50%, 75%, 80%, 85%, 90%, 95%, 96%, 97%, 98%, 99%, or 100% base pairing with another sequence. For example, the first sequence, when read from the 3' end to the 5' end, can be complementarily paired with the sixth sequence read from the 5' end to the 3' end according to the rules of A-U and C-G. The 5' homology arm and the 3' homology arm in the circularizable RNA, when brought into proximity with each other, are capable of: forming a palindromic stem of about 20-50 base pairs in length through complementary pairing between the second (or fifth) sequence and the third (or fourth) sequence, and forming a hairpin structure lacking a loop portion or a palindromic arm truncated at the turn through complementary pairing between the first (or sixth) sequence and the sixth (or first) sequence, wherein the complementary portion (the stem portion of the hairpin) is about 3-20 base pairs in length. In addition to the complementary pairing mentioned above, the fourth (or third) sequence and the fifth (or second) sequence in the 3' homology arm (FIG. 1B, upper panel) or the 5' homology arm (FIG. 1B, lower panel) also form a complete hairpin structure or palindromic arm, wherein the stem portion of the hairpin structure is about 3-20 base pairs in length, the loop portion is 1-15 nucleotides in length, and the sequence within the loop does not undergo complementary pairing.

The 5' spacer sequence and/or the 3' spacer sequence can be 5-70 nucleotides in length, which spacer sequence is used to prevent mutual interference between adjacent or proximate, or even distant elements during processes such as transcription and RNA folding.

The circularizable RNA molecule of the present application can be obtained by transcription using the vector of the present application.

The present application further provides a circular RNA, which can be obtained using a vector of the present application by a method for preparing a circular RNA of the present application; or can be obtained by treating the circularizable RNA molecule of the present application under suitable conditions, wherein the suitable conditions comprise the presence of GTP and magnesium ions. The circular RNA can be about 500 to about 10000 nucleotides in length.

The present application provides a composition, which can comprise the single-stranded DNA molecule, the double-stranded DNA molecule, the vector, the circularizable RNA molecule, or the cell comprising the single-stranded DNA molecule, double-stranded DNA molecule, the vector, or the circularizable RNA molecule of the present application. The composition can be used to prepare a circularizable RNA and/or a circular RNA, particularly for producing a circular RNA that can be translated into a protein or a biologically active circular RNA *in vitro* or in a cell. The biologically active circular RNA can be, for example, a miRNA sponge or a non-coding RNA.

The present application further provides a composition, which can comprise a circular RNA molecule or a cell comprising a circular RNA molecule of the present application. The composition can be a pharmaceutical composition, and can further comprise a pharmaceutically acceptable carrier, excipient or diluent.

The pharmaceutically acceptable carrier can be a component in the composition other than the active ingredient (i.e., the vector, the cell, the precursor RNA, or the circular RNA of the present application). The pharmaceutically acceptable carrier can include, but is not limited to, a buffer, an excipient, a stabilizer, or a preservative. Examples of pharmaceutically acceptable carriers are solvents, dispersion media, coatings, antibacterial and antifungal agents, isotonic and absorption delaying agents, etc. that are physiologically compatible, such as salts, buffers, saccharides, antioxidants, aqueous or non-aqueous carriers, preservatives, wetting agents, surfactants or emulsifying agents, or combinations thereof. An amount of the pharmaceutically acceptable carrier in a pharmaceutical composition can be determined experimentally based on an activity of the carrier and the desired properties of the preparation, such as stability and/or minimal oxidation.

In certain embodiments, the composition of the present application can comprise buffers such as acetic acid, citric acid, histidine, boric acid, formic acid, succinic acid, phosphoric acid, carbonic acid, malic acid, aspartic acid, Tris buffers, HEPPSO, HEPES, neutral buffered saline, phosphate buffered saline; carbohydrates such as glucose, sucrose, mannose, or dextran, mannitol; proteins; polypeptides or amino acids, such as glycine; antioxidants; chelating agents such as EDTA or glutathione; adjuvants (e.g., aluminium hydroxide); antibacterial and antifungal agents; and preservatives.

The present application also provides the use of the composition for the preparation of a circular RNA, or for *in vivo* therapy, etc. For example, in one embodiment, the present application provides a method for expressing a vaccine, a therapeutic protein, or other types of proteins in a subject in need thereof, comprising administering to the subject a composition comprising a circular RNA of the present application. The therapeutic protein can be, for example, an antibody or a fusion protein. In one embodiment, the subject is a subject with a loss of a functional gene, and the circular RNA in the composition of the present application can function as the RNA transcribed from the missing gene, or can continuously express a protein translated from the missing gene *in vivo.*

The term "subject" includes any human or non-human animal. The term "non-human animal" includes all vertebrates, for example, mammals and non-mammals, such as non-human primates, sheep, dogs, cats, cattle, horses, chickens, amphibians, and reptiles, although mammals are preferred, such as non-human primates, sheep, dogs, cats, cattle, and horses.

When the circular RNA translationally expresses a therapeutic protein, or exerts a therapeutic effect, a therapeutically effective amount of a composition comprising the circular RNA is administered to a subject. A "therapeutically effective amount" of the compositions of the present application preferably causes a reduction in severity of disease symptoms, an increase in frequency and duration of symptom-free periods, or prevention of damage or disability caused by the disease. For example, for the treatment of tumour-bearing subjects, a "therapeutically effective amount" refers to, relative to an untreated subject, preferably at least about 40% inhibition of tumour growth, more preferably at least about 60% inhibition, more preferably at least about 80% inhibition, and more preferably at least about 99% inhibition. A therapeutically effective amount of the fusion protein of the present application can reduce tumour volume or alleviate symptoms in a subject (usually a human, or another mammal).

When necessary, the precursor RNA or circular RNA in the present application can be purified. For example, when the composition of the present application is administered to a subject, the circular RNA is preferably purified. Circular RNA or precursor RNA can be purified by a size exclusion column in a high-performance liquid chromatography (HPLC) system.

The administration of the pharmaceutical composition can be specifically determined by a medical worker, such as a physician, based on specific conditions of the subject, such as gender, age, and past medical history.

The pharmaceutical composition of the present application can be formulated for oral, intravenous, intramuscular, subcutaneous, parenteral, spinal or epidermal administration (e.g., by injection or bolus). The term "parenteral administration" refers to a mode other than enteral and topical administration, usually performed by injection, including but not limited to intravenous, intramuscular, intraarterial, intrathecal, intracapsular, intraorbital, intracardiac, intradermal, intraperitoneal, transtracheal, subcutaneous, subepidermal, intraarticular, subcapsular, subarachnoid, intraspinal, epidural and intrasternal injection and bolus. In one embodiment, the composition may be formulated for infusion or intravenous administration. The composition disclosed herein can be provided, for example, as a sterile liquid preparation, such as an isotonic aqueous solution, an emulsion, a suspension, a dispersion, or a viscous composition, which may be buffered to a desirable pH. In certain embodiments, the administration of the compositions may be performed in any manner, for example, by parenteral or non-parenteral administration, including by aerosol inhalation, injection, infusion, ingestion, transfusion, implantation or transplantation. For example, the composition described herein can be administered to a patient intravenously, intranasally, intrathecally, intraperitoneally, transarterially, intradermally, subcutaneously, intratumorally, intramedullary, intranodally, or intramuscularly.

The composition of the present application can be transfected into cells via, for example, lipofection, electroporation, or nanocarrier encapsulation. The nanocarriers can be, for example, lipids, polymers or lipid-polymer hybrids.

The composition of the present application can be delivered to a subject via a release delivery system. Release delivery systems include polymer-based systems, such as poly(lactide-glycolide), copolyoxalates, polyesteramides, polyorthoesters, polycaprolactones, polyhydroxybutyric acid, and polyanhydrides. Delivery systems also include non-polymer systems that are lipids, including sterols, such as cholesterol, cholesterol esters, and fatty acids or neutral fats, such as mono-di- and tri-glycerides; peptide-based systems; hydrogel release systems; wax coatings; compressed tablets using conventional binders and excipients; partially fused implants; etc. In certain embodiments, lipid nanoparticles or polymers are used as delivery vehicles for the therapeutic circular RNAs described herein, including delivery of RNA to tissues.

The pharmaceutical composition can be a sustained-release agent, including implants and microencapsulated delivery systems. Biodegradable, biocompatible polymers can be used, such as ethylene-vinyl acetate, polyanhydride, poly(glycolic acid), collagen, polyorthoester, and poly(lactic acid). See, e.g., Sustained and Controlled Release Drug Delivery Systems, J. R. Robinson, ed., Marcel Dekker, Inc., New York, 1978. The pharmaceutical composition can be administered via a medical device, such as (1) a needle-free subcutaneous injection device (e.g., U.S. Patents 5,399,163; 5,383,851; 5,312,335; 5,064,413; 4,941,880; 4,790,824 and 4,596,556); (2) a microinfusion pump (U.S. Patent 4,487,603); (3) a transdermal drug delivery device (U.S. Patent 4,486,194); (4) a bolus device (U.S. Patents 4,447,233 and 4,447,224); and (5) an osmotic device (U.S. Patents 4,439,196 and 4,475,196).

The composition of the present application can be used in combination with other therapeutic agents, vaccines, etc. The combination of therapeutic agents discussed herein can be administered simultaneously as a single composition in a pharmaceutically acceptable carrier, or administered simultaneously as separate compositions with each agent in a pharmaceutically acceptable carrier. In another embodiment, the combination of therapeutic agents can be administered sequentially.

Furthermore, if multiple combination therapy administrations are performed and the agents are administered sequentially, the order of sequential administration at each time point may be reversed or maintained the same, and the sequential administration may be combined with simultaneous administration or any combination thereof.

The present application will be further illustrated with reference to the following non-limiting examples.

### Examples

### Example 1. Construction of vector for producing circularizable RNA using inverted terminal repeat (ITR)

Adeno-associated virus (AAV) is a single-stranded DNA virus belonging to the genus *Dependoparvovirus* ^{[25-27]}. Recombinant AAV vectors are considered to be ideal gene therapy vectors due to their safety, high efficiency and stability, and have been widely used in clinical applications ^{[28, 29]}. The genome of an AAV vector contains one expression cassette and two inverted terminal repeats (ITRs). The ITR sequence consists of two palindromic arms (stem-loop) and a long palindromic stem ^{[30, 31]}.

The inventors of the present application believe that if this highly complementary palindromic structure is applied to RNA circularization, it may be possible to enhance the circularizability of RNAs by shortening the distance between the RNA ends and maintaining the stability of the self-splicing intron ribozyme during the circularization process.

Therefore, based on the disclosure of Daniel G. et al. ^{[21]}, two ITR sequences, namely wild-type ITR ^{[31]} and CpG motif-free ITR ^{[32]}, are used to replace the homology arms described therein to construct a vector for preparing a circularizable RNA. The strategy for constructing vectors according to the publication of Daniel G. is also referred to as the "published strategy", whereas the vector construction strategy described herein is also referred to as the "ITR strategy".

According to the ITR strategy of the present application, each ITR is divided into two segments, and the 3' end sequence is used as the 5' ITR, and the 5' end sequence is used as the 3' ITR, replacing the 5' homology arm and 3' homology arm in the strategy of Daniel G. et al. ^{[21]}, respectively (FIG. 1B, upper panel). Alternatively, the 5' end sequence can be used as the 5' ITR and the 3' end sequence can be used as the 3' ITR, replacing the 5' homology arm and 3' homology arm in the strategy of Daniel G. et al. ^{[21]}, respectively (FIG. 1B, lower panel). Taking the design in the upper panel of FIG. 1B as an example, the transcript of the 3' ITR includes the 5' palindromic arm and one side of the 3' palindromic arm, and the transcript of the 5' ITR includes the other side of the 3' palindromic arm with the turn of the right palindromic arm as the boundary, as shown in the schematic diagram of the intact ITR transcript in FIG. 1A.

### 1.1 Vector construction

With reference to the article published by Daniel G. ^{[21]}, a control vector and a vector for preparing a circularizable RNA in the present application were constructed.

Specifically, with regard to the construction of the control vector, a DNA fragment was synthesised, the fragment sequentially comprising from the 5' end to the 3' end: a T7 promoter (SEQ ID NO: 1), a 5' homology arm (SEQ ID NO: 2), a 3' intron and exon E2 (SEQ ID NO: 3), a 5' spacer sequence (SEQ ID NO: 4), a CVB3 IRES element (SEQ ID NO: 5), a firefly luciferase (FLUC) encoding gene (SEQ ID NO: 6), a 3' spacer sequence (SEQ ID NO: 9), a 5' intron and exon E1 (SEQ ID NO: 10), a 3' homology arm (SEQ ID NO: 11), and an EcoRV restriction enzyme site for plasmid linearization (GATATC), wherein the intron was an *Anabaena* intron, and the exons E1 and E2 were sequences required for the self-splicing function of the *Anabaena* intron. The DNA fragment synthesised above was cloned into a pUC57 vector (SEQ ID NO: 12), which was digested with EcoRV to obtain a linearized vector product.

With regard to the vector for preparing a circularizable RNA in the present application, the construction method was the same as above, except that the 5' homology arm and the 3' homology arm were replaced with a 5' ITR sequence and a 3' ITR sequence, respectively. The specific structure can be seen in FIG. 1B (upper panel). The 5' ITR sequence and the 3' ITR sequence derived from the wild-type ITR are shown in SEQ ID NOs: 13 and 14, respectively, and the engineered CpG-free 5' ITR and 3' ITR sequences are shown in SEQ ID NOs: 15 and 16, respectively. The complementary sequences in the 5' ITR and the 3' ITR are shown underlined, and the palindromic arm-forming sequences are shown in bold italics.
5' ITR and 3' ITR derived from wild type: 
GCCCGGGCGGCCTCAGTGAGCGAGCGAGCGCGCAGAGAGGGAGTGGC
CAAC (SEQ ID NO: 13)
 
GTTGGCCACTCCCTCTCTGCGCGCTCGCTCGCTCACTGAGGCCGGGCGA
CCAAAGGTCGCCCGACGCCCGGGC (SEQ ID NO: 14)
CpG-free 5'ITR and 3'ITR:

GGTCCCCAGGCCTCAGTGAGTGAGTGAGTGAGC (SEQ ID NO: 15)
GCTCACTCACTCACTCACTGAGGCCTGCAGAGCAAAGCTCTGCAGTCTG
GGGACC (SEQ ID NO: 16)

The gene synthesis, vector construction, and plasmid linearization described above were all completed by GenScript.

### 1.2 In vitro transcription

The linearized plasmid vector obtained above was subjected to *in vitro* transcription (IVT) to obtain RNA products. Specifically, the plasmid vector was incubated in the reaction system shown in Table 1 at 37°C for 2 h. Then, 2 µl of DNase I (RNase-free, ON-109, Hongene) was added, and the system was mixed uniformly and incubated at 37°C for 30 min.

**Table 1. Reaction system for in vitro transcription**

| Reagents | Catalog number/manufacturer | Volume |
|---|---|---|
| 10× T7 transcription buffer | 22020420SR01, GenScript | 2 µl |
| ATP (100 mM) | ATP001, SYNTHGENE | 2 µl |
| UTP (100 mM) | UTP001, SYNTHGENE | 2 µl |
| CTP (100 mM) | CTP001, SYNTHGENE | 2 µl |
| GTP (100 mM) | GTP001, SYNTHGENE | 2 µl |
| RNase inhibitor (40 U/µl) | DD4102-PA-02, Vazyme | 0.3 µl |
| Inorganic pyrophosphatase (0.1 U/µl) | DD4103-PC-02, Vazyme | 0.3 µl |
| T7 RNA polymerase | GenScript | 0.5 µl |
| Linearized plasmid | Obtained in 1.1 | 1 µg |
| Ambion^{™} nuclease-free water | AM9937, Thermo Fisher | Up to 20 µl |

### 1.3 RNA purification by LiCl precipitation

The RNA product obtained above was purified by LiCl precipitation. Specifically, 22 µl of nuclease-free water (AM9937, Thermo Fisher) and 20 µl of 8 M LiCl solution (DNase- or RNase-free, ST498-100 ml, Beyotime) were added to the IVT stock solution, bringing the LiCl concentration to 2.5 M. The mixture was mixed uniformly and incubated at -20°C for at least 30 min. The mixture was centrifuged at 12000 g for 15 min at 4°C, and the supernatant was discarded. 500 µl of 70% ethanol was added, and the system was inverted to ensure uniform mixing, centrifuged at 12000 g at 4°C for 3 min, the supernatant was discarded; and this step was repeated. Centrifugation was performed at 12000 g at 4°C for 1 min, all the supernatant was pipetted off, and 100 µl of nuclease-free water was added to dissolve the RNA.

The concentration of purified RNA was detected using a Nanodrop ONEC micro-volume UV-visible spectrophotometer (ND-ONEC-W, Thermo scientific).

### 1.4 RNA circularization

The purified mRNA obtained above was subjected to circularization treatment (one-step circularization method). Specifically, 20 µg of purified RNA was taken, adjusted to about 0.5-2 µg/µl with nuclease-free water, incubated at 70°C for 5 min, and then immediately placed on ice for 3 min. GTP (100 mM) was added to reach a final concentration of 2 mM, and Mg²⁺-containing T4 RNA ligase buffer (10×) (B0216S, NEB) was added to bring the final Mg²⁺ concentration to 10 mM. The system was incubated at 55°C for 8 min.

The product was purified by LiCl precipitation as described above, and the concentration of the purified RNA was detected by Nanodrop.

### 1.5 Analysis using gel electrophoresis

The purified circularized RNA product described above was analysed by E-Gel^{™} EX gel electrophoresis. Specifically, 150-200 ng of purified RNA was taken and adjusted to 10 µl with nuclease-free water, and then 10 µl of gel loading buffer II (AM8546G, ThermoFisher) was added and the mixture was mixed uniformly. E-Gel^{™} EX agarose gel (2%, G401002, ThermoFisher) was taken, the comb was removed, and the gel was placed into the gel tank of the electrophoresis system. 20 µl of the uniformly mixed sample was added to each of the gel wells. Electrophoresis was started. The electrophoresis time was set to 10-20 min. After the electrophoresis, the filter was opened, and cooling was performed for 5-10 min. The electrophoresis results were observed using the built-in image acquisition system of the E-Gel^{™} EX device (G8300, ThermoFisher), and the images were acquired.

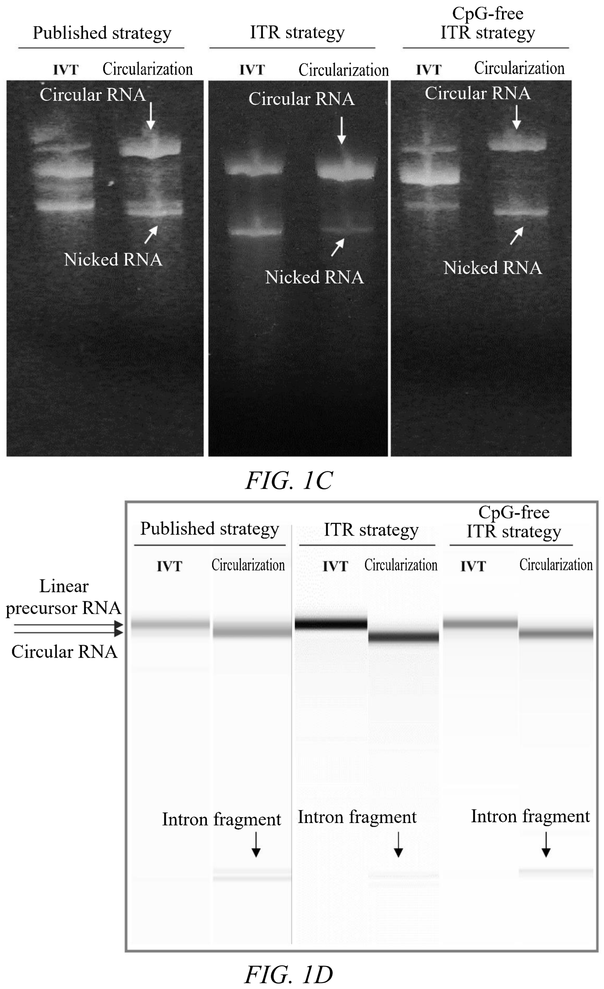

In the E-Gel^{™} EX gel electrophoresis image, circular RNA usually shows the lowest migration rate, followed by precursor linear RNA, while nicked RNA (nicked circular RNA) migrates the fastest. The circularization effects of RNA prepared according to the published strategy and the ITR strategy of the present application are shown in FIG. 1C. Specifically, the band of the circular RNA prepared by the ITR strategy of the present application exhibited higher purity, with no significant residual precursor linear RNA bands or interference from other bands. The content of nicked RNA byproducts (the lowest band in the lane in the figure) was also lower, demonstrating that the ITR construction strategy can enhance the purity of circular RNA and reduce the content of nicked RNA byproducts.

### 1.6 Capillary gel electrophoresis

To detect the RNA circularization efficiency, the RNA before and after circularization was subjected to capillary gel electrophoresis using an Agilent 5200 fragment analyser (M53100AA, Agilent) and an Agilent DNF-471 RNA kit (15 nt, DNF-471-0500, Agilent).

Specifically, the purified RNA from 1.3 and 1.4 was taken, adjusted with nuclease-free water to a concentration of 50-100 ng/µl, and mixed with an RNA ladder and reacted at 80°C for 2 min. After the reaction was completed, the reaction system was immediately placed on a 4°C ice box for instant cooling. 22 µL of diluent standard (RNA diluent standard), 2 µL of test sample, and RNA ladder were added to the sample plate, respectively. Capillary electrophoresis experiment was performed, and reference can be made to the instruction manual of the Agilent 5200 fragment analyser for the specific device operation methods.

The results are shown in FIG. 1D. It can be seen from the band size that the band of the *in vitro* transcription product before the circularization is slightly larger than that of the circular RNA after the circularization. This is because the linear precursor RNA contains a self-splicing intron sequence, and the intron is excised during the circularization process, resulting in a free intron fragment. In addition, the circular RNA band produced by the published strategy had an upper band of uncircularized precursor RNA, whereas the circular RNA produced by the ITR strategy showed no obvious residual precursor band above it (all the samples were not digested with RNase R, which can specifically eliminate linear RNA). It can be seen that ITR is superior to the homology arm in the published strategy in bringing the two ends of the mRNA into close proximity to assist the circularization reaction, representing a more ideal circularization strategy.

### 1.7 Sequencing of circularized RNA

The RNA obtained in 1.4 was subjected to reverse transcription, PCR, and gel extraction, followed by sequencing of the circularization junction site.

The products obtained from the *in vitro* self-splicing reaction described above were reverse transcribed using the HiScript^{®} II 1st Strand cDNA synthesis kit (+ gDNA wiper) (R212-01, Vazyme). Specifically, the reaction system was prepared as shown in Table 2, mixed uniformly by pipetting, and heated at 42°C for 2 min.

**Table 2. Reverse transcription reaction system**

| | |
|---|---|
| 4 × gDNA wiper mixture | 4 µl |
| RNA | 50 ng |
| RNase-free ddH₂O | Up to 16 µl |

Then, 4 µl of 5 × HiScript II qRT SuperMix II was added to the mixture described above, and the resulting mixture was mixed uniformly by pipetting and reacted at 50°C for 15 min and 85°C for 5 sec.

After the reverse transcription was completed, the obtained cDNA was added to the PCR reaction system shown in Table 3 and the resulting mixture was mixed uniformly by pipetting. Then the PCR reaction was performed according to the program shown in Table 4.

**Table 3. PCR reaction system**

| | |
|---|---|
| 5 × SF buffer | 10 µl |
| dNTP mixture | 1 µl |
| Upstream primer (SEQ ID NO: 26, 10 µM) | 2 µl |
| Downstream primer (SEQ ID NO: 27, 10 µM) | 2 µl |
| cDNA | 1 µl |
| Phanta super-fidelity DNA polymerase | 1 µl |
| ddH₂O | Up to 50 µl |

**Table 4. PCR program setting**

| Temperature | Time | Number of cycles |
|---|---|---|
| 95°C | 3 min | 1 |
| 95°C | 10 sec | 30 |
| 60°C | 10 sec | |
| 72°C | 45 sec | |
| 72°C | 5 min | 1 |
| 8°C | ∞ | 1 |

All the reaction products were subjected to gel electrophoresis and extraction. The target bands were excised using a TGreen Transilluminator (OSE-470L, Tiangen Biotech Co., Ltd.). Gel extraction was performed according to the instruction manual of the E.Z.N.A.^{®} gel extraction kit (D2500-01/D2500-02, Omega). The extracted products were sent for Sanger sequencing, and the sequences of the sequencing primers for circularization junction were SEQ ID NOs: 26 and 27.

The sequencing results of the RNA circularization junction sites are shown in SEQ ID NO: 29, and the sequences of the 3' spacer sequence (in bold italics), E1 (underlined), E2 (in italics) and the 5' spacer sequence (in bold and underlined) are represented differently. It can be seen that the intron has been removed, E1 and E2 are connected together.

AACGGCTATTATGCGTTACCGGCGAGACGCTACGGACTTAAAATCCGTTG
ACCTTAAACGGTCGTGTGGGTTCAAGTCCCTCCACCCCCACGCCGGAAAC
GCAATAGCCGaaaaacaa (SEQ ID NO: 29)

### Example 2. Screening of introns having in vitro self-splicing capability

Currently, RNA circularization is primarily based on the self-splicing of group I and group II introns. To achieve higher RNA circularization efficiency, self-splicing introns are screened.

First, to obtain an intron having *in vitro* self-splicing capability, the EGFP coding sequence (SEQ ID NO: 8, used as a nonsense sequence for detecting intron splicing) was truncated and loaded onto both ends of the candidate intron (FIG. 2A). Specifically, a DNA sequence was synthesised, the sequence comprising, from the 5' end to the 3' end: a T7 promoter (SEQ ID NO: 1); a 5' EGFP coding sequence (SEQ ID NO:17); an intron to be screened derived from the recA gene of *Bacillus anthracis,* the 23S ribosomal RNA gene of *Coxiella burnetii* (*Rickettsia burneti*)*,* the tRNA-fMet gene of *Scytonema hofmanni,* the 23S ribosomal RNA gene-2 of *Coxiella burnetii* (*Rickettsia burneti*) (another gene fragment of the 23S ribosomal RNA gene of *Coxiella burnetii*), the tRNA-fMet gene of *Synechocystis sp.,* the 23S rRNA gene of *Thermotoga naphthophila,* the ndhL and trnL-UAA genes of *Synechococcus elongatus,* the 23S rRNA gene of *Thermotoga subterranea,* or the trnL gene of *Phormidium sp.,* & flanking exons (SEQ ID NOs: 18-22 and SEQ ID NOs: 59-62); and a 3'EGFP coding sequence (SEQ ID NO:23). The synthesised gene fragment was cloned into the pUC57 vector (SEQ ID NO:12). Gene synthesis, vector construction, and plasmid linearization were all completed by GenScript.

With reference to 1.2 to 1.5, the resulting linearized plasmids were subjected to *in vitro* transcription, circularization (under suitable conditions, for intron splicing), and E-Gel^{™} EX gel electrophoresis to preliminarily determine whether intron splicing had occurred. In addition, agarose gel electrophoresis was used to more directly determine whether intron splicing had occurred. The method was as follows:

The purified circularized RNA product and uncircularized RNA described above were analysed by agarose gel electrophoresis. Specifically, 100-150 ng of purified RNA was taken and adjusted to 9 µl with nuclease-free water. Subsequently, 1 µl of 10★ dye (GenScript) was added and the mixture was mixed uniformly. The agarose gel (1%, GenScript) was taken and placed in the electrophoresis tank. The gel wells were submerged in the electrophoresis buffer. 10 µl of the uniformly mixed sample was added into each gel well. Electrophoresis was started. The voltage was set to 220 V. The electrophoresis time was set to 8-10 min. After the electrophoresis was completed, the electrophoresis results were observed using the built-in image acquisition system of the multifunctional gel image analysis system (Tanon-1600R), and the images were acquired.

In the agarose gel electrophoresis image, it is usually shown that the migration rate slows down as the molecular weight increases. The molecular weight of the spliced RNA is smaller than that of the unspliced RNA. Therefore, the appearance of two bands (one ahead of the other) in the figure indicates that splicing has occurred.

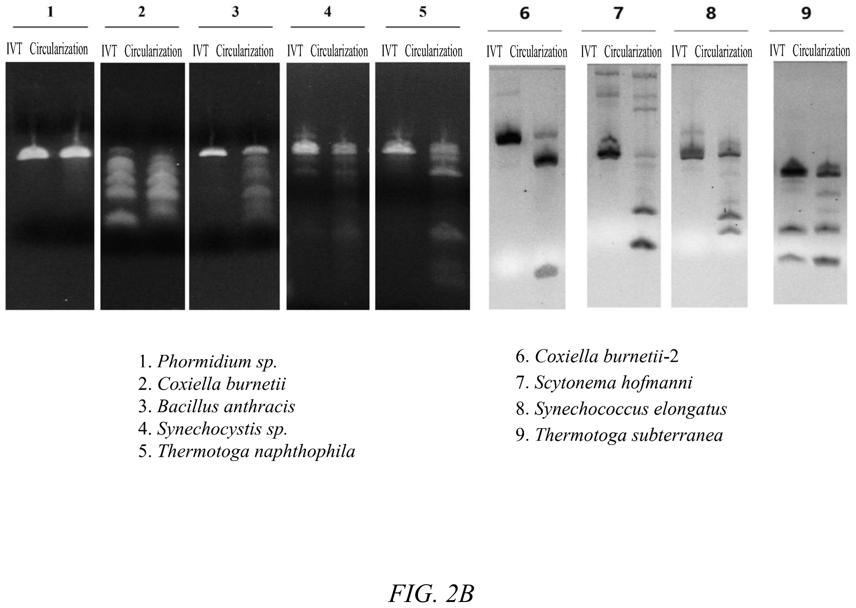

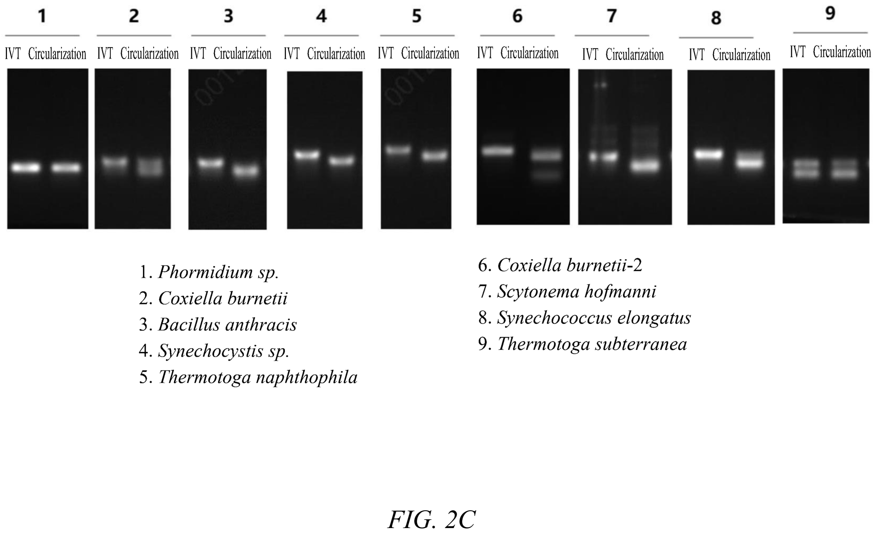

The results are shown in FIGs. 2B and 2C. Eight out of the nine introns, namely the introns derived from *Bacillus anthracis, Coxiella burnetii, Synechocystis sp., Thermotoga naphthophila, Synechococcus elongatus, Scytonema hofmanni* and *Thermotoga subterranea,* underwent self-splicing reaction, with shorter spliced bands appearing in E-Gel^{™} EX gel electrophoresis (FIG. 2B) and two bands, one ahead of the other, appearing in agarose gel electrophoresis (FIG. 2C). In contrast, the intron derived from *Phormidium sp.* did not undergo self-splicing reaction, indicating that it does not have *in vitro* self-splicing capability.

With reference to the method and steps in 1.7, the purified RNA obtained with reference to 1.4 was subjected to reverse transcription, PCR, and gel extraction, followed by sequencing of the circularization junction site. The PCR primers were SEQ ID NOs: 24 and 25, and the same primers were also used for sequencing the circularization junction. The sequencing results of the circularized RNAs transcribed from the plasmids containing the introns of *Phormidium sp., Coxiella burnetii, Bacillus anthracis, Synechocystis sp., Thermotoga naphthophila, Coxiella burnetii-2, Scytonema hofmanni, Synechococcus elongatus* and *Thermotoga subterranea* are as shown in SEQ ID NOs: 30-34 and SEQ ID NOs:63-66, respectively, wherein the two EGFP coding sequence fragments are represented in bold, the sequences of the two exons are represented underlined and in italics, respectively, and the intron is represented underlined and in italics.

It can be seen that the eight introns that underwent the self-splicing reaction have been successfully excised, whereas the unspliced *Phormidium sp.* intron is connected to the truncated fragment of the GFP coding sequence, indicating that the eight introns (except for the *Phormidium sp.* intron) have *in vitro* self-splicing capability.

GCGACGTAAACGGCCACAAGTTCAGCGTGTCTGGCGAGGagaaaactga
gccttagtggagaaatccgctaagtggaagctctcaaactcagggaaaccta (SEQ ID NO: 30)

ACAAGTTCAGCGTGTCTGGCGAGGtaaacggcggccgtaactataacggtcctaaggtag
cgaaCCTACGGCAAGCTGACCCTGAAGTTCATC (SEQ ID NO: 31)

ACGGCCACAAGTTCAGCGTGTCTGGCGAGGttggggttatgttcggaaatccagaaa
caactccaggtggCCTACGGCAAGCTGACCCTGAAGTTCATC (SEQ ID NO:
32)

TCAGCGTGTCTGGCGAGGaagcgacgcgggatagagcagtctggtagctcgtcgggctcataac
ccgaaggtcggtggttcaaatccgcctcccgccaCCTACGGCAAGCTGACCCTGAAGTT
CAT (SEQ ID NO: 33)

AGTTCAGCGTGTCTGGCGAGGccccggtaaacggcggccgtaactataacggtcctaaggta
gcgaaattccttgtcgggtaagttccgacctgcatgaatCCTACGGCAAGCTGACCCTGA
(SEQ ID NO: 34)

CGTAAACGGCCACAAGTTCAGCGTGTCTGGCGAGGcgaaattccttgtcgggta
agttccgacctgcacgaatggCCTACGGCAAGCTGACCCTGAAGTTCAT (SEQ ID
NO: 63)

GCGACGTAAACGGCCACAAGTTCAGCGTGTCTGGCGAGGctggcgaggc
tcataacctacggcaaCCTACGGCAAGCTGACCCTGAAGTTCATCTG (SEQ ID
NO: 64)

GCGACGTAAACGGCCACAAGTTCAGCGTGTCTGGCGAGGacggtagacg
cagcggacttaaaatccgctgactgtaaaggtcgCCTACGGCAAGCTGACCCTGAAGTT
CA (SEQ ID NO: 65)

TAAACGGCCACAAGTTCAGCGTGTCTGGCGAGGggtaaacggcggccgtaacta
taacggtcctaaggtagcgaaCCTACGGCAAGCTGACCCTGAAGTTCATCTGCA
CCA (SEQ ID NO: 66)

### Example 3. Intron-assisted circularization test

Group I intron-mediated mRNA circularization primarily involves truncating the intron sequence at specific sites and adding the resulting fragments to both flanks of the desired circularization sequence (gene of interest (GOI)). During the subsequent RNA circularization, the introns flanking the GOI form a ribozyme structure that self-splices to connect the GOI into a loop. Truncation sites of an intron may affect the formation of the ribozyme structure of the intron and influence the final splicing efficiency.

In this example, the eight introns screened in Example 2 were truncated at different sites and these sites were screened for the splicing efficiency. The truncation position (P) for each of the eight introns is marked in bold underlined uppercase in SEQ ID NOs: 18-21 and SEQ ID NOs: 59-62 in Table 7. Specifically, the sequences are truncated after position P. The sequences of the 3' intron & exon E2 and the 5' intron & exon E1 formed after truncation are summarized in Table 5.

**Table 5. Intron truncation sites and open reading frame (ORF) sequences in constructed vectors**

| Intron source | Truncation position | Truncation site | 3' intron & exon E2/5' intron & exon E1 | ORF |
|---|---|---|---|---|
| *Bacillus anthracis* | P1 | Position 133 | SEQ ID NOs: 39 and 40 | FLUC coding sequence |
| | P2 | Position 195 | SEQ ID NOs: 41 and 42 | |
| | P3 | Position 294 | SEQ ID NOs: 43 and 44 | GLUC coding sequence |
| *Coxiella burnetii* | P1 | Position 157 | SEQ ID NOs: 45 and 46 | FLUC coding sequence |
| | P2 | Position 206 | / | |
| | P3 | Position 245 | / | GLUC coding sequence |
| | P4 | Position 100 | SEQ ID NOs: 47 and 48 | |
| *Synechocystis sp.* | P1 | Position 393 | / | FLUC coding sequence |
| | P2 | Position 246 | / | |
| | P3 | Position 112 | SEQ ID NOs: 49 and 50 | GLUC coding sequence |
| | P4 | Position 298 | SEQ ID NOs: 51 and 52 | |
| | P5 | Position 500 | / | |
| *Thermotoga naphthophila* | P1 | Position 369 | SEQ ID NOs: 53 and 54 | FLUC coding sequence |
| | P2 | Position 408 | SEQ ID NOs: 55 and 56 | |
| | P3 | Position 314 | / | GLUC coding sequence |
| | P4 | Position 130 | SEQ ID NOs: 57 and 58 | |
| *Coxiella burnetii-2* | P1 | Position 313 | SEQ ID NOs: 67 and 68 | EGFP coding sequence |
| | P2 | Position 453 | SEQ ID NOs: 69 and 70 | |
| *Scytonema hofmanni* | P1 | Position 71 | SEQ ID NOs: 71 and 72 | EGFP coding sequence |
| | P2 | Position 140 | SEQ ID NOs: 73 and 74 | |
| | P3 | Position 184 | SEQ ID NOs: 75 and 76 | |
| | P4 | Position 216 | SEQ ID NOs: 77 and 78 | |
| *Synechococcus elongatus* | P1 | Position 135 | SEQ ID NOs: 79 and 80 | EGFP coding sequence |
| | P2 | Position 186 | SEQ ID NOs: 81 and 82 | |
| *Thermotoga subterranea* | P1 | Position 94 | SEQ ID NOs: 83 and 84 | EGFP coding sequence |
| | P2 | Position 336 | SEQ ID NOs: 85 and 86 | |
| | P3 | Position 492 | SEQ ID NOs: 87 and 88 | |
| | P4 | Position 577 | SEQ ID NOs: 89 and 90 | |

With reference to Example 1 and FIG. 1B, the vectors were constructed using the ITR strategy of the present application. Specifically, a DNA sequence was synthesised, the sequence sequentially comprising, from the 5' end to the 3' end: a T7 promoter (SEQ ID NO: 1), a CpG-free 5' ITR sequence (SEQ ID NO: 13), a 3' intron & exon E2 (see the truncation sites in Table 5), a 5' spacer sequence (SEQ ID NO: 4), a CVB3 IRES element (SEQ ID NO: 5), a firefly luciferase (FLUC) or *Gaussia* luciferase (GLUC) encoding gene (SEQ ID NO: 6 or 7), a 3' spacer sequence (SEQ ID NO: 9), a 5' intron & exon E1 (see the truncation sites in Table 5), a CpG-free 3' ITR sequence (SEQ ID NO: 14), and restriction enzyme site EcoRV for plasmid linearization (GATATC). The DNA fragment synthesised above was cloned into the pUC57 vector (SEQ ID NO: 12) to obtain a linearized vector product. The control vector was constructed according to the published strategy, and the specific structure was the same as that described in 1.1, except that the FLUC coding gene was replaced with the EGFP coding gene (SEQ ID NO: 8). Gene synthesis, vector construction, and plasmid linearization were all completed by GenScript.

Then, *in vitro* transcription, circularization, and capillary gel electrophoresis were performed. Reference can be made to 1.2, 1.3, 1.4, and 1.6 for the specific method and steps.

The capillary gel electrophoresis results of some transcripts are shown in FIGs. 3A-3F. The samples were not digested with RNase R. Since RNase R can specifically eliminate linear RNA, researchers usually show the purity obtained after RNase R digestion. Construction methods with low circularization efficiency can also obtain relatively pure circular RNA by using RNase R to eliminate linear RNA precursors and other linear byproducts. However, this presentation method masks the circularization efficiency of the construction itself. A construction method with low circularization efficiency will result in much greater consumption of process reagents compared with a higher-efficiency method and a greatly reduced circular RNA yield compared with a higher-efficiency method.

The purity of circular RNA was calculated based on the results of capillary gel electrophoresis. The results are listed in Table 6.

**Table 6. Purity of circular RNAs obtained with introns truncated at various sites**

| Intron source | Truncation position | Truncation site | Circular RNA purity |
|---|---|---|---|
| *Bacillus anthracis* | P1 | Position 133 | 62.3% |
| | P2 | Position 195 | 84.0% |
| | P3 | Position 294 | 84.6% |
| *Coxiella burnetii* | P1 | Position 157 | 65.0% |
| | P2 | Position 206 | 36.1% |
| | P3 | Position 245 | 24.8% |
| | P4 | Position 100 | 68.1% |
| *Synechocystis sp.* | P1 | Position 393 | 43.9% |
| | P2 | Position 246 | 55.2% |
| | P3 | Position 112 | 63.0% |
| | P4 | Position 298 | 57.7% |
| | P5 | Position 500 | 11.2% |
| *Thermotoga naphthophila* | P1 | Position 369 | 68.8% |
| | P2 | Position 408 | 51.4% |
| | P3 | Position 314 | 59.5% |
| | P4 | Position 130 | 58.7% |
| *Coxiella burnetii-2* | P1 | Position 313 | 67.4% |
| | P2 | Position 453 | 63% |
| *Scytonema hofmanni* | P1 | Position 71 | 57% |
| | P2 | Position 140 | 45.5% |
| | P3 | Position 184 | 71.9% |
| | P4 | Position 216 | 48.5% |
| *Synechococcus elongatus* | P1 | Position 135 | 61.8% |
| | P2 | Position 186 | 56.1% |
| *Thermotoga subterranea* | P1 | Position 94 | 47.0% |
| | P2 | Position 336 | 77.1% |
| | P3 | Position 492 | 78.1% |
| | P4 | Position 577 | 55.3% |

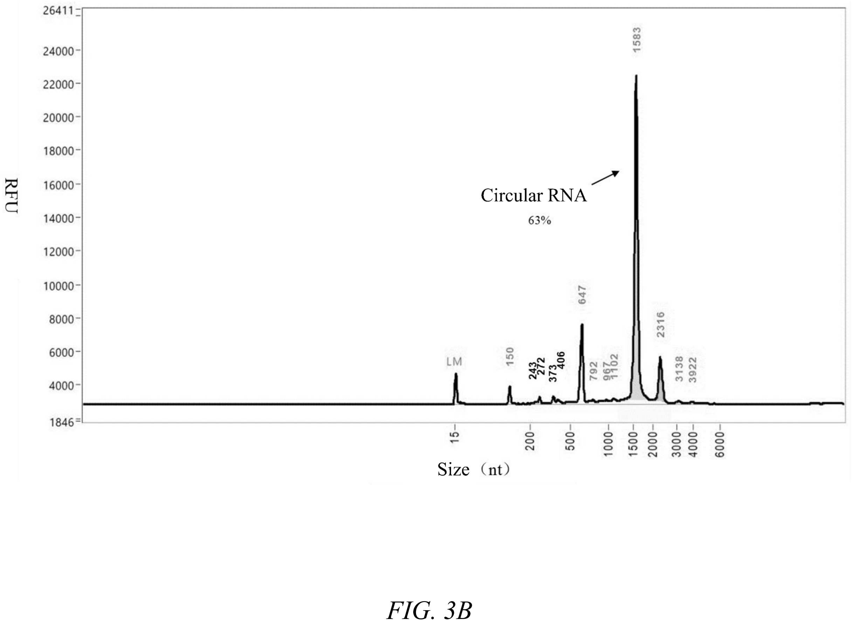

It can be seen that the introns and truncation positions thereof that provide great circularization effects include: the intron from *Synechocystis sp.,* P3; the intron from *Coxiella burnetii,* P4; the intron from *Thermotoga naphthophila,* P1; the intron from *Bacillus anthracis,* P2; the intron from *Bacillus anthracis,* P3; the intron from *Coxiella burnetii-2,* P1; the intron from Coxiella burnetii-2, P2; the intron from *Scytonema hofmanni,* P3; the intron from *Synechococcus elongatus,* P1; the intron from *Thermotoga subterranea,* P2 and the intron from *Thermotoga subterranea,* P3 (FIGs. 3B-3L). Compared with the *Anabaena* intron (59.5%, FIG. 3A), these introns show significant advantages.

The purified RNA obtained above was subjected to reverse transcription, PCR, and gel extraction, followed by sequencing of the circularization junction site with reference to 1.7. The primer pairs used in PCR amplification and sequencing were SEQ ID NOs: 27 and 26, or SEQ ID NOs: 27 and 28, or SEQ ID NOs: 91 and 92.

The sequencing results show that the introns are excised, leaving the flanking exons. The sequencing results of exemplary circularization junctions (including those of the circularization junctions with the introns from *Coxiella burnetii,* P4; *Bacillus anthracis,* P3; *Synechocystis sp.,* P1; *Thermotoga naphthophila,* P1; *Coxiella burnetii*-2, P1; *Scytonema hofmanni,* P3; *Synechococcus elongatus,* P1; *Thermotoga subterranea,* P2) are shown in SEQ ID NOs: 35-38 and SEQ ID NOs: 93-96, respectively, wherein the 3' spacer sequence (in bold), E1 (underlined), E2 (in italics) and the 5' spacer sequence (in bold and underlined) are represented differently.

AAAAAACAAAAAACAAAACGGCTATTATGCGTTACCGGCtaaacggcggc
cgtaactataacggtcctaaggtagcgaaGCCGGAAACGCAATAGCCGaaaaacaaaaaac
aaaaaa (SEQ ID NO: 35)

AAAAAACAAAAAACAAAACGGCTATTATGCGTTACCGGCttggggttatgtt
cggaaatccagaaacaactccaggtggGCCGGAAACGCAATAGCCGaaaaacaaaaaaca
aaaaa (SEQ ID NO: 36)

AAACGGCTATTATGCGTTACCGGCaagcgacgcgggatagagcagtctggtagctcgtcg
ggctcataacccgaaggtcggtggttcaaatccgcctcccgccaGCCGGAAACGCAATAGCCG
(SEQ ID NO: 37)

AAAACGGCTATTATGCGTTACCGGCccccggtaaacggcggccgtaactataacggtcct
aaggtagcgaaattccttgtcgggtaagttccgacctgcatgaatGCCGGAAACGCAATAGCCG
aaaaacaaaaa (SEQ ID NO: 38)

AAAAAACAAAACGGCTATTATGCGTTACCGGC_cgaaattccttgtcgggtaagttc
cgacctgcacgaatggGCCGGAAACGCAATAGCCGaaaaacaaaaacaaaaaaaaaa
aaaaa (SEQ ID NO: 93)

AAAAAACAAAAAACAAAACGGCTATTATGCGTTACCGGCctggcgaggct
cataacctacggcaaGCCGGAAACGCAATAGCCGAaaaacaaaaaacaaaaaaaaaaa
aaaaaaaaaaaa (SEQ ID NO: 94)

AAAAAACAAAAAACAAAACGGCTATTATGCGTTACCGGCacggtagacgc
agcggacttaaaatccgctgactgtaaaggtcgGCCGGAAACGCAATAGCCGaaaaacaaaa
aacaaaaaaaaa (SEQ ID NO: 95)

AAAAAACAAAAAACAAAACGGCTATTATGCGTTACCGGCggtaaacggcg
gccgtaactataacggtcctaaggtagcgaaGCCGGAAACGCAATAGCCGaaaaacaaaaa
acaaaaaaaaaaaaaaaaaaaaaa (SEQ ID NO: 96)

### Example 4. Co-transcriptional circularization

The intron derived from *Bacillus anthracis,* when truncated at P3, exhibits a significantly enhanced self-splicing capability, enabling the mRNA to undergo autonomous circularization simultaneously with transcription without requiring additional circularization steps. In other words, RNA transcription and circularization can be completed in 1.2.

Specifically, the control vector and the vector containing the *Bacillus anthracis* intron truncated at position P3 in Example 3 were constructed, and *in vitro* transcription was performed according to 1.2. The obtained RNA was purified according to 1.3 and then subjected to capillary gel electrophoresis according to 1.6.

The results of capillary gel electrophoresis are shown in FIGs. 4A and 4B. The circular RNA obtained using the *Bacillus anthracis* intron (truncated at P3) achieved a purity of 71.5% (FIG. 4B), whereas no circular RNA was produced from the control vector after the transcription and the proportion of linear RNA reached 89.6% (FIG. 4A).

The circular RNA was subjected to reverse transcription, PCR, and gel extraction, followed by sequencing the circularization junction with reference to 1.7. The sequencing result obtained using the *Bacillus anthracis* intron (truncated at P3) was identical to SEQ ID NO: 36, indicating that the transcribed RNA was successfully circularized in the method and steps of 1.2 without requiring the circularization process in 1.4.

Co-transcriptional circularization is a very promising method for producing circular RNA, which plays an important role in improving yield and reducing the risk of process residues.

**Table 7. List of partial sequences involved in the present application**

| Description/ |
|---|
| Sequence+sequence number |
| T7 promoter |
| taatacgactcactatagG (SEQ ID NO: 1) |
| 5' homology arm |
| CCGTCGATTGTCCACTGGTC (SEQ ID NO: 2) |
| 3' intron & exon E2 |
| AACAATAGATGACTTACAACTAATCGGAAGGTGCAGAGACTCGACGGGAGCTA CCCTAACGTCAAGACGAGGGTAAAGAGAGAGTCCAATTCTCAAAGCCAATAGG CAGTAGCGAAAGCTGCAAGAGAATGAAAATCCGTTGACCTTAAACGGTCGTGT GGGTTCAAGTCCCTCCACCCCCAC (SEQ ID NO: 3) |
| 5' spacer sequence |
| GCCGGAAACGCAATAGCCGaaaaacaaaaaacaaaaaaAaaaaaaaaaaaaa (SEQ ID NO: 4) |
| CVB3 IRES element |
| TTAAAACAGCCTGTGGGTTGATCCCACCCACAGGCCCATTGGGCGCTAGCACTC TGGTATCACGGTACCTTTGTGCGCCTGTTTTATACCCCCTCCCCCAACTGTAACT TAGAAGTAACACACACCGATCAACAGTCAGCGTGGCACACCAGCCACGTTTTG ATCAAGCACTTCTGTTACCCCGGACTGAGTATCAATAGACTGCTCACGCGGTTG AAGGAGAAAGCGTTCGTTATCCGGCCAACTACTTCGAAAAACCTAGTAACACC GTGGAAGTTGCAGAGTGTTTCGCTCAGCACTACCCCAGTGTAGATCAGGTCGAT GAGTCACCGCATTCCCCACGGGCGACCGTGGCGGTGGCTGCGTTGGCGGCCTGC CCATGGGGAAACCCATGGGACGCTCTAATACAGACATGGTGCGAAGAGTCTAT TGAGCTAGTTGGTAGTCCTCCGGCCCCTGAATGCGGCTAATCCTAACTGCGGAG CACACACCCTCAAGCCAGAGGGCAGTGTGTCGTAACGGGCAACTCTGCAGCGG AACCGACTACTTTGGGTGTCCGTGTTTCATTTTATTCCTATACTGGCTGCTTATG GTGACAATTGAGAGATCGTTACCATATAGCTATTGGATTGGCCATCCGGTGACT AATAGAGCTATTATATATCCCTTTGTTGGGTTTATACCACTTAGCTTGAAAGAGG TTAAAACATTACAATTCATTGTTAAGTTGAATACAGCAAA (SEQ ID NO: 5) |
| Firefly luciferase (FLUC) encoding gene |
| ATGGAAGACGCCAAAAACATAAAGAAAGGCCCGGCGCCATTCTATCCGCTGGA AGATGGAACCGCTGGAGAGCAACTGCATAAGGCTATGAAGAGATACGCCCTGG TTCCTGGAACAATTGCTTTTACAGATGCACATATCGAGGTGGACATCACTTACG CTGAGTACTTCGAAATGTCCGTTCGGTTGGCAGAAGCTATGAAACGATATGGGC TGAATACAAATCACAGAATCGTCGTATGCAGTGAAAACTCTCTTCAATTCTTTA TGCCGGTGTTGGGCGCGTTATTTATCGGAGTTGCAGTTGCGCCCGCGAACGACA TTTATAATGAACGTGAATTGCTCAACAGTATGGGCATTTCGCAGCCTACCGTGG TGTTCGTTTCCAAAAAGGGGTTGCAAAAAATTTTGAACGTGCAAAAAAAGCTCC CAATCATCCAAAAAATTATTATCATGGATTCTAAAACGGATTACCAGGGATTTC AGTCGATGTACACGTTCGTCACATCTCATCTACCTCCCGGTTTTAATGAATACGA TTTTGTGCCAGAGTCCTTCGATAGGGACAAGACAATTGCACTGATCATGAACTC CTCTGGATCTACTGGTCTGCCTAAAGGTGTCGCTCTGCCTCATAGAACTGCCTGC GTGAGATTCTCGCATGCCAGAGATCCTATTTTTGGCAATCAAATCATTCCGGAT ACTGCGATTTTAAGTGTTGTTCCATTCCATCACGGTTTTGGAATGTTTACTACAC TCGGATATTTGATATGTGGATTTCGAGTCGTCTTAATGTATAGATTTGAAGAAG AGCTGTTTCTGAGGAGCCTTCAGGATTACAAGATTCAAAGTGCGCTGCTGGTGC CAACCCTATTCTCCTTCTTCGCCAAAAGCACTCTGATTGACAAATACGATTTATC TAATTTACACGAAATTGCTTCTGGTGGCGCTCCCCTCTCTAAGGAAGTCGGGGA AGCGGTTGCCAAGAGGTTCCATCTGCCAGGTATCAGGCAAGGATATGGGCTCAC TGAGACTACATCAGCTATTCTGATTACACCCGAGGGGGATGATAAACCGGGCGC GGTCGGTAAAGTTGTTCCATTTTTTGAAGCGAAGGTTGTGGATCTGGATACCGG GAAAACGCTGGGCGTTAATCAAAGAGGCGAACTGTGTGTGAGAGGTCCTATGA TTATGTCCGGTTATGTAAACAATCCGGAAGCGACCAACGCCTTGATTGACAAGG ATGGATGGCTACATTCTGGAGACATAGCTTACTGGGACGAAGACGAACACTTCT TCATCGTTGACCGCCTGAAGTCTCTGATTAAGTACAAAGGCTATCAGGTGGCTC CCGCTGAATTGGAATCCATCTTGCTCCAACACCCCAACATCTTCGACGCAGGTG TCGCAGGTCTTCCCGACGATGACGCCGGTGAACTTCCCGCCGCCGTTGTTGTTTT GGAGCACGGAAAGACGATGACGGAAAAAGAGATCGTGGATTACGTCGCCAGTC AAGTAACAACCGCGAAAAAGTTGCGCGGAGGAGTTGTGTTTGTGGACGAAGTA CCGAAAGGTCTTACCGGAAAACTCGACGCAAGAAAAATCAGAGAGATCCTCAT AAAGGCCAAGAAGGGCGGAAAGATCGCCGTGTAA (SEQ ID NO: 6) |
| *Gaussia* luciferase encoding gene |
| ATGGGAGTCAAAGTTCTGTTTGCCCTGATCTGCATCGCTGTGGCCGAGGCCAAG CCCACCGAGAACAACGAAGACTTCAACATCGTGGCCGTGGCCAGCAACTTCGC GACCACGGATCTCGATGCTGACCGCGGGAAGTTGCCCGGCAAGAAGCTGCCGC TGGAGGTGCTCAAAGAGATGGAAGCCAATGCCCGGAAAGCTGGCTGCACCAGG GGCTGTCTGATCTGCCTGTCCCACATCAAGTGCACGCCCAAGATGAAGAAGTTC ATCCCAGGACGCTGCCACACCTACGAAGGCGACAAAGAGTCCGCACAGGGCGG CATAGGCGAGGCGATCGTCGACATTCCTGAGATTCCTGGGTTCAAGGACTTGGA GCCCATGGAGCAGTTCATCGCACAGGTCGATCTGTGTGTGGACTGCACAACTGG CTGCCTCAAAGGGCTTGCCAACGTGCAGTGTTCTGACCTGCTCAAGAAGTGGCT GCCGCAACGCTGTGCGACCTTTGCCAGCAAGATCCAGGGCCAGGTGGACAAGA TCAAGGGGGCCGGTGGTGACTAA (SEQ ID NO: 7) |
| Encoding gene-EGFP |
| atggtgagcaagggcgaggagctgttcaccggggtggtgcccatcctggtcgagctggacggcgacgtaaacggccacaagttc agcgtgtccggcgagggcgagggcgatgccacctacggcaagctgaccctgaagttcatctgcaccaccggcaagctgcccgtg ccctggcccaccctcgtgaccaccctgacctacggcgtgcagtgcttcagccgctaccccgaccacatgaagcagcacgacttctt caagtccgccatgcccgaaggctacgtccaggagcgcaccatcttcttcaaggacgacggcaactacaagacccgcgccgaggt gaagttcgagggcgacaccctggtgaaccgcatcgagctgaagggcatcgacttcaaggaggacggcaacatcctggggcacaa gctggagtacaactacaacagccacaacgtctatatcatggccgacaagcagaagaacggcatcaaggtgaacttcaagatccgcc acaacatcgaggacggcagcgtgcagctcgccgaccactaccagcagaacacccccatcggcgacggccccgtgctgctgccc gacaaccactacctgagcacccagtccgccctgagcaaagaccccaacgagaagcgcgatcacatggtcctgctggagttcgtga ccgccgccgggatcactctcggcatggacgagctgtacaagTAA (SEQ ID NO: 8) |
| 3' spacer sequence |
| AAAAAACAAAAAACAAAACGGCTATTATGCGTTACCGGC (SEQ ID NO: 9) |
| 5' intron & exon E1 |
| GAGACGCTACGGACTTAAATAATTGAGCCTTAAAGAAGAAATTCTTTAAGTGGA TGCTCTCAAACTCAGGGAAACCTAAATCTAGTTATAGACAAGGCAATCCTGAGC CAAGCCGAAGTAGTAATTAGTAAG (SEQ ID NO: 10) |
| 3' homology arm |
| ACCAGTGGACAATCGACGG (SEQ ID NO: 11) |
| EcoRV |
| GATATC |
| pUC57 vector |
| tcgcgcgtttcggtgatgacggtgaaaacctctgacacatgcagctcccggacgtcattgtcgatctccccatgcgagagtagggaa ctgccaggcatcaaataaaacgaaaggctcagtcgaaagactgggcctttcgttttatctgttgtttgtcggtgaacgctctcctgagta ggacaaatccgccgggagcggatttgaacgttgcgaagcaacggcccggagggtggcgggcaggacgcccgccataaactgcc aggcatcaaattaagcagaaggccatcctgacggatggcctttttgcgtttctacaaactctctctggctaactagagaacccactgctt actggcttatcgaaatgctagcgtttaaacttaagcttggtaccgagctcggatccTCTAGAgggcccgtttaaacccgctgatc agcctcgactCTAGCTTGGATTCTCACCAATAAAAAACGCCCGGCGGCAACCGAGCG TTCTGAACAAATCCAGATGGAGTTCTGAGGTCATTACTGGATCTATCAACAGGA GTCCAAGCTCAGatcattgtcacatgtgagcaaaaggccagcaaaaggccaggaaccgtaaaaaggccgcgttgctgg cgtttttccataggctccgcccccctgacgagcatcacaaaaatcgacgctcaagtcagaggtggcgaaacccgacaggactataa agataccaggcgtttccccctggaagctccctcgtgcgctctcctgttccgaccctgccgcttaccggatacctgtccgcctttctccct tcgggaagcgtggcgctttctcatagctcacgctgtaggtatctcagttcggtgtaggtcgttcgctccaagctgggctgtgtgcacga accccccgttcagcccgaccgctgcgccttatccggtaactatcgtcttgagtccaacccggtaagacacgacttatcgccactggca gcagccactggtaacaggattagcagagcgaggtatgtaggcggtgctacagagttcttgaagtggtggcctaactacggctacact agaagaacagtatttggtatctgcgctctgctgaagccagttaccttcggaaaaagagttggtagctcttgatccggcaaacaaacca ccgctggtagcggtggtttttttgtttgcaagcagcagattacgcgcagaaaaaaaggatctcaagaagatcctttgatcttttctacgg ggtctgacgctcagtggaacgaaaactcacgttaagggattttggtcatgagattatcaaaaaggatcttcacctagatccttttaaatta aaaatgaagttttaaatcaagcccaatctgaataatgttacaaccaattaaccaattctgattagaaaaactcatcgagcatcaaatgaa actgcaatttattcatatcaggattatcaataccatatttttgaaaaagccgtttctgtaatgaaggagaaaactcaccgaggcagttccat aggatggcaagatcctggtatcggtctgcgattccgactcgtccaacatcaatacaacctattaatttcccctcgtcaaaaataaggtta tcaagtgagaaatcaccatgagtgacgactgaatccggtgagaatggcaaaagtttatgcatttctttccagacttgttcaacaggcca gccattacgctcgtcatcaaaatcactcgcatcaaccaaaccgttattcattcgtgattgcgcctgagcgaggcgaaatacgcgatcg ctgttaaaaggacaattacaaacaggaatcgaatgcaaccggcgcaggaacactgccagcgcatcaacaatattttcacctgaatca ggatattcttctaatacctggaatgctgtttttccggggatcgcagtggtgagtaaccatgcatcatcaggagtacggataaaatgcttg atggtcggaagaggcataaattccgtcagccagtttagtctgaccatctcatctgtaacatcattggcaacgctacctttgccatgtttca gaaacaactctggcgcatcgggcttcccatacaagcgatagattgtcgcacctgattgcccgacattatcgcgagcccatttataccc atataaatcagcatccatgttggaatttaatcgcggcctcgacgtttcccgttgaatatggctcataacaccccttgtattactgtttatgta agcagacagttttattgttcatgatgatatatttttatcttgtgcaatgtaacatcagagattttgagacacgggccagagctgca (SEQ ID NO: 12) |
| 5' EGFP coding sequence |
| ATGGTGAGCAAGGGCGAGGAGCTGTTCACCGGGGTGGTGCCCATCCTGGTCGA GCTGGACGGCGACGTAAACGGCCACAAGTTCAGCGTGTCTGGCGAGG (SEQ ID NO: 17) |
| Intron & exon derived from *Bacillus anthracis* |
| ttggggttatgttcggaaatcaactacaagttgcttaatcgtaaagattaaggagtttaccatgggtttcccttgctagtgatagcatgtta aaaaaccttgtgaaccttattgcctaagggtgtaactattCatatagttgctaacggtgaagcccaccattattcagggtaataccgtgc caagctccatagTgatatggggaaggtgtagagactaccgaaaaggacttttagttaactgagtagggtacggcagatgataggct actgctggaagtgcaaggctctctgTaaagagatgaagagatagtccggactatagagatggaaaatctatagatagtgccagaa acaactccaggtgg (SEQ ID NO: 18) |
| Intron & exon derived from *Coxiella burnetii* |
| taaacggcggccgtaactataaattaaccgttgtagttacgtaaataaacttggctatatgctggaaactccgagtatcctccagtactc atctcaactAaaattgaggtagtaaaaatctgcgaggtgcggacaatcagcaggaaagactaatctTtttgattagaatcctcagag actaatacgccgagcgtacttagaaaaaAtccattctaagtgtgatgatagagtccgatctctatagCgatatagagggttttcaaaa gaaacctgttaatcattatcccatgattaacataacgtcaataaacggtcctaaggtagcgaa (SEQ ID NO: 19) |
| Intron & exon derived from *Synechocystis sp.* |
| aagcgacgcgggatagagcagtctggtagctcgtcgggctcaggtcgcaagatgtaaaccatgagcactaagcttaagggtgacat tgcagagcaagccgccattcttcgtGcattgaagcttggttggggggttctgaagccattgggagaccgtctttcttacgatttggtctt tgatgtcgaagggatccttctgaaggtgcaggttaaaagctcctggttcagcgaaaagactggcaactaTgttgttgacaatcgcag aacaaggacaaacagaagaaacattgttagatctCcttatcgtgggaatgactttgattttgctgttgcctacgtcgaagaacttgagct gttttacgtcttcccggttgatgtctttatcagttacggCagtgaaattcacctggtcgaaactgataagcgtcagagaaaaccaaggtc attcggttaccgagaggcttggcatctcatcttgcaaaaaggggctgcgcaaaaggAaacttctgcgtgattatctctcaaattcggg gaagcctttcaaatggtaatcccgagccaaacctaggaatgcttggtgtttctgggaaggtgtagagacttaatgggagacaccctaa cagaaaagctgagggtgaagagaaagtccagaccacaaactgacagagtcaggcagtgaaaactgtagttggtaagcataaccc gaaggtcggtggttcaaatccgcctcccgcca (SEQ ID NO: 20) |
| Intron & exon derived from *Thermotoga naphthophila* |
| ccccggtaaacggcggccgtaactataacggtcctaaggtagcattcgctgcctgcccacagggaaacctgtggggcaatacccg gctgtatgcgggaaagcccttagagcccttcctacctgggggctGcggccagcccaggtaaaaatggaagggattgggtaatccg caggaaaccccgatgagggggatcctcagagactgtacgccgggcatccagtccccgtgagaaggggtggaaatcttggatctca agcctgattgggttgttggattcgtcgatggtgaaggttgtttctacgtgggtgttaaTagaaatcgcaccatgaagacaggttaccaa gttctccctgaattcaggattgttCagcatgaaagggatatacagattctgtacgcattgaagAagttctttgggtgcggtgttgtacga aaaaaccacgatgatcgatatgaacttcggatccggaagaagtcttgcttgaagaaagtagtggaattcttcgaaaagcaccctctga aaacgaagaagaacgttgacttcaagaagttcagaagaataatcctgatgatggaacggggagaacacctgacgaaagaaggtct aatcaagatcttagaaatcgccatggaaatgaacacaggtaaccacgagagactgcgacgagtccttgaagagatccggggactg gatgaagatacagtccacccccattccgaaaggatgggataagggtgagcgaaattccttgtcgggtaagttccgacctgcatgaa t (SEQ ID NO: 21) |
| Intron & exon derived from *Phormidium sp.* |
| agaaastgcgccttactggagaaatcctttaagtgacckctctcaaattcagggaaacctaactctggcaacagacaaggcaatcctg agccaagccgaaatttcggaaggtgcagagactcgacgggagctaccctaacgtaaagccgagggtaaagggagagtccaattct caaagcctaattatagggcagcagcgaaagttgcggagaatg (SEQ ID NO: 22) |
| 3' EGFP coding sequence |
| CCTACGGCAAGCTGACCCTGAAGTTCATCTGCACCACCGGCAAGCTGCCCGTGC CCTGGCCCACCCTCGTGACCACCCTGACCTACGGCGTGCAGTGCTT (SEQ ID NO: 23) |
| Intron-F (junction sequencing for intron screening) |
| GATGGTGAGCAAGGGCGAG (SEQ ID NO: 24) |
| Intron-R (junction sequencing for intron screening) |
| CAAGCACTGCACGCCGTAG (SEQ ID NO: 25) |
| Luc-F (Sequencing primers for circularization junction) |
| AATCCGGAAGCGACCAACGC (SEQ ID NO: 26) |
| CVB3J-R (Sequencing primers for circularization junction) |
| GGGGCCGGAGGACTACCAAC (SEQ ID NO: 27) |
| Gluc-F (Sequencing primers for circularization junction) |
| GTTGCCCGGCAAGAAGCTGC (SEQ ID NO: 28) |
| *Bacillus anthracis*-P1-3' intron & exon E2 |
| atatagttgctaacggtgaagcccaccattattcagggtaataccgtgccaagctccatagtgatatggggaaggtgtagagactacc gaaaaggacttttagttaactgagtagggtacggcagatgataggctactgctggaagtgcaaggctctctgtaaagagatgaagag atagtccggactatagagatggaaaatctatagatagtgccagaaacaactccaggtgg (SEQ ID NO: 39) |
| *Bacillus anthracis*-P1-5' intron & exon E1 |
| ttggggttatgttcggaaatcaactacaagttgcttaatcgtaaagattaaggagtttaccatgggtttcccttgctagtgatagcatgtta aaaaaccttgtgaaccttattgcctaagggtgtaactattc (SEQ ID NO: 40) |
| *Bacillus anthracis*-P2-3' intron & exon E2 |
| gatatggggaaggtgtagagactaccgaaaaggacttttagttaactgagtagggtacggcagatgataggctactgctggaagtgc aaggctctctgtaaagagatgaagagatagtccggactatagagatggaaaatctatagatagtgccagaaacaactccaggtgg (SEQ ID NO: 41) |
| *Bacillus anthracis*-P2-5' intron & exon E1 |
| ttggggttatgttcggaaatcaactacaagttgcttaatcgtaaagattaaggagtttaccatgggtttcccttgctagtgatagcatgtta aaaaaccttgtgaaccttattgcctaagggtgtaactattcatatagttgctaacggtgaagcccaccattattcagggtaataccgtgcc aagctccatagt (SEQ ID NO: 42) |
| *Bacillus anthracis*-P3-3' intron & exon E2 |
| aaagagatgaagagatagtccggactatagagatggaaaatctatagatagtgccagaaacaactccaggtgg (SEQ ID NO: 43) |
| *Bacillus anthracis-*P3-5' intron & exon E1 |
| ttggggttatgttcggaaatcaactacaagttgcttaatcgtaaagattaaggagtttaccatgggtttcccttgctagtgatagcatgtta aaaaaccttgtgaaccttattgcctaagggtgtaactattcatatagttgctaacggtgaagcccaccattattcagggtaataccgtgcc aagctccatagtgatatggggaaggtgtagagactaccgaaaaggacttttagttaactgagtagggtacggcagatgataggctact gctggaagtgcaaggctctctgt (SEQ ID NO: 44) |
| *Coxiella burnetii*-P1-3' intron & exon E2 |
| tttgattagaatcctcagagactaatacgccgagcgtacttagaaaaaatccattctaagtgtgatgatagagtccgatctctatagcgat atagagggttttcaaaagaaacctgttaatcattatcccatgattaacataacgtcaataaacggtcctaaggtagcgaa (SEQ ID NO: 45) |
| *Coxiella burnetii*-P1-5' intron & exon E1 |
| taaacggcggccgtaactataaattaaccgttgtagttacgtaaataaacttggctatatgctggaaactccgagtatcctccagtactc atctcaactaaaattgaggtagtaaaaatctgcgaggtgcggacaatcagcaggaaagactaatctt (SEQ ID NO: 46) |
| *Coxiella burnetii*-P4-3' intron & exon E2 |
| aaattgaggtagtaaaaatctgcgaggtgcggacaatcagcaggaaagactaatctttttgattagaatcctcagagactaatacgcc gagcgtacttagaaaaaatccattctaagtgtgatgatagagtccgatctctatagcgatatagagggttttcaaaagaaacctgttaat cattatcccatgattaacataacgtcaataaacggtcctaaggtagcgaa (SEQ ID NO: 47) |
| *Coxiella burnetii*-P4-5' intron & exon E1 |
| taaacggcggccgtaactataaattaaccgttgtagttacgtaaataaacttggctatatgctggaaactccgagtatcctccagtactc atctcaacta (SEQ ID NO: 48) |
| *Synechocystis sp.*-P3-3' intron & exon E2 |
| cattgaagcttggttggggggttctgaagccattgggagaccgtctttcttacgatttggtctttgatgtcgaagggatccttctgaaggt gcaggttaaaagctcctggttcagcgaaaagactggcaactatgttgttgacaatcgcagaacaaggacaaacagaagaaacattgt tagatctccttatcgtgggaatgactttgattttgctgttgcctacgtcgaagaacttgagctgttttacgtcttcccggttgatgtctttatca gttacggcagtgaaattcacctggtcgaaactgataagcgtcagagaaaaccaaggtcattcggttaccgagaggcttggcatctcat cttgcaaaaaggggctgcgcaaaaggaaacttctgcgtgattatctctcaaattcggggaagcctttcaaatggtaatcccgagccaa acctaggaatgcttggtgtttctgggaaggtgtagagacttaatgggagacaccctaacagaaaagctgagggtgaagagaaagtc cagaccacaaactgacagagtcaggcagtgaaaactgtagttggtaagcataacccgaaggtcggtggttcaaatccgcctcccgc ca (SEQ ID NO: 49) |
| *Synechocystis* sp.-P3-5' intron & exon E1 |
| aagcgacgcgggatagagcagtctggtagctcgtcgggctcaggtcgcaagatgtaaaccatgagcactaagcttaagggtgacat tgcagagcaagccgccattcttcgtg (SEQ ID NO: 50) |
| *Synechocystis* sp.-P4-3' intron & exon E2 |
| cttatcgtgggaatgactttgattttgctgttgcctacgtcgaagaacttgagctgttttacgtcttcccggttgatgtctttatcagttacgg cagtgaaattcacctggtcgaaactgataagcgtcagagaaaaccaaggtcattcggttaccgagaggcttggcatctcatcttgcaa aaaggggctgcgcaaaaggaaacttctgcgtgattatctctcaaattcggggaagcctttcaaatggtaatcccgagccaaacctag gaatgcttggtgtttctgggaaggtgtagagacttaatgggagacaccctaacagaaaagctgagggtgaagagaaagtccagacc acaaactgacagagtcaggcagtgaaaactgtagttggtaagcataacccgaaggtcggtggttcaaatccgcctcccgcca (SEQ ID NO: 51) |
| *Synechocystis* sp.-P4-5' intron & exon E1 |
| aagcgacgcgggatagagcagtctggtagctcgtcgggctcaggtcgcaagatgtaaaccatgagcactaagcttaagggtgacat tgcagagcaagccgccattcttcgtgcattgaagcttggttggggggttctgaagccattgggagaccgtctttcttacgatttggtcttt gatgtcgaagggatccttctgaaggtgcaggttaaaagctcctggttcagcgaaaagactggcaactatgttgttgacaatcgcagaa caaggacaaacagaagaaacattgttagatctc (SEQ ID NO: 52) |
| *Thermotoga naphthophila*-P1-3' intron & exon E2 |
| agcatgaaagggatatacagattctgtacgcattgaagaagttctttgggtgcggtgttgtacgaaaaaaccacgatgatcgatatgaa cttcggatccggaagaagtcttgcttgaagaaagtagtggaattcttcgaaaagcaccctctgaaaacgaagaagaacgttgacttca agaagttcagaagaataatcctgatgatggaacggggagaacacctgacgaaagaaggtctaatcaagatcttagaaatcgccatg gaaatgaacacaggtaaccacgagagactgcgacgagtccttgaagagatccggggactggatgaagatacagtccacccccatt ccgaaaggatgggataagggtgagcgaaattccttgtcgggtaagttccgacctgcatgaat (SEQ ID NO: 53) |
| *Thermotoga naphthophila*-P1-5' intron & exon E1 |
| ccccggtaaacggcggccgtaactataacggtcctaaggtagcattcgctgcctgcccacagggaaacctgtggggcaatacccg gctgtatgcgggaaagcccttagagcccttcctacctgggggctgcggccagcccaggtaaaaatggaagggattgggtaatccg caggaaaccccgatgagggggatcctcagagactgtacgccgggcatccagtccccgtgagaaggggtggaaatcttggatctca agcctgattgggttgttggattcgtcgatggtgaaggttgtttctacgtgggtgttaatagaaatcgcaccatgaagacaggttaccaag ttctccctgaattcaggattgttc (SEQ ID NO: 54) |
| *Thermotoga naphthophila*-P2-3' intron & exon E2 |
| agttctttgggtgcggtgttgtacgaaaaaaccacgatgatcgatatgaacttcggatccggaagaagtcttgcttgaagaaagtagtg gaattcttcgaaaagcaccctctgaaaacgaagaagaacgttgacttcaagaagttcagaagaataatcctgatgatggaacgggga gaacacctgacgaaagaaggtctaatcaagatcttagaaatcgccatggaaatgaacacaggtaaccacgagagactgcgacgag tccttgaagagatccggggactggatgaagatacagtccacccccattccgaaaggatgggataagggtgagcgaaattccttgtcg ggtaagttccgacctgcatgaat (SEQ ID NO: 55) |
| *Thermotoga naphthophila*-P2-5' intron & exon E1 |
| ccccggtaaacggcggccgtaactataacggtcctaaggtagcattcgctgcctgcccacagggaaacctgtggggcaatacccg gctgtatgcgggaaagcccttagagcccttcctacctgggggctgcggccagcccaggtaaaaatggaagggattgggtaatccg caggaaaccccgatgagggggatcctcagagactgtacgccgggcatccagtccccgtgagaaggggtggaaatcttggatctca agcctgattgggttgttggattcgtcgatggtgaaggttgtttctacgtgggtgttaatagaaatcgcaccatgaagacaggttaccaag ttctccctgaattcaggattgttcagcatgaaagggatatacagattctgtacgcattgaaga (SEQ ID NO: 56) |
| *Thermotoga naphthophila*-P4-3' intron & exon E2 |
| cggccagcccaggtaaaaatggaagggattgggtaatccgcaggaaaccccgatgagggggatcctcagagactgtacgccgg gcatccagtccccgtgagaaggggtggaaatcttggatctcaagcctgattgggttgttggattcgtcgatggtgaaggttgtttctac gtgggtgttaatagaaatcgcaccatgaagacaggttaccaagttctccctgaattcaggattgttcagcatgaaagggatatacagat tctgtacgcattgaagaagttctttgggtgcggtgttgtacgaaaaaaccacgatgatcgatatgaacttcggatccggaagaagtctt gcttgaagaaagtagtggaattcttcgaaaagcaccctctgaaaacgaagaagaacgttgacttcaagaagttcagaagaataatcct gatgatggaacggggagaacacctgacgaaagaaggtctaatcaagatcttagaaatcgccatggaaatgaacacaggtaaccac gagagactgcgacgagtccttgaagagatccggggactggatgaagatacagtccacccccattccgaaaggatgggataagggt gagcgaaattccttgtcgggtaagttccgacctgcatgaat (SEQ ID NO: 57) |
| *Thermotoga naphthophila*-P4-5' intron & exon E1 |
| ccccggtaaacggcggccgtaactataacggtcctaaggtagcattcgctgcctgcccacagggaaacctgtggggcaatacccg gctgtatgcgggaaagcccttagagcccttcctacctgggggctg (SEQ ID NO: 58) |
| Intron & exon derived from *Coxiella burnetii-2* |
| cgaaattccttgtcgggtaaaaaattgccccgctaaactgtaaagtttagtgataaagtcagctcatatcgggggaaccctcctgcttttt agcaaagggcaatcccgagggaagtcttaaatgaccccgtaacgactgatccgaaaggtgaggcacgcatttgtgtgcaatacgct gacttcttgcccttgggtttatactcaagaagtaagtgactatggagaatttatttatgtatttacatttagagccatggtatgtaagtggttt aacagaaggagaagggtgtttttcgatatcatttaacttcaGaaaaaagttaaaaattggtattgaaacaagaccatctttttccatcacg ttaaatcaaagagatttacctctcttaaaagagatacattcgttttttgaatgtggagcaattagattttcacgaggggatcgtacgtatAa atatgaagtccgttccgtaaaagatcttgtgaagcgaataattccacacttcgataactatcctcttagaggaggtaaacaaaaagatttt cgatatttttctgaaatctgtaaaaaaatacataccaattttcatttaaataaacaacatttaattgaaataataaaaatggcttatctgatga atccatcaggtaaacgtaggcatgaaataaatgatctcctaaggttgcttggtgagaaaatggtatagtctactccctatggaaacatag gaaagagtgaagttccgacctgcacgaatgg (SEQ ID NO: 59) |
| Intron & exon derived from *Scytonema hofmanni* |
| ctggcgaggctcaacgagtaagattgactaaacgcttaaaagttattcttactatgggcggtacgtaaagAaacttacgtatgtttacct gtcaaactcggggaagccaatagcgcggtaatcccgaaccaagctccagAaatggagaaggtgtagagactggaaggcaggta ccctaacagtAaagctgagggtaaagggacagtccagaccacAaactggttaatccaggcagtgaaaactgtagatggtaagcat aacctacggcaa (SEQ ID NO: 60) |
| Intron & exon derived from *Synechococcus elongatus* |
| acggtagacgcagcggacttagaaaactgggcctcgatcgcgaaagggatcgagtggcagctctcaaactcagggaaacctaaa actttaaacattaaagtcatggcaatcctgagccaagctaaagctaaacaActaacagctttagaaggtgcagagactagacggga gctaccctaacggatTcagccgagggtaaagggatagtccaattctcaacatcgcgattgttgatggcagcgaaagttgcagagag aatgaaaatccgctgactgtaaaggtcg (SEQ ID NO: 61) |
| Intron & exon derived from *Thermotoga subterranea* |
| ggtaaacggcggccgtaactataaagaccgttgtagttacggtaaaaaactcggctgtatgcgggaaactctggagtgccggtgggt accacaGgtgtggtaacaatcccacggagtgcgaaagcgccagacaacccgcaggaaactcatctaagttaaggagaattttccat gctggacgttaaaagaattccaccgaaaataggttactaccttggcaggctttgcagacggagaagggagtttcatggttgttgtaag aaagaaagatgattataaaagtggttggaagatatctgtagctttcaatgtctcgaataaagataaggtagtgcTtgcactttttaaaag gtatttgaactgtggaactctccgtcagcgtaaagatggtgtgtggtactatgaagtaggtaatttcaacgcgattgtggaaaatgtgat acctttctttgacagattcagatttctttctgcagccaagagaaattcatAttcaaaattcaaaaaagttgtagaaataattggaagatctg aacatacgacaaaggaaggcattgaaaagatattatctttgagGaatcaaatgagtaataatcggtcaaagcgaaaacacaccgatc aggaagttttgggtagcttagatgaggatcctcagagaccatacgccgagcatcacatgggtttgaccccaaacggggatgcgccg ccgacccatggatgatgatatggtccaatcctatggtaacatagggcctaaaatgggatagcctcaattgaggtgaatgaagtattaac gaacggtcctaaggtagcgaa (SEQ ID NO: 62) |
| *Coxiella burnetii*-2-P1-3' intron & exon E2 |
| Aaaaaagttaaaaattggtattgaaacaagaccatctttttccatcacgttaaatcaaagagatttacctctcttaaaagagatacattcgt tttttgaatgtggagcaattagattttcacgaggggatcgtacgtataaatatgaagtccgttccgtaaaagatcttgtgaagcgaataatt ccacacttcgataactatcctcttagaggaggtaaacaaaaagattttcgatatttttctgaaatctgtaaaaaaatacataccaattttcat ttaaataaacaacatttaattgaaataataaaaatggcttatctgatgaatccatcaggtaaacgtaggcatgaaataaatgatctcctaa ggttgcttggtgagaaaatggtatagtctactccctatggaaacataggaaagagtgaagttccgacctgcacgaatgg (SEQ ID NO: 67) |
| *Coxiella burnetii*2-P1-5' intron & exon E1 |
| Cgaaattccttgtcgggtaaaaaattgccccgctaaactgtaaagtttagtgataaagtcagctcatatcgggggaaccctcctgctttt tagcaaagggcaatcccgagggaagtcttaaatgaccccgtaacgactgatccgaaaggtgaggcacgcatttgtgtgcaatacgc tgacttcttgcccttgggtttatactcaagaagtaagtgactatggagaatttatttatgtatttacatttagagccatggtatgtaagtggttt aacagaaggagaagggtgtttttcgatatcatttaacttcag (SEQ ID NO: 68) |
| *Coxiella burnetii*2-P2-3' intron & exon E2 |
| Aatatgaagtccgttccgtaaaagatcttgtgaagcgaataattccacacttcgataactatcctcttagaggaggtaaacaaaaagat tttcgatatttttctgaaatctgtaaaaaaatacataccaattttcatttaaataaacaacatttaattgaaataataaaaatggcttatctgatg aatccatcaggtaaacgtaggcatgaaataaatgatctcctaaggttgcttggtgagaaaatggtatagtctactccctatggaaacata ggaaagagtgaagttccgacctgcacgaatgg (SEQ ID NO: 69) |
| *Coxiella burnetii*2-P2-5' intron & exon E1 |
| Cgaaattccttgtcgggtaaaaaattgccccgctaaactgtaaagtttagtgataaagtcagctcatatcgggggaaccctcctgctttt tagcaaagggcaatcccgagggaagtcttaaatgaccccgtaacgactgatccgaaaggtgaggcacgcatttgtgtgcaatacgc tgacttcttgcccttgggtttatactcaagaagtaagtgactatggagaatttatttatgtatttacatttagagccatggtatgtaagtggttt aacagaaggagaagggtgtttttcgatatcatttaacttcagaaaaaagttaaaaattggtattgaaacaagaccatctttttccatcacgt taaatcaaagagatttacctctcttaaaagagatacattcgttttttgaatgtggagcaattagattttcacgaggggatcgtacgtata (SEQ ID NO: 70) |
| *Scytonema hofmanni*-P1-3' intron & exon E2 |
| Aacttacgtatgtttacctgtcaaactcggggaagccaatagcgcggtaatcccgaaccaagctccagaaatggagaaggtgtaga gactggaaggcaggtaccctaacagtaaagctgagggtaaagggacagtccagaccacaaactggttaatccaggcagtgaaaac tgtagatggtaagcataacctacggcaa (SEQ ID NO: 71) |
| *Scytonema hofmanni-*P1-5' intron & exon E1 |
| Ctggcgaggctcaacgagtaagattgactaaacgcttaaaagttattcttactatgggcggtacgtaaaga (SEQ ID NO: 72) |
| *Scytonema hofmanni*-P2-3' intron & exon E2 |
| Aatggagaaggtgtagagactggaaggcaggtaccctaacagtaaagctgagggtaaagggacagtccagaccacaaactggtt aatccaggcagtgaaaactgtagatggtaagcataacctacggcaa (SEQ ID NO: 73) |
| *Scytonema hofmanni-*P2-5' intron & exon E1 |
| Ctggcgaggctcaacgagtaagattgactaaacgcttaaaagttattcttactatgggcggtacgtaaagaaacttacgtatgtttacct gtcaaactcggggaagccaatagcgcggtaatcccgaaccaagctccaga (SEQ ID NO: 74) |
| *Scytonema hofmanni*-P3-3' intron & exon E2 |
| Aagctgagggtaaagggacagtccagaccacaaactggttaatccaggcagtgaaaactgtagatggtaagcataacctacggca a (SEQ ID NO: 75) |
| *Scytonema hofmanni*-P3-5' intron & exon E1 |
| Ctggcgaggctcaacgagtaagattgactaaacgcttaaaagttattcttactatgggcggtacgtaaagaaacttacgtatgtttacct gtcaaactcggggaagccaatagcgcggtaatcccgaaccaagctccagaaatggagaaggtgtagagactggaaggcaggtac cctaacagta (SEQ ID NO: 76) |
| *Scytonema hojinanni*-P4-3' intron & exon E2 |
| Aactggttaatccaggcagtgaaaactgtagatggtaagcataacctacggcaa (SEQ ID NO: 77) |
| *Scytonema hofmanni*-P4-5' intron & exon E1 |
| Ctggcgaggctcaacgagtaagattgactaaacgcttaaaagttattcttactatgggcggtacgtaaagaaacttacgtatgtttacct gtcaaactcggggaagccaatagcgcggtaatcccgaaccaagctccagaaatggagaaggtgtagagactggaaggcaggtac cctaacagtaaagctgagggtaaagggacagtccagaccaca (SEQ ID NO: 78) |
| *Synechococcus elongatus* -P1-3' intron & exon E2 |
| Ctaacagctttagaaggtgcagagactagacgggagctaccctaacggattcagccgagggtaaagggatagtccaattctcaaca tcgcgattgttgatggcagcgaaagttgcagagagaatgaaaatccgctgactgtaaaggtcg (SEQ ID NO: 79) |
| *Synechococcus elongatus* -P1-5' intron & exon E1 |
| Acggtagacgcagcggacttagaaaactgggcctcgatcgcgaaagggatcgagtggcagctctcaaactcagggaaacctaaa actttaaacattaaagtcatggcaatcctgagccaagctaaagctaaacaa (SEQ ID NO: 80) |
| *Synechococcus elongatus* -P2-3' intron & exon E2 |
| Cagccgagggtaaagggatagtccaattctcaacatcgcgattgttgatggcagcgaaagttgcagagagaatgaaaatccgctga ctgtaaaggtcg (SEQ ID NO: 81) |
| *Synechococcus elongatus* -P2-5' intron & exon E1 |
| Acggtagacgcagcggacttagaaaactgggcctcgatcgcgaaagggatcgagtggcagctctcaaactcagggaaacctaaa actttaaacattaaagtcatggcaatcctgagccaagctaaagctaaacaactaacagctttagaaggtgcagagactagacgggag ctaccctaacggatt (SEQ ID NO: 82) |
| *Thermotoga subterranea*-P1-3' intron & exon E2 |
| Gtgtggtaacaatcccacggagtgcgaaagcgccagacaacccgcaggaaactcatctaagttaaggagaattttccatgctggac gttaaaagaattccaccgaaaataggttactaccttggcaggctttgcagacggagaagggagtttcatggttgttgtaagaaagaaa gatgattataaaagtggttggaagatatctgtagctttcaatgtctcgaataaagataaggtagtgcttgcactttttaaaaggtatttgaa ctgtggaactctccgtcagcgtaaagatggtgtgtggtactatgaagtaggtaatttcaacgcgattgtggaaaatgtgatacctttcttt gacagattcagatttctttctgcagccaagagaaattcatattcaaaattcaaaaaagttgtagaaataattggaagatctgaacatacga caaaggaaggcattgaaaagatattatctttgaggaatcaaatgagtaataatcggtcaaagcgaaaacacaccgatcaggaagtttt gggtagcttagatgaggatcctcagagaccatacgccgagcatcacatgggtttgaccccaaacggggatgcgccgccgacccat ggatgatgatatggtccaatcctatggtaacatagggcctaaaatgggatagcctcaattgaggtgaatgaagtattaacgaacggtc ctaaggtagcgaa (SEQ ID NO: 83) |
| *Thermotoga subterranea*-P1-5' intron & exon E1 |
| Ggtaaacggcggccgtaactataaagaccgttgtagttacggtaaaaaactcggctgtatgcgggaaactctggagtgccggtggg taccacag (SEQ ID NO: 84) |
| *Thermotoga subterranea*-P2-3' intron & exon E2 |
| Tgcactttttaaaaggtatttgaactgtggaactctccgtcagcgtaaagatggtgtgtggtactatgaagtaggtaatttcaacgcgatt gtggaaaatgtgatacctttctttgacagattcagatttctttctgcagccaagagaaattcatattcaaaattcaaaaaagttgtagaaat aattggaagatctgaacatacgacaaaggaaggcattgaaaagatattatctttgaggaatcaaatgagtaataatcggtcaaagcga aaacacaccgatcaggaagttttgggtagcttagatgaggatcctcagagaccatacgccgagcatcacatgggtttgaccccaaac ggggatgcgccgccgacccatggatgatgatatggtccaatcctatggtaacatagggcctaaaatgggatagcctcaattgaggtg aatgaagtattaacgaacggtcctaaggtagcgaa (SEQ ID NO: 85) |
| *Thermotoga subterranea*-P2-5' intron & exon E1 |
| Ggtaaacggcggccgtaactataaagaccgttgtagttacggtaaaaaactcggctgtatgcgggaaactctggagtgccggtggg taccacaggtgtggtaacaatcccacggagtgcgaaagcgccagacaacccgcaggaaactcatctaagttaaggagaattttccat gctggacgttaaaagaattccaccgaaaataggttactaccttggcaggctttgcagacggagaagggagtttcatggttgttgtaag aaagaaagatgattataaaagtggttggaagatatctgtagctttcaatgtctcgaataaagataaggtagtgct (SEQ ID NO: 86) |
| *Thermotoga subterranea*-P3-3' intron & exon E2 |
| Ttcaaaattcaaaaaagttgtagaaataattggaagatctgaacatacgacaaaggaaggcattgaaaagatattatctttgaggaatc aaatgagtaataatcggtcaaagcgaaaacacaccgatcaggaagttttgggtagcttagatgaggatcctcagagaccatacgccg agcatcacatgggtttgaccccaaacggggatgcgccgccgacccatggatgatgatatggtccaatcctatggtaacatagggcct aaaatgggatagcctcaattgaggtgaatgaagtattaacgaacggtcctaaggtagcgaa (SEQ ID NO: 87) |
| *Thermotoga subterranea*-P3-5' intron & exon E1 |
| Ggtaaacggcggccgtaactataaagaccgttgtagttacggtaaaaaactcggctgtatgcgggaaactctggagtgccggtggg taccacaggtgtggtaacaatcccacggagtgcgaaagcgccagacaacccgcaggaaactcatctaagttaaggagaattttccat gctggacgttaaaagaattccaccgaaaataggttactaccttggcaggctttgcagacggagaagggagtttcatggttgttgtaag aaagaaagatgattataaaagtggttggaagatatctgtagctttcaatgtctcgaataaagataaggtagtgcttgcactttttaaaagg tatttgaactgtggaactctccgtcagcgtaaagatggtgtgtggtactatgaagtaggtaatttcaacgcgattgtggaaaatgtgata cctttctttgacagattcagatttctttctgcagccaagagaaattcata (SEQ ID NO: 88) |
| *Thermotoga subterranea*-P4-3' intron & exon E2 |
| aatcaaatgagtaataatcggtcaaagcgaaaacacaccgatcaggaagttttgggtagcttagatgaggatcctcagagaccatac gccgagcatcacatgggtttgaccccaaacggggatgcgccgccgacccatggatgatgatatggtccaatcctatggtaacatag ggcctaaaatgggatagcctcaattgaggtgaatgaagtattaacgaacggtcctaaggtagcgaa (SEQ ID NO: 89) |
| *Thermotoga subterranea*-P4-5' intron & exon E1 |
| Ggtaaacggcggccgtaactataaagaccgttgtagttacggtaaaaaactcggctgtatgcgggaaactctggagtgccggtggg taccacaggtgtggtaacaatcccacggagtgcgaaagcgccagacaacccgcaggaaactcatctaagttaaggagaattttccat gctggacgttaaaagaattccaccgaaaataggttactaccttggcaggctttgcagacggagaagggagtttcatggttgttgtaag aaagaaagatgattataaaagtggttggaagatatctgtagctttcaatgtctcgaataaagataaggtagtgcttgcactttttaaaagg tatttgaactgtggaactctccgtcagcgtaaagatggtgtgtggtactatgaagtaggtaatttcaacgcgattgtggaaaatgtgata cctttctttgacagattcagatttctttctgcagccaagagaaattcatattcaaaattcaaaaaagttgtagaaataattggaagatctgaa catacgacaaaggaaggcattgaaaagatattatctttgagg (SEQ ID NO: 90) |
| EGFP-F (Sequencing primers for circularization junction) |
| Gccgggatcactctcggcatggacgagctgtacaagtaa (SEQ ID NO: 91) |
| EGFP-F (Sequencing primers for circularization junction) |
| Gggcctgtgggtgggatcaacccacaggctgttttaa (SEQ ID NO: 92) |

Although the present application has been described in conjunction with one or more embodiments, it should be understood that the present application is not limited to those embodiments. The description in the present application is intended to cover all variants and equivalents, which are within the gist and scope of the attached claims. All documents cited herein are incorporated herein by reference in their entirety.

### References

1. Baden, L. R. et al. Efficacy and safety of the mRNA-1273 SARS-CoV-2 vaccine. N. Engl. J. Med. 384, 403-416 (2021).
2. Walsh, E. E. et al. Safety and immunogenicity of two RNA-based Covid-19 vaccine candidates. N. Engl. J. Med. 383, 2439-2450 (2020).
3. Kaczmarek, J. C., Kowalski, P. S. & Anderson, D. G. Advances in the delivery of RNA therapeutics: from concept to clinical reality. Genome Med. 9, 60 (2017).
4. Holtkamp, S. et al. Modification of antigen-encoding RNA increases stability, translational efficacy, and T-cell stimulatory capacity of dendritic cells. Blood 108, 4009-4017 (2006).
5. Kuhn, A. N. et al. Phosphorothioate cap analogs increase stability and translational efficiency of RNA vaccines in immature dendritic cells and induce superior immune responses in vivo. Gene Ther. 17, 961-971 (2010).
6. Presnyak, V. et al. Codon optimality is a major determinant of mRNA stability. Cell 160, 1111-1124 (2015).
7. Kariko, K., Muramatsu, H., Ludwig, J. & Weissman, D. Generating the optimal mRNA for therapy: HPLC purification eliminates immune activation and improves translation of nucleoside-modified, protein-encoding mRNA. Nucleic Acids Res. 39, e142-e142 (2011).
8. Jeck, W. R. & Sharpless, N. E. Detecting and characterizing circular RNAs. Nat. Biotechnol. 32, 453-461 (2014).
9. Barrett, S. P. & Salzman, J. Circular RNAs: analysis, expression and potential functions. Development 143, 1838-1847 (2016).
10. Hansen, T. B. et al. Natural RNA circles function as efficient microRNA sponges. Nature 495, 384-388 (2013).
11. Li, Z. et al. Exon-intron circular RNAs regulate transcription in the nucleus. Nat. Struct. Mol. Biol. 22, 256-264 (2015)
12. Chen, L.-L. & Yang, L. Regulation of circRNA biogenesis. RNA Biol. 12, 381-388 (2015).
13. Jeck, W. R. & Sharpless, N. E. Detecting and characterizing circular RNAs. Nat. Biotechnol. 32, 453-461 (2014).
14. Wang, Y. & Wang, Z. Efficient backsplicing produces translatable circular mRNAs. RNA 21, 172-179 (2015).
15. Legnini, I. et al. Circ-ZNF609 is a circular RNA that can be translated and functions in myogenesis. Mol. Cell 66, 22-37.e9 (2017).
16. Pamudurti, N. R. et al. Translation of CircRNAs. Mol. Cell 66, 9-21.e7 (2017).
17. Enuka, Y. et al. Circular RNAs are long-lived and display only minimal early alterations in response to a growth factor. Nucleic Acids Res. 44, 1370-1383 (2016).
18. Cech, T. R. 1990. Self-splicing of group I introns. Annu. Rev. Biochem. 59:543-568.
19. Saldanha, R., G. Mohr, M. Belfort, and A. M. Lambowitz. 1993. Group I and group II introns. FASEB J. 7:15-24
20. Jacquier, A. 1996. Group II introns: elaborate ribozymes. Biochimie 78:474- 487.
21. Wesselhoeft, R. A., Kowalski, P. S. & Anderson, D. G. Engineering circular RNA for potent and stable translation in eukaryotic cells. Nat. Commun. 9, 2629 (2018).
22. Chen R, Wang SK, Belk JA, Amaya L, Li Z, Cardenas A, Abe BT, Chen CK, Wender PA, Chang HY. Engineering circular RNA for enhanced protein production. Nat Biotechnol. 2023 Feb;41(2):262-272.
23. Chen, C., Wei, H., Zhang, K., Li, Z., Wei, T., Tang, C., Yang, Y., & Wang, Z. (2022). A flexible, efficient, and scalable platform to produce circular RNAs as new therapeutics. bioRxiv.
24. Qiu, Z., Zhao, Y., Hou, Q., Zhu, J., Zhai, M., Li, D., Li, Y., Liu, C., Li, N., Cao, Y., Yang, J., Sun, Z., & Zuo, C. (2022). Clean-PIE: a novel strategy for efficiently constructing precise circRNA with thoroughly minimized immunogenicity to direct potent and durable protein expression. bioRxiv
25. Atchison RW, Casto BC, Hammon WM. Adenovirus-associated defective virus particles. Science 1965; 149: 754-6. [PubMed: 14325163].
26. Carter BJ. Adeno-associated virus and the development of adeno-associated virus vectors: a historical perspective. Mol Ther 2004; 10(6): 981-9. [PubMed: 15564130].
27. Flotte TR, Berns KI. Adeno-associated virus: a ubiquitous commensal of mammals. Hum Gene Ther 2005; 16(4): 401-7. [PubMed: 15871671].
28. Maguire AM, Simonelli F, Pierce EA et al: Safety and efficacy of gene transfer for Leber's congenital amaurosis. N Engl J Med, 2008; 358(21): 2240-48.
29. Bainbridge JW, Smith AJ, Barker SS et al: Effect of gene therapy on visual function in Leber's congenital amaurosis N Engl J Med, 2008; 358(21): 2231-39.
30. Wilmott P, Lisowski L, Alexander IE, Logan GJ. A User's Guide to the Inverted Terminal Repeats of Adeno-Associated Virus. Human gene therapy methods 2019; 30(6): 206-213. [PubMed: 31752513]
31. Duan D, Yan Z, Yue Y, Engelhardt JF. Structural analysis of adeno-associated virus transduction intermediates. Virology 1999; 261(1): 8-14. [PubMed: 10484751].
32. Pan X, Yue Y, Boftsi M, Wasala LP, Tran NT, Zhang K, Pintel DJ, Tai PWL, Duan D. Rational engineering of a functional CpG-free ITR for AAV gene therapy. Gene Ther. 2022 Jun;29(6):333-345.

## Claims

1. A single-stranded DNA molecule, **characterized by** sequentially comprising from the 5' end to the 3' end: a) a 3' self-splicing intron fragment containing a 3' splice site, and b) a 5' self-splicing intron fragment containing a 5' splice site, wherein the elements are operatively connected,
wherein the 3' self-splicing intron fragment containing the 3' splice site and the 5' self-splicing intron fragment containing the 5' splice site are derived from introns of the recA gene of *Bacillus anthracis,* the 23S ribosomal RNA gene of *Coxiella burnetii,* the tRNA-fMet gene of *Scytonema hofmanni,* the tRNA-fMet gene of *Synechocystis sp.,* the 23S rRNA gene of *Thermotoga naphthophila,* the ndhL and trnL-UAA genes of *Synechococcus elongatus,* or the 23S rRNA gene of *Thermotoga subterranea.*

2. The single-stranded DNA molecule of claim 1, **characterized in that** the intron of the recA gene of *Bacillus anthracis* comprises a nucleotide sequence that has at least 75%, 80%, 85%, 90%, 91%, 92%, 93%, 94%, 95%, 96%, 97%, 98%, 99% or 100% sequence identity to SEQ ID NO: 18;
the intron of the 23S ribosomal RNA gene of *Coxiella burnetii* comprises a nucleotide sequence that has at least 75%, 80%, 85%, 90%, 91%, 92%, 93%, 94%, 95%, 96%, 97%, 98%, 99% or 100% sequence identity to SEQ ID NO: 19 or 59;
the intron of the tRNA-fMet gene of *Synechocystis sp.* comprises a nucleotide sequence that has at least 75%, 80%, 85%, 90%, 91%, 92%, 93%, 94%, 95%, 96%, 97%, 98%, 99% or 100% sequence identity to SEQ ID NO: 20;
the intron of the 23S rRNA gene of *Thermotoga naphthophila* comprises a nucleotide sequence that has at least 75%, 80%, 85%, 90%, 91%, 92%, 93%, 94%, 95%, 96%, 97%, 98%, 99% or 100% sequence identity to SEQ ID NO: 21;
the intron of the tRNA-fMet gene of *Scytonema hofmanni* comprises a nucleotide sequence that has at least 75%, 80%, 85%, 90%, 91%, 92%, 93%, 94%, 95%, 96%, 97%, 98%, 99% or 100% sequence identity to SEQ ID NO: 60;
the introns of the ndhL and trnL-UAA genes of *Synechococcus elongatus* comprise a nucleotide sequence that has at least 75%, 80%, 85%, 90%, 91%, 92%, 93%, 94%, 95%, 96%, 97%, 98%, 99% or 100% sequence identity to SEQ ID NO: 61; or
the intron of the 23S rRNA gene of *Thermotoga subterranea* comprises a nucleotide sequence that has at least 75%, 80%, 85%, 90%, 91%, 92%, 93%, 94%, 95%, 96%, 97%, 98%, 99% or 100% sequence identity to SEQ ID NO: 62.

3. The single-stranded DNA molecule of claim 1 or 2, **characterized in that** the 3' self-splicing intron fragment containing the 3' splice site and the 5' self-splicing intron fragment containing the 5' splice site are obtained by truncating SEQ ID NO: 18 after the nucleotide at position 133, 195 or 294 of SEQ ID NO: 18;
the 3' self-splicing intron fragment containing the 3' splice site and the 5' self-splicing intron fragment containing the 5' splice site are obtained by truncating SEQ ID NO: 19 after the nucleotide at position 100, 157, 206 or 245 of SEQ ID NO: 19;
the 3' self-splicing intron fragment containing the 3' splice site and the 5' self-splicing intron fragment containing the 5' splice site are obtained by truncating SEQ ID NO: 20 after the nucleotide at position 112, 246, 298, 393 or 500 of SEQ ID NO: 20;
the 3' self-splicing intron fragment containing the 3' splice site and the 5' self-splicing intron fragment containing the 5' splice site are obtained by truncating SEQ ID NO: 21 after the nucleotide at position 130, 314, 369 or 408 of SEQ ID NO: 21;
the 3' self-splicing intron fragment containing the 3' splice site and the 5' self-splicing intron fragment containing the 5' splice site are obtained by truncating SEQ ID NO: 59 after the nucleotide at position 313 or 453 of SEQ ID NO: 59;
the 3' self-splicing intron fragment containing the 3' splice site and the 5' self-splicing intron fragment containing the 5' splice site are obtained by truncating SEQ ID NO: 60 after the nucleotide at position 71, 140, 184 or 216 of SEQ ID NO: 60;
the 3' self-splicing intron fragment containing the 3' splice site and the 5' self-splicing intron fragment containing the 5' splice site are obtained by truncating SEQ ID NO: 61 after the nucleotide at position 135 or 186 of SEQ ID NO: 61; or
the 3' self-splicing intron fragment containing the 3' splice site and the 5' self-splicing intron fragment containing the 5' splice site are obtained by truncating SEQ ID NO: 62 after the nucleotide at position 94, 336, 492 or 577 of SEQ ID NO: 62.

4. The single-stranded DNA molecule of any one of claims 1-3, **characterized in that** the 3' self-splicing intron fragment containing the 3' splice site and the 5' self-splicing intron fragment containing the 5' splice site comprise nucleotide sequences having at least 75%, 80%, 85%, 90%, 91%, 92%, 93%, 94%, 95%, 96%, 97%, 98%, 99% or 100% sequence identity to i) SEQ ID NOs: 41 and 42, ii) SEQ ID NOs: 43 and 44, iii) SEQ ID NOs: 47 and 48, iv) SEQ ID NOs: 75 and 76, v) SEQ ID NOs: 39 and 40, vi) SEQ ID NOs: 49 and 50, vii) SEQ ID NOs: 51 and 52, viii) SEQ ID NOs: 53 and 54, ix) SEQ ID NOs: 55 and 56, x) SEQ ID NOs: 57 and 58, xi) SEQ ID NOs: 67 and 68, xii) SEQ ID NOs: 69 and 70, xiii) SEQ ID NOs: 71 and 72, xiv) SEQ ID NOs: 73 and 74, xv) SEQ ID NOs: 45 and 46, xvi) SEQ ID NOs: 77 and 78, xvii) SEQ ID NOs: 79 and 80, xviii) SEQ ID NOs: 81 and 82, xix) SEQ ID NOs: 83 and 84, xx) SEQ ID NOs: 85 and 86, xxi) SEQ ID NOs: 87 and 88, or xxii) SEQ ID NOs: 89 and 90, respectively.

5. The single-stranded DNA molecule of any one of claims 1-4, **characterized by** further comprising a target gene between the 3' self-splicing intron fragment containing the 3' splice site and the 5' self-splicing intron fragment containing the 5' splice site.

6. The single-stranded DNA molecule of claim 5, **characterized in that** the target gene is a protein coding sequence or a non-coding sequence.

7. The single-stranded DNA molecule of any one of claims 1-6, **characterized by** further comprising a 5' homology arm at the 5' end of the 3' self-splicing intron fragment containing the 3' splice site and a 3' homology arm at the 3' end of the 5' self-splicing intron fragment containing the 5' splice site.

8. The single-stranded DNA molecule of claim 7, **characterized in that** the sequences of the 5' homology arm and the 3' homology arm are capable of complementarily forming a stem-loop structure, wherein
the 5' end of the 5' homology arm and the 3' end of the 3' homology arm are adjacent in the stem;
the sequences of the 5' homology arm and the 3' homology arm are capable of complementarily forming a structure containing a palindromic stem and a stem-loop, wherein the palindromic stem is connected to the stem of the stem-loop, wherein the 5' end of the 5' homology arm and the 3' end of the 3' homology arm are adjacent in the palindromic stem; or
the sequences of the 5' homology arm and the 3' homology arm are capable of complementarily forming a structure containing a palindromic stem and two stem-loops, wherein the palindromic stem is connected to the stems of the two stem-loops, wherein the 5' end of the 5' homology arm and the 3' end of the 3' homology arm are adjacent in the palindromic stem or in the loop of one of the stem-loops.

9. The single-stranded DNA molecule of claim 7 or 8, **characterized in that** the 5' homology arm and/or the 3' homology arm comprises a CpG-free sequence.

10. The single-stranded DNA molecule of any one of claims 5-9, **characterized by** further comprising an internal ribosome entry site (IRES) encoding sequence between the 3' self-splicing intron fragment containing the 3' splice site and the target gene.

11. The single-stranded DNA molecule of claim 10, **characterized in that** the IRES is selected from the IRESs of Coxsackie virus B3 (CVB3), Coxsackie virus A (CVB1/2), Taura syndrome virus, Triatoma virus, Theiler's encephalomyelitis virus, Simian virus 40, Solenopsis invicta virus 1, Rhopalosiphum padi virus, Reticuloendotheliosis virus, Human poliovirus 1, Soybean looper virus, Kashmir bee virus, Human rhinovirus 2, Homalodisca coagulata virus-1, Human immunodeficiency virus type 1, Louse P virus, Hepatitis C virus, Hepatitis A virus, Hepatitis GB virus, Foot and mouth disease virus, Human enterovirus 71, Equine rhinitis virus, Ectropis obliqua picorna-like virus, Encephalomyocarditis virus (EMCV), Drosophila C virus, Crucifer tobamovirus, Cricket paralysis virus, Bovine viral diarrhea virus 1, Black queen cell virus, Aphid lethal paralysis virus, Avian encephalomyelitis virus, Acute bee paralysis virus, Hibiscus chlorotic ringspot virus, Classical swine fever virus, Human FGF2, Human SFTPA1, Human AML1/RLTNX1, *Drosophila* antennapedia, Human AQP4, Human AT1R, Human BAG-1, Human BCL2, Human BiP, Human c-IAPl, Human c-myc, Human eIF4G, Mouse NDST4L, Human LEF1, Mouse HIF1α, Human n.myc, Mouse Gtx, Human p27kipl, Human PDGF2/c-sis, Human p53, Human Pim-1, Mouse Rbm3, *Drosophila* reaper, Canine Scamper, *Drosophila* Ubx, Salivirus, Cosavirus, Parechovirus, Human UNR, Mouse UtrA, Human VEGF-A, Human XIAP, *Drosophila* hairless, *Saccharomyces cerevisiae* TFIID, *Saccharomyces cerevisiae* YAP1, Human c-src, Human FGF-1, Simian picornavirus, Turnip crinkle virus and an eIF4G aptamer.

12. The single-stranded DNA molecule of claim 10 or 11, **characterized by** further comprising a 5' spacer sequence between the 3' self-splicing intron fragment containing the 3' splice site and the IRES encoding sequence.

13. The single-stranded DNA molecule of any one of claims 5-12, **characterized by** further comprising a 3' spacer sequence between the target gene and the 5' self-splicing intron fragment containing the 5' splice site.

14. The single-stranded DNA molecule of claim 12 or 13, **characterized in that** the 5' spacer sequence and/or 3' spacer sequence is at least 10 nucleotides in length.

15. The single-stranded DNA molecule of any one of claims 7-14, **characterized by** further comprising an RNA polymerase promoter upstream of the 5' homology arm.

16. The single-stranded DNA molecule of claim 15, **characterized in that** the RNA polymerase promoter is an RNA polymerase promoter derived from T7 virus, T6 virus, SP6 virus, T3 virus or T4 virus.

17. The single-stranded DNA molecule of any one of claims 1-16, **characterized by** comprising:
a) a 5' homology arm, b) a 3' self-splicing intron fragment containing a 3' splice site, c) a 5' spacer sequence, d) an internal ribosome entry site encoding sequence, e) a protein coding sequence, f) a 3' spacer sequence, g) a 5' self-splicing intron fragment containing a 5' splice site, and h) a 3' homology arm; or
a) a 5' homology arm, b) a 3' self-splicing intron fragment containing a 3' splice site, c) a non-coding sequence, d) a 5' self-splicing intron fragment containing a 5' splice site, and e) a 3' homology arm.

18. A double-stranded DNA molecule, **characterized by** comprising: i) the single-stranded DNA molecule of any one of claims 1-17, and ii) a second strand complementary to the single-stranded DNA molecule.

19. A vector for preparing a circular RNA, **characterized by** comprising the single-stranded DNA molecule of any one of claims 1-17 or the double-stranded DNA molecule of claim 18.

20. A method for preparing a circular RNA, **characterized by** comprising: i) *in vitro* transcribing a precursor RNA from the vector of claim 19 under suitable conditions, and ii) incubating the precursor RNA under suitable conditions, wherein the suitable conditions in step ii) comprise the presence of magnesium ions and guanosine triphosphate.

21. A method for preparing a circular RNA molecule, **characterized by** comprising subjecting the vector of claim 19 to *in vitro* transcription under suitable conditions, wherein the 3' self-splicing intron fragment containing the 3' splice site and the 5' self-splicing intron fragment containing the 5' splice site in the vector comprise nucleotide sequences having at least 75%, 80%, 85%, 90%, 91%, 92%, 93%, 94%, 95%, 96%, 97%, 98%, 99% or 100% sequence identity to SEQ ID NOs: 41 and 42 or SEQ ID NOs: 43 and 44, respectively, wherein the suitable conditions comprise the presence of magnesium ions and a nucleoside triphosphate.

22. A circularizable RNA molecule, **characterized by** sequentially comprising from the 5' end to the 3' end: a) a 5' homology arm, b) a 3' self-splicing intron fragment containing a 3' splice site, c) a 5' spacer sequence, d) an internal ribosome entry site, e) a protein coding sequence, f) a 3' spacer sequence, g) a 5' self-splicing intron fragment containing a 5' splice site, and h) a 3' homology arm; or a) a 5' homology arm, b) a 3' self-splicing intron fragment containing a 3' splice site, c) a non-coding sequence, d) a 5' self-splicing intron fragment containing a 5' splice site, and e) a 3' homology arm,
wherein the 3' self-splicing intron fragment containing the 3' splice site and the 5' self-splicing intron fragment containing the 5' splice site are derived from introns of the recA gene of *Bacillus anthracis,* the 23S ribosomal RNA gene of *Coxiella burnetii,* the tRNA-fMet gene of *Scytonema hofmanni,* the tRNA-fMet gene of *Synechocystis sp.,* the 23S rRNA gene of *Thermotoga naphthophila,* the ndhL and trnL-UAA genes of *Synechococcus elongatus,* or the 23S rRNA gene of *Thermotoga subterranea.*

23. The circularizable RNA molecule of claim 22, **characterized in that** the 3' self-splicing intron fragment containing the 3' splice site and the 5' self-splicing intron fragment containing the 5' splice site are transcribed from nucleotide sequences having at least 75%, 80%, 85%, 90%, 91%, 92%, 93%, 94%, 95%, 96%, 97%, 98%, 99% or 100% sequence identity to i) SEQ ID NOs: 41 and 42, ii) SEQ ID NOs: 43 and 44, iii) SEQ ID NOs: 47 and 48, iv) SEQ ID NOs: 75 and 76, v) SEQ ID NOs: 39 and 40, vi) SEQ ID NOs: 49 and 50, vii) SEQ ID NOs: 51 and 52, viii) SEQ ID NOs: 53 and 54, ix) SEQ ID NOs: 55 and 56, x) SEQ ID NOs: 57 and 58, xi) SEQ ID NOs: 67 and 68, xii) SEQ ID NOs: 69 and 70, xiii) SEQ ID NOs: 71 and 72, xiv) SEQ ID NOs: 73 and 74, xv) SEQ ID NOs: 45 and 46, xvi) SEQ ID NOs: 77 and 78, xvii) SEQ ID NOs: 79 and 80, xviii) SEQ ID NOs: 81 and 82, xix) SEQ ID NOs: 83 and 84, xx) SEQ ID NOs: 85 and 86, xxi) SEQ ID NOs: 87 and 88, or xxii) SEQ ID NOs: 89 and 90, respectively.

24. The circularizable RNA molecule of claim 22 or 23, **characterized in that** the sequences of the 5' homology arm and the 3' homology arm are capable of complementarily forming a stem-loop structure, wherein
the 5' end of the 5' homology arm and the 3' end of the 3' homology arm are adjacent in the stem;
the sequences of the 5' homology arm and the 3' homology arm are capable of complementarily forming a structure containing a palindromic stem and a stem-loop, wherein the palindromic stem is connected to the stem of the stem-loop, wherein the 5' end of the 5' homology arm and the 3' end of the 3' homology arm are adjacent in the palindromic stem; or
the sequences of the 5' homology arm and the 3' homology arm are capable of complementarily forming a structure containing a palindromic stem and two stem-loops, wherein the palindromic stem is connected to the stems of the two stem-loops, wherein the 5' end of the 5' homology arm and the 3' end of the 3' homology arm are adjacent in the palindromic stem or in the loop of one of the stem-loops.

25. The circularizable RNA molecule of any one of claims 22-24, **characterized by** being transcribed from the vector of claim 19.

26. A circular RNA molecule, **characterized by** being transcribed from the vector of claim 19, being prepared by the method of claim 20 or 21, or being circularized from the circularizable RNA molecule of any one of claims 22-25.

27. Use of an intron derived from the recA gene of *Bacillus anthracis,* the 23S ribosomal RNA gene of *Coxiella burnetii,* the tRNA-fMet gene of *Synechocystis sp.,* the tRNA-fMet gene of *Scytonema hofmanni,* the 23S rRNA gene of *Thermotoga naphthophila,* the ndhL and trnL-UAA genes of *Synechococcus elongatus,* or the 23S rRNA gene of *Thermotoga subterranea* in the preparation of a vector for the preparation of a circular RNA.

28. The use of claim 27, **characterized in that**
the intron of the recA gene of *Bacillus anthracis* comprises a nucleotide sequence that has at least 75%, 80%, 85%, 90%, 91%, 92%, 93%, 94%, 95%, 96%, 97%, 98%, 99% or 100% sequence identity to SEQ ID NO: 18;
the intron of the 23S ribosomal RNA gene of *Coxiella burnetii* comprises a nucleotide sequence that has at least 75%, 80%, 85%, 90%, 91%, 92%, 93%, 94%, 95%, 96%, 97%, 98%, 99% or 100% sequence identity to SEQ ID NO: 19 or 59;
the intron of the tRNA-fMet gene of *Synechocystis sp.* comprises a nucleotide sequence that has at least 75%, 80%, 85%, 90%, 91%, 92%, 93%, 94%, 95%, 96%, 97%, 98%, 99% or 100% sequence identity to SEQ ID NO: 20;
the intron of the 23S rRNA gene of *Thermotoga naphthophila* comprises a nucleotide sequence that has at least 75%, 80%, 85%, 90%, 91%, 92%, 93%, 94%, 95%, 96%, 97%, 98%, 99% or 100% sequence identity to SEQ ID NO: 21;
the intron of the tRNA-fMet gene of *Scytonema hofmanni* comprises a nucleotide sequence that has at least 75%, 80%, 85%, 90%, 91%, 92%, 93%, 94%, 95%, 96%, 97%, 98%, 99% or 100% sequence identity to SEQ ID NO: 60;
the introns of the ndhL and trnL-UAA genes of *Synechococcus elongatus* comprise a nucleotide sequence that has at least 75%, 80%, 85%, 90%, 91%, 92%, 93%, 94%, 95%, 96%, 97%, 98%, 99% or 100% sequence identity to SEQ ID NO: 61; or
the intron of the 23S rRNA gene of *Thermotoga subterranea* comprises a nucleotide sequence that has at least 75%, 80%, 85%, 90%, 91%, 92%, 93%, 94%, 95%, 96%, 97%, 98%, 99% or 100% sequence identity to SEQ ID NO: 62.

29. A host cell, **characterized by** comprising the single-stranded DNA molecule of any one of claims 1-17, the double-stranded DNA molecule of claim 18, the vector of claim 19, the circularizable RNA molecule of any one of claims 22-25, or the circular RNA molecule of claim 26.

30. A method for screening an intron having self-splicing capability, **characterized by** comprising:
i) providing a double-stranded DNA molecule comprising, from the 5' end to the 3' end, a promoter, a first sequence, a 5' natural adjacent exon of an intron to be screened or a sequence corresponding to the natural adjacent exon, the intron to be screened, a 3' natural adjacent exon of the intron to be screened or a sequence corresponding to the natural adjacent exon, and a second sequence;
ii) subjecting the double-stranded DNA molecule in step i) to transcription to obtain an RNA molecule;
iii) treating the RNA molecule of step ii) under suitable conditions, wherein the suitable conditions comprise the presence of magnesium ions and guanosine triphosphate; and
iv) analysing whether the RNA molecule treated in step iii) comprises the transcript sequence of the intron to be screened, wherein the absence of the transcript sequence of the intron to be screened in the RNA molecule treated in step iii) indicates that the intron to be screened is an intron having self-splicing capability.

31. The method of claim 30, **characterized in that** the transcription in step ii) is performed by *in vitro* transcription.

32. The method of claim 30 or 31, **characterized in that** the analysing in step iv) comprises:
reverse transcribing the RNA molecule treated in step iii) into a single-stranded DNA molecule and sequencing the single-stranded DNA molecule, or sequencing the RNA molecule treated in step iii), wherein the absence of the sequence of the intron to be screened indicates that the intron to be screened is an intron having self-splicing capability;
reverse transcribing the RNA molecule treated in step iii) into a single-stranded DNA molecule, converting the single-stranded DNA molecule into a double-stranded DNA molecule, and comparing the length of the double-stranded DNA molecule with that of the double-stranded DNA molecule in step i), wherein the length of the double-stranded DNA molecule being shorter than that of the double-stranded DNA molecule of step i) indicates that the intron to be screened is an intron having self-splicing capability; or
comparing the migration rate of the RNA molecule of step ii) with that of the RNA molecule treated in step iii) in gel electrophoresis, wherein the migration rate of the RNA molecule treated in step iii) being greater than that of the RNA molecule of step ii) indicates that the intron to be screened is an intron having self-splicing capability.
